# EUROPEAN PATENT APPLICATION

(11) **EP 1 396 544 A1**
(43) Date of publication of application: **10.03.2004**
(21) Application number: 02078687.7
(22) Date of filing: 06.09.2002
(51) Int. Cl.: C12N 15/53, C12N 15/11, C12N 9/08, C12N 5/10, C12Q 1/68, C07K 16/40, A01H 5/00, A61K 38/44, A61K 48/00, A01K 67/027, G01N 33/573

(54) **Nucleic acids encoding human thyroid dehalogenases**

(71) Applicant: ACADEMISCH ZIEKENHUIS BIJ DE UNIVERSITEIT VAN AMSTERDAM, 1105 AZ Amsterdam (NL)
(72) Inventor: Ris-Stalpers, Carolyn, 3641 AP Mijdrecht (NL); Moreno Navarro, José Carlos, 1093 MK Amsterdam (NL)
(74) Representative: van Westenbrugge, Andries

(57) **Abstract**

The present invention concerns a nucleic acid sequence encoding the human thyroid dehalogenase and related sequences. The nucleic acids may be used in methods for producing a dehalogenase, as well as in methods for diagnosing or treating iodityrosine dehalogenase deficiencies, e.g. in some forms of (congenital) hypothyroidism. The dehalogenases may be used for dehalogenation of halogenated products as used in diagnosis and in treatments as those involved in toxicology.

SEQUENCE LISTING

<110> Academisch Ziekenhuis bij de Universiteit van Amsterdam

<120> Nucleic acids encoding human thyroid dehalogenases

<130> BO 45459 EP

<140>

<141> 2002-09-06

<160> 3

<170> PatentIn version 3.1

<210> 1

<211> 288

<212> PRT

<213> Homo sapiens

<400> 1

Met Tyr Phe Leu Thr Pro Ile Leu Val Ala Ile Leu Cys Ile Leu Val
1               5                   10                  15

Val Trp Ile Phe Lys Asn Ala Asp Arg Ser Met Glu Lys Lys Lys Gly
            20                  25                  30

Glu Pro Arg Thr Arg Ala Glu Ala Arg Pro Trp Val Asp Glu Asp Leu
        35                  40                  45

Lys Asp Ser Ser Asp Leu His Gln Ala Glu Asp Ala Asp Glu Trp Gln
    50                  55                  60

Glu Ser Glu Glu Asn Val Glu His Ile Pro Phe Ser His Asn His Tyr

65                  70                  75                  80

Pro Glu Lys Glu Met Val Lys Arg Ser Gln Glu Phe Tyr Glu Leu Leu
                85                  90                  95

Asn Lys Arg Arg Ser Val Arg Phe Ile Ser Asn Glu Gln Val Pro Met
            100                 105                 110

Glu Val Ile Asp Asn Val Ile Arg Thr Ala Gly Thr Ala Pro Ser Gly
        115                 120                 125

Ala His Thr Glu Pro Trp Thr Phe Val Val Val Lys Asp Pro Asp Val
    130                 135                 140

Lys His Lys Ile Arg Lys Ile Ile Glu Glu Glu Glu Glu Ile Asn Tyr
145                 150                 155                 160

Met Lys Arg Met Gly His Arg Trp Val Thr Asp Leu Lys Lys Leu Arg
                165                 170                 175

Thr Asn Trp Ile Lys Glu Tyr Leu Asp Thr Ala Pro Ile Leu Ile Leu
            180                 185                 190

Ile Phe Lys Gln Val His Gly Phe Ala Ala Asn Gly Lys Lys Lys Val
        195                 200                 205

His Tyr Tyr Asn Glu Ile Ser Val Ser Ile Ala Cys Gly Ile Leu Leu
    210                 215                 220

Ala Ala Leu Gln Asn Ala Gly Leu Val Thr Val Thr Thr Thr Pro Leu
225                 230                 235                 240

Asn Cys Gly Pro Arg Leu Arg Val Leu Leu Gly Arg Pro Ala His Glu
                245                 250                 255

Lys Leu Leu Met Leu Leu Pro Val Gly Tyr Pro Ser Lys Glu Ala Thr
            260                 265                 270

Val Pro Asp Leu Lys Arg Lys Pro Leu Asp Gln Ile Met Val Thr Val
        275                 280                 285

<210>  2

<211>  1174

<212>  DNA

<213>  Homo sapiens

<400>  2
gaagagttct gcactttgtt ccttccctgg cacacctgtg tctgcattcc ttctatctcc
60

cggcattctc cactcctgtc tctgtgtgtt taaaaaccgg tgtgggaagt gtgcacgcct
120

gtgacgtcag actccagacc atgtatttcc tgactcccat cttggtagcc attctctgca
180

ttttggttgt gtggatcttt aaaaatgccg acagaagcat ggagaaaaag aagggggagc
240

ctagaaccag ggccgaagct cgcccctggg tggatgaaga cttaaaagac agcagtgacc
300

tgcaccaagc agaagatgct gatgaatggc aagaatcaga agaaaatgtt gaacacatcc
360

ccttctctca taaccactat cctgagaagg aaatggttaa gaggtctcag gaattttatg
420

aacttctcaa taagagacgg tcagtcaggt tcataagtaa tgagcaagtc ccaatggaag
480

tcattgataa tgtcatcaga acggcaggaa cagccccgag tggggctcac acagagccct
540

ggaccttcgt ggttgtgaag gacccagacg tgaagcacaa gattcgaaag atcattgagg
600

aggaagagga gatcaactac atgaaaagga tgggacatcg ctgggtcaca gacctcaaga
660

aactgagaac caactggatt aaagagtact tggatactgc ccctattttg attctcattt
720

tcaaacaagt acatggtttc gccgcaaatg gcaagaaaaa agtccactac tacaatgaga
780

tcagtgtttc catcgcttgt ggcatcctgc tagctgccct gcagaatgca ggtctggtga
840

ctgtcactac cactcctctc aactgtggcc ctcgactgag ggtgctcctg ggccgccccg
900

cacatgaaaa gctgctgatg ctgctccccg tggggtaccc cagcaaggag gccacggtgc
960

ctgacctcaa gcgcaaacct ctggaccaga tcatggtgac agtgtaggca gggcccccca
1020

agggagtggc agggagatgg cgcccctgct tttccctgag cctctcgcct gctcctcttg
1080

ggtctcttgg ctgctctttc tccaggtgtc aggtcccctc attgctcttc tcaggtggcc
1140

acactatgtc aagaagcctc tccacactct gtgg
1174

<210> 3

<211> 107159

<212> DNA

<213> Homo sapiens

<400> 3
gatcaggttg tctgtagccc caagactgag tgcacacgtt tccatcaagc caggcagaag
60

ggccaccagg tgaccgcagc agccaacccc aaggggtctc tggcagagct ttgggcttct
120

ctgcagatgt cgaggttgga tttattcttc ctgccctcct accgagcatg tcttggggat
180

tagtgggaaa ctgactgtaa agatcttttg cttccttttt gcatatagct atgtaatctg
240

acttcaggtg aaaatttatg tctaagtggt aaatgttttt gagaacccag tgaggattgt
300

gaatcccttt gccagtttca tgatggaggg agttaacatg gatcataaaa gggctgaaag
360

agtgtctgag gttacctcta ccttgtatca ccatacgaag tagccaacaa ctccatgaga
420

taagtttttc tccagcagca tcattggagc ttgcagcaca agttggaaaa gatggggaaa
480

acccactttt ctcttatatg aaaatgactt ctcccaatcc tgttttgtct cagcatacaa
540

tatatatggt tgttaattta ctacccacag catcatggac tcatcctctt ccctgaatcg
600

cttgtaaata ttttctcaaa ctgagtgtat tttccaatgt ttatccaggt atctgcatat
660

ccaactcttc attgtccctg gtgcaggtta accatgctct gcagggtacc tctttatccc
720

ctcagcctgt cccaggcctc ctgccagtgt ccacagtgct gtgcgttggg tgtctaattc
780

cacctccccc tgagaggtag gtggccagcc acccctagga gaagctgaca ccaagggtgg
840

ccatatgggg ggaaggaaga ggttggtgct ggttacctcc caccttggac tatgttcatt
900

tctcttttcc ttactgcagg tcactgacat gtgaacacat gctcctaaca agttgacccc
960

agtcttgctg aaattgccta gactgtatta ttttcttccc catacttgac atagagcata
1020

agctgagagg tacataatac atgccataat ttatgacggt gcttaggtaa agccatgcaa
1080

actcgtctat cattgccagg ttcatttcca gtggtgttca ttgaaggtaa gttgactcat
1140

ttatacaaaa caattccctc atttcccttt gagcaattgt caagtgtgct tgacgcaggc
1200

caagtgacct tcaaatgcgg agtggggttt ggcctggaat gtcagtgtgc atttggcagc
1260

atgaattgct ccaaagaatc ctgatttctt atgcatgaac tgctgacccc agcactgggg
1320

cctggagagc gggacgggaa ggaaggtgaa ctcctgctga ttcaggggcc ctcccagcag
1380

cctttccacg gtgtcagcag tattcacatc actttcctct cctccctctt ccgccccacc
1440

gtaagtcctg tccctggaag cacttttcag ttgccaggaa tcatgaacat taggaaacaa
1500

tccaaaaaaa cggggtgtct actttatatt ctgtggccca cacggtcaca catgggcctg
1560

gttttaacac cgggtcatag gtgtggaaac agcctggcct agaggaagga acactgaacc
1620

agtgagagag gccttcagtg actccaggac caagcaagtg tgtggcctgg aggctggcct
1680

ttctaagagg cctgtctcgg tggaaacgga ggaatgttct actgctggct ttccagagcg
1740

ctcctctagc cccactactt ggagcgatgc ttcccggtgt tgaagctgca gatctaagga
1800

ccatgtttct tcaaacacaa ccaggcctct agggcaagat cgtttccagg agatgtaatg
1860

aaatatgaag gaaaacgccc agcaggcaac atgcctgcct gctgaggatc acaggaaaca
1920

gccttactac caatctacca atactgggcc ctgagctgga gaaacagctc cttttttcat
1980

ttcaaaatat cactaatgtt ccatggtgca ggacatattt caaatgcctc aattggaaaa
2040

aacaggctcc aaaggcatca cggtgtgcct gtggctcttg tctgtgctag gcagcagcac
2100

aagggcctgc tcattagctc actgatgaca ggtttatccg caactgtata tcaagcaatg
2160

agaaagtcag ctgtgcgagc aaacgtccct cagtcatatt gcacaaagcc ttcgtaaagg
2220

gatagagtgc tgaaagcctc ctaactcctc tctagctgaa catcttttcc cctccgctac
2280

ctctgaacct ctcatccttc caactcagct caagaacgca gaagctgttt gccgacccct
2340

tctgctagtg tcctgtcctt ggtaattcaa agacgccccc gggatggccc acaaagagcc
2400

ctgtgtgcat gcacacgcgc cactgtcact gataaggcca ctgggtggtg agcggttctt
2460

ttcacacttc acatccagag gcttttcctg tgatttgtgt gtgaagcacc tgtttgcatg
2520

gtccgtccac ccttatctga gattctgcct cttaggcacc agggcaggtt gaagcctttt
2580

tcacccactt acaaagcaac agcttattcc tgagaagtgt gtcaagctgg tatgtccact
2640

gtgctcccca gactcaccaa taagatcact gacttaattg ttttctgccc cttactttta
2700

gaagaaagaa gttaggcaat ctaaaggagt caaactgata atgttgcttt tgaaaaaaag
2760

taaacaaata ataaaacatc tcccagagtg tatggggtcc aaaacacatt taacccagtt
2820

tctcacactt tccatatgaa catttactca ataatcctac gtgatgttta gaatgaataa
2880

ttactgtggg gcccctcatc aaacatcaga aagagaccaa taggttcgat attttgccgt
2940

aaagcaggaa attgaactaa gtatcaagaa ataaatacca acagagagaa taacaaagga
3000

aggaaggagg gattaattgt cattgttaga gaaaaactca ttttctgatg atgatggtca
3060

agaccttatc ttataatcct cctaactata gcctcctttt ctttgcattg ggtacccaac
3120

atccattatg actgttgtct ccagaagccc agagacattc atctttttgc tctcctttca
3180

ctgatgagga gactggacgt cacgtgataa ttcagctgcc cagtcttatg gcaagcgaga
3240

gagacctatt ttgaactgag gcatgctgga cttcaaagtc catgttcttg ctcttagagc
3300

aagtctctaa aacaagattt tcactaattc ttaacaattt attgagttca gcagttactg
3360

agcatctatc atgtcttggc agtgataggg aatacagata taaaaaagtt acagccctac
3420

cttcacttga gccaaaaata tttttcttta gggaatcttt agaacaaaca ctttcagaaa
3480

atttttctaa ttgagcttga gatgttgact tgactctcat ttttaaaact aactaattgg
3540

ctatcaaaat ggcaacactt acaagataat gctttctgta tttcttcatg catttaactc
3600

cctaactaat gaaaaacttc atgatatttt ctcccaaagg aggggataga agtgtctttt
3660

cttctcacac tgtgaagaca agtaaacgta atttagtttc aatacagtta tattagcttt
3720

agataataat gagctattgc catatatcag accacaaaaa aagaaccaaa actaaacatt
3780

aatacaaggt atgtttttat ttctattata ctttaagttc tgggatacat gtgcagaatt
3840

tgcaggtttg ttacataagt atacacgtgc catggtggtt tgctgcaccc atcaacccat
3900

catctacatt aggtatttct cctaatgcta ttcctcccct agccctccac cccccaacag
3960

gccccagtgt gcgatgttcc cctccctgtg tccatgtgtt ctcattgttc aatgtgtaac
4020

agagttggag tatggcttct aaaaatcata atttcatatg cataatggca attgctagac
4080

aagaattctg taactagagc tagttacatg agcaaaaact gcctcatagt tcaggttgca
4140

aagtgggcag ccattatagc ccagaagaca ctcccagaag aaattggtga gctaactttg
4200

tatatttatg aacatttttg gtattttcag tctttccgaa gcacctggtg atggccagtg
4260

tggaagacta ggtggagcat gacaagatca ttgtctgtaa gcactcactg tgctgtttct
4320

agaagacgct tctgtaggtg aataaaggtg gttattgctg agtctttctt caccaaaaaa
4380

taatgaaact aatttaatag gctgatttgg aaaagagcct agtaaaatcc gctgcagaat
4440

tgggatttgc tttgaaaagt gtctctgtcc ttacaggcac actgatggag tattgttttg
4500

ttcctcatgt ggcatttttc aagccacttc aagtctgttt tcaaactaaa ttgggaatta
4560

aaaaatcata tgataagtaa attgaaaaat gtaactcact agtgtccatt ctctagcatc
4620

caaagtattt gacctattac aaatttatca acagtaaata tttcttagac taatttgtaa
4680

cctgttataa attcacccaa aggaatagta attttcataa taatggaata aaagagcttt
4740

caatgtccct tatgaagctg tgttcaatgg aaataaagca ttgggtgtag cggtgggtat
4800

gtgaatggat ggagaactgg ctgagcagcc tatgggttga ttagccatag aaacattggg
4860

ccttgattgc tgcaagccct cagcctgggc ccagtttaag agcagcctga gggtaggagg
4920

ggcttgatgt gtctgaatgt ggcaggttga caataaatta tacttatttt gtaaacactt
4980

acatagactt tatgccagtt ctgctctata accttcgcag ttattaatgc attttgtcct
5040

cataactctt tatttattta tttatttatt tatttattta tttattttga gaacagagtc
5100

tcactctgtt ttcccaggct ggagtgcagt ggcatgatct cagttcattg caacctccgc
5160

ctcccaggtt caagcaattc tcctgcctca gccccccaag tagctgggat tataggctcc
5220

cgccaccacg ccctgccaat ttttatattt ttagtagaga cgaggtttca tcatgttggc
5280

caggctggtc tcaaactcct gacctcaggt gatccacctg cctcagcctc ccaaagttct
5340

gggattacag gtgtgagcca ccgtgcccag ccctttatct agtttagagg taagaaaact
5400

gaggcagaga gaagataagt tgtccaaggt cacacacagc gagcactgga gtcaggatta
5460

caaatgcagg cagctgggct ccagaattgt cacctttcaa cactgtgcca tgcagccttc
5520

caggtaagcc agctgcatgt ttgtgaacac ttgcaaaact ggctccattt ctaggcggct
5580

gactcaatat atctgaccta aggtacctca cctgcccacc caactcttac cttcaaaagg
5640

ttcctgtcta gccaggcaca gtggctcaca cctgtaatca cagctactca gaaggctgag
5700

gcaggaggat tagagcccag gagttgagaa caacctgggc aacacagcaa gaccccatct
5760

ttaaaaaatt taattaaaaa aaaaagggtt tctgtctgca agtgaaggaa gcattgcttc
5820

tggaaggacg ctactgtaat ggaaatgtgt taataactgg gagaataaac cctaaggcca
5880

tcagtgctct gtgactttca gtagggaatt gttttcttct aagctatgag tcatcagaga
5940

gaggttgggt ccttaagaaa caaagcacaa gataactcca tgaaggtaaa gctagaaaaa
6000

aaatgggcta acatcaagtc tgttaccagg aagaggtttg gatagggcat cctgagaggc
6060

ctggagaaaa acctggtaag atgttaaaat tttgcctatg aaaaattcat taaagcttta
6120

tttttaatag tgagaaagat ggaaacaccc aaatatccat caataggtgg ttgattaaat
6180

aagtatttta cattcatgta atgagctaat ccacagtcac taaaagataa gattctatac
6240

attagacagg gtatgatccc attttgtaaa tatgtacaaa aactagtgct gtacatatat
6300

caaaaaatct caagagaata taatgaaact gacaatagtg gttaccttga tggatgggat
6360

tactagatat tatatttttg tgtttataca tttcagaatt actagcattt tcataataaa
6420

tgtacattat tttgtacaaa tgaaaaaaaa ttaaagattt tggctttttt ttcagagaag
6480

gtaagcctat gtgttactgg ggagaaaggg aggtttgcag tatcattaaa gggctgcatc
6540

ttggatatta agaaatttct ttgccctctc tgccatattt ccttaatctc acaaaaatcc
6600

ctcttattag actaaacttt gatattatac cattatttcc ttttgagaaa aataagaaac
6660

agttgcacaa aatgtgtcac tgtatctatt gaagaagata ttgttgagga tgttgtccat
6720

tatactagct tttccatatg agacagcttt ctcgggcccc cttatcgtga aaagctacct
6780

tcctgaacag ctgggcatct taccttaagg acactgtgaa taaccttgca tatctctttg
6840

ctttggctac tgggaagctt agagacagaa ttctcacctg tctggcagct gtacagaatt
6900

agctagtgta gatttggata cgctattcaa acagctaact ttgctctaat tcctttctct
6960

tttattcttt tgtattttat gtatctttgt cagttacctc aaatagatac atttcaagag
7020

gaagtcattg ttgaataaca atttaatgac aggtgtgcca cctcgctaca gccttttcga
7080

aggacatttg tttgtacaga gtgaagcata gtccttcgtg caggtgccat gaggaccacc
7140

tccactttga tctcttttac agattgggat tgtagatgga acttccttta ataaaaagat
7200

tccatttcct taaaaatagt tcaaaaaacc ctctaataca gaaatgcagt tggaggcatt
7260

ttaatgcatg tacttgaatt attttacata acacatttca agcatgtagt gttgtatatg
7320

atagtctttt gatatctttg cctgtcatta tttatatgat ttattatttt taccctgacc
7380

acatgtatcc cttcagctaa tcccatttct tttctctcgt tcagagccag acttctcaag
7440

acctgtcttc acctgctgtc tccacctcct gttcaccact caacccattc catgtggctg
7500

ttgccctcat catcctatta aaaccagctg caataatctc catgttagta aatccatgta
7560

catttttcag gctcttgact tctcagcagc attcaacaca gttagttgac tgcgcgctgc
7620

cataaatctt ctattatttc accaatacac ctctggccgt cctttttcct tatgagtttt
7680

tcctcttctc actggctatt aacattgaga ggcactgcct aggacctctt tccttctttg
7740

cacaggtccc ctggataatt ccatgtgtgc ctatgttttc aattacaata taccattgtc
7800

ttccagattt gtaccttcag cctagacccc tcattcaact ctagacctat gggtctgact
7860

gcttattcaa catctcctta gggtgacttc aaattcaaca tgatcaaaac tgcgtgatct
7920

tctgctgagc ctggttcctc tactaaggtt tcctacctta ctgaatgact ctctttcatc
7980

catacaagct ggaagaatag agccttcttg tccttaccac taattcctat ccacccacct
8040

gtatctcaca aaactttcca cacctctttg tctccatagt caccacttca atccaagcca
8100

ccatactgtc tcactggagt tcaatggcct cttttttttt tttttttttt tttgaaatgg
8160

agtctcactc tgtcacccag gctggagtgc agtggtgcga tctcagctca cttgcaagct
8220

ccgcctcccg ggttcacgcc attctcctgc ctcagcctcc caagtagcct gggagctgcc
8280

agctactgaa acataaaaca gattcaaacg ggactcaatc taatacaaaa agcatgttcc
8340

atagatggaa acattaaact ccatccaaat taaaaggttc tgaaatttta agagatgctg
8400

ttaagaaaat gaaaaggcaa gccacaaatt gaaaaaatat ttgcaaaatc acagatctga
8460

taaagtgtct agaatatgga aagaattctc aaaactcaaa attaagcaaa catctcaatg
8520

aaaaaattag gaaaagcttt gagtaggcac tgcatcaact cagatagaca ggtggcaaat
8580

aagtatatga aaagatgctc aacatcagtc attagggaaa tgcaaattta gtgagatact
8640

actatatagc tattagaatg gcaacaaaac aaaactgaca atattaaagt gctcttgagt
8700

gcttggagta actggaactc tcatacgttg ttggtgccag agggcacagc cactttggaa
8760

aacagtgtag cagtttctcg taaagttaaa catatacttt ccatgtagcc cagaaatcca
8820

gctcctaggt attcacccaa gtgaaatgaa atcttatgtt catgtaagtg actgtctatg
8880

gaagcttgat ctgtaattat gaaaaagttg aaagaaccca aatgtctacg aagtggtgaa
8940

tgaatgatga atctgtgata catctgtaca ttgaaatact acttagcaat aaaaaggaat
9000

gaagtagtga tatgttcagc agcatggatg actcccttct gtgttgcatg ccccagctga
9060

atggcacatc agaagctcaa gttcacctag ggtggcaaat gtcctcagag caaagcaact
9120

ttggtgcttc ttattaccac tctaggcgtc agcttccctt agatttgggc ctgattattc
9180

cttactatct tctcgatgct ttgatgcttt taagaagata tttaaacatt tttttatcca
9240

gaatttttag ctatcagcaa aaagactggt ttaaataaca ttgtccactg tgtcaccaga
9300

gacagaagtt gagtaattgt acttatatat catagtcaaa ggaaaggatt ggtgtggtgg
9360

gtttcaggca cctagataga gtcaggagac atgtttcggg aaaggagatg ttttccccat
9420

gctactggta tctcatctca aaggcgagta cgtattcata tgctgggtta tggcaagagc
9480

aatgacattg actgaaagga tatccttcag ggcacagcat cagaacaatc taccatgaag
9540

ttcaacccta gagttttctt taattcgaca ccacttttcc cattccaggt aaccatatag
9600

aaatgggact gtggggccct ttccattggt aacctcagcc tgccactgtg gcaccgcccc
9660

ctggagtgtc tcatccttaa cacttcagac tcagtttcct tttttccctt ctccagcatc
9720

tcttcactct tcacacctga taccactttc ttcctgagcc ttggctctcc tcccccagct
9780

ctgtgcttct caagcttcct ggccctacct ggccctgggc ctgcctacct gggcatgacc
9840

ccttagaggg tggcacacga ctatctctgc ctgagccagg ggcagcaccc agacactaca
9900

cgcagatgcc acagttctca agacagagcc ttctatccaa attcaaatac gaaagttaaa
9960

tcattcaatt ccaaaacgtg tcttcaccgt ccagtgatct gtttccaggg tcaatggcaa
10020

tttaaaagct actagaaagt gcaagcttcc atttcttaca tttttgtgtc ctgtataaat
10080

gattcattta aaaatttctt agagaaatta attatatgcc actctaactt taatactttc
10140

tcctggtagt ctctacatca catcaaaaat aaaaatgagc cacatctgca gaaaacatat
10200

tataactgat aactcctttc ctttcaggga tcacttacta agagagttac aaagatcagc
10260

tttggggaca tataaggata aataattgga ataatactgt gttggagctt aaataacccc
10320

atttcccaat atctaacatt ggttgatgca ataaagatgt gtgtgtgcgc ttcataaaac
10380

atgaagccag agagtgggag gttctgggag gggtcagtgt ttcagcagtg cctacaggcc
10440

cagcctcttt ctgctgcacc atcctcgtga gaagcatctg tcctgggact tgcctggtac
10500

agtcacagga tggctgccag agccccaagc ccgaggcttc acatgctatg ggcaaggcgg
10560

taagaaggga aagtggctct cttcccaccc tctcctcttt tcattaggaa gcaaatttct
10620

tcattactca catggaccag aacattctgt attaatttcc agggctgcct tagcaaagta
10680

ccagagactg gatggcttaa accgcagaca ttgatggtct cacagttctg gaggctggaa
10740

tttcagcatc agggtgtcaa caggatcagc tccttctgag ggctctgagg gaaaggtgta
10800

ttccagactc tttcctgggc ttggagatgg catcttctcc ctgtgtctct ttacatggtc
10860

ttccctctag agaggtctct gtgtcaaaat ttcccctttt ataaggatac cagtaatatt
10920

ggattagaat ccacctgaat ggcctctttt taactcaatt gtctctgtaa agaccctatt
10980

tttaattagg tcacattcta agatactagg ggttaggact ccaacatttt tttttgagg
11040

gtggatgggg ataccattca acccataaca catggccacc ccaaaccaat cactagcatg
11100

gggaaattac tgtgattggc tcagaccaat cagattcatc ccccgaggct gaaagtgggg
11160

cacaactttc ctgagcagtg accgcctgaa caaaactggt tttccataga tgaaggggaa
11220

ggagtacggt tagtgggtgc caattcaatc caaatggagg cccagagaga tgaagcgaca
11280

catttaaagt cacataacca acccatactc cctcttggac accatgctga gatacattgt
11340

gtcagtatag gaattaccag tgcttgcttt tcctggaaaa taaaatctgt cttccttttc
11400

cttctctcct gacttctgta cccagggagt gggggagaaa aagataccaa ttacgcctta
11460

accaattttt ctctttactc ctgccccaaa gcttttttgc ttcacttcac ccagttaccc
11520

ttgagatgct acttgcctat aatcctaagt tttgagcacg gaaaaccaat ataaccttag
11580

agtttctacc tgaaacaaaa agattaaaat gatcatgtcc ttgatgtctc ataactgaga
11640

tgaggtaata cacaagagat taacaaggac aagtattgac cagactgtag tatcctgtgg
11700

ctgggatatc ttgtggtgag aatcattaaa ataagatcat gcaatagagc tttgcaagat
11760

gttctcaaaa acaaaatctc atcatggtgc tggacacgtg aaccaaaaaa aaaaaaaaaa
11820

aaagagttta agcctgaaaa aaaacaaaaa caaaatcttc aaattcagct ggaactgaat
11880

gcttctcttt tactcggtac aggaagcaca tacatatgca gttctccagg ctcaagatgg
11940

attctcagga aaaccaaaca agataggagc agagggccag catggtggct catgcctgta
12000

atcccagcac tttgggaggc tgaggcaggc agataacttg aggttgggag tttgagacta
12060

gattggccaa catggtgaaa ccctgtctct actaaaagca caaaaattag ctggctatgg
12120

tggcacacac ctgtaatccc agctacttgg gaggctaagg caggagaatc gcttgaacct
12180

gggaggtgga ggttgcagtg atccaagatt gtgccactgc actccaacct gggtgacaga
12240

gtgagactat gtctcaaaga aaaagaaaaa aaaaaaaaag gaatgagcac agaactcata
12300

cacaatgtat ttatctttac aagtaaagta taagtactta attaatgcaa ggacttaagg
12360

gtttattgaa ttatctcctg catgtgtgca cttcttaaaa taaatggtga ttgggtccta
12420

gactatcctc aaaaaccctg ttccatctgt aatatttctg aagtataaaa ctacatgttg
12480

tgtatgtata tagttgacag ttgaacaatg gagggttagg ggcatcaacc ccctgtgcag
12540

gtcagaaatt tgggtctaac tttgactccc ccagaactga attactaata acctcctgtt
12600

caccaggagg cttacagata atataaatag tccattaaca catattctat atgttatatg
12660

tattatatat tgtatccata caattaaata tactagagaa aataaaatgt tttgagaaag
12720

tcataagaaa gagaaaatat atttactgct tactaagtgg aagtggatta tcctaaaggt
12780

cttcatcctt gttttcacgt tgagtaggct taggagaagg tggatgagga ggggctagtc
12840

ttgctgtctt ggggcagcag aggtggaggc aggaagggca ggagaggcag gcactcagta
12900

taaatttata gaaagacatc ataatttctg tttgactttc ttgctttttt gtttctctaa
12960

aagtgtttct atgctacagt gccaattctt caaccatttg cttcagtttc agtgccagta
13020

tcatggaaga gtccatgtca taaaagaatt caaagcagtc ttgaataatc agaagacttc
13080

tcccagattg tctaatgttt tctggcactg cttcatctgt ctttctcttc atagtctggc
13140

actggtttgg cgtcactctt ctccatcaag tcatcttttg ttaattcctc tggtgtggtg
13200

tcttttagct ctttaatttc tccaagaacc gtatcttgac accctttacc cctatctttt
13260

tttccataat ccctttcatt atttccttga ttggctctgt tgtaaatcct gtgaagtcat
13320

gtacaacgtg tggacacagt tttctccaat agtaatttct tgttttgggc ttgatggctt
13380

ccatgttttt tttctgtaac agtgatggca tcttcaatgg cgtaaacctt ccagactttc
13440

ataatgttct ctctaatcag ggatctcttc catagcattg acaacatttc ccatagagta
13500

ccatgtgtaa tgagtcttaa agatccttat gatctcctaa tattggggct gaattagaga
13560

ctttgtgttt gggggcatgt agaccacttc aaccacttca cctttaaatt tctggggttc
13620

tcagtggcca gaggcattgt ctaatatcaa aagaacttta aaaggcaatc ccttactggc
13680

aaggtacttt ctgacttcag tgacaaacca tcaatgaaac caatccagaa aaaagggttc
13740

ttcttgtcca ggccttcttg ttgtacaacc aaaagactag cagctggtgt gtgtcctttc
13800

tctttaaggc tcagtggtta acagctttac agataagggc aatcctgatc acaaacccaa
13860

ctgaatttgc acagaacagt acagttagct accccttcct gccttaaatc ctggtgcttg
13920

cttctcttct tactaataaa tgtcatttgt ggcatttttc ccccagaata ggccactttc
13980

atctgcatta aaaatttatt caggcaagtc ctaattacct tcttaatggt gcctgggaac
14040

tcatctggtg cctcttcatc agtagaagct gcttctcatg ttatcttgat gttttaaaaa
14100

gtcaagcctc tttctaaaat tatcaaacca tcctttgctg gcattaaatt ctccaacttt
14160

acattcttca ccttcctttg ctttaaggtg tcatatagtg acttagcttt ttctctaatc
14220

atattagagt ctgtaggtat gcgtttctta cagcaatcct gcaaccacac acacaaaagt
14280

tgcattttca atacaagatt aaaaaggtgt atttcacaaa aagtacaagg tttttgtgcc
14340

tgctgacaga tgcagggatg gtttcacaaa tttccttttc tttttctttt ttttttttta
14400

caatgatcct tacaccagat taatttgtct tgaaatggca ggcaattgca atggcagaca
14460

tcaacctatg atatacataa agtaattaag tttttttttt ttttttgtaa tgtcatgact
14520

ttgcttcttg ggagcacttc cagcatcact agtgacactt cctatgagtc ccatgatgtt
14580

attcaaggtt tatggtattg cataaacaca atgaaaaatg caagagaact gtgagagatc
14640

actttttact ggtgtacaca acctactgga gagatgaact aatcatacgg agataattag
14700

cataatacag tctttaaatg gatactcaca acacttgggc tcacctcaat aaaaacagga
14760

agtggctacg aaattattac agtagtgctg tatgtactat agttaattct atgctatatg
14820

attttttttt tttttttttt tttgagacag agttttgctc tgtcgcccag gccagagtgc
14880

agtggcgcga tctcggctca ctgcaagatc tgcctcccgg gttcaagcca ttctcctgcc
14940

ccagcctccc gagtggctgg gactacaggc gcctgccacc gctccaggtt aatttctttt
15000

ttttgtattt ttagtagaga cagggtttca cagtgttagc caggttggtc tcggtctcct
15060

gacatcgtga tcttcctgcc tcggtctccc aaagtgctgg gattacaggt gtaagctacc
15120

gcgcccagcc tgtatgattt ttttttacta taattgttct tttctttaaa atgtttattt
15180

caatagtttt gggggaacag gtggtttttg gttacatgga aaatttcttt aatgatgatt
15240

tctgagattt tggtgcaccc atcaccccag cagtgtgcac tgaacctaat gtgtagtatt
15300

ttatttctca cccacctccc acccttctcc ctggctccct aaagtccatt atatcattct
15360

tatgcttttg catcctcata gtttagctaa atgagaacat acaatactta ttttttcatt
15420

cctgagttac ttcatttaaa ataatggtct ccaactccat ccagcttgct gcaacgccat
15480

tatttcgttc tttttatagc tgagcagtat tccatggtgt atacattttc tttatctact
15540

cattggttga tgggcattta ggttggtttc atttttgcag ttgtaaattg tgctgctata
15600

aacatgcatg tgcaagtgtc ttctttatgt aatgacttct tttcctttgg attgaaactc
15660

agtagtggga ttgctggatc aaatggtagt tctacctcag ttctttaagg aatttctata
15720

ctgttttcca tagtagttgt gctagtttac attcccacca gcagtgtaaa acgttacttt
15780

tcaccacatc tatgccaaca tctgttgttt tttgattttc aaattatggc cattcttgca
15840

ggagtaaggt ggtacctcac tggttttaat ttgtacttct cttacagtga tgttggacat
15900

tttttcatag gtttgttggc catttgtata tcttcttttg agaattgtct attcatgtcc
15960

tttgcccact ttttgctgga gttatttgtt tttccttgct cttttgtttg agttccttgt
16020

agattctaga tgttagttct ttgtctgatg catagtttgt gaatattttc tcccactctg
16080

tgggttgtct atttactctg ctgattatta ttattattat tattattatt attattatta
16140

ttttgctgtg caaagctttt tagtttaatt aagtccctta tttatttttg gttttgttca
16200

tttgcttttg gtttcttggt catgaattct ttgcctaagc caatgtctag cagggttttt
16260

ctgatgttat cttctagaat ttttatggct tcaggtctta gattcaagtc tgatccatct
16320

tgaattgatt gtcgtataag gtaagagatg aagatccagt tttattattc tacatgtggc
16380

ttgccaatta tcccagtacc atttgttgaa tagggtgtcc tttccccact tgatgttttt
16440

gtttgctttg tcaaagatca gttgactgta agtatttggc tttatttctg ggttctctcc
16500

tctgttccat tggtctacat gcctattttt ataccagtac catgctgtct tggtaactac
16560

agccttgtag tatagcttga agttgggtaa tgggatacct ccagatttgt cctttttgct
16620

tagtattgct ttggctaagt gggctctttt ttggttccat atgaatttta ggattgtttt
16680

ttctagttct gggaagaatg gtgatggtat tttgatggga attgcattga atctgtagat
16740

tgcttttggc agtatggtca ttttcatgat attgattcta cccatccatg agcatgggat
16800

gtgttttcat ttgtttgtgt catctatgat ttcttttttg cagctgttgc aaaagggatt
16860

gggttcttga tttgattctc agtttggtcg tgttggtgta tagcagtggt actgatttgc
16920

ttacattgat tttgtatcct gaaacttcac tgaattcatt tatcagatct aggagttttt
16980

gaatgagtct ttagagtttt ccaggtatac aatcatatca ttggcaatca gtgacagttt
17040

gacttcctct tttccaattt ggatgccttt atttctttct cttgtctaat tgctctagct
17100

aggactatgc catatgattt aatacaatat ctttatgttt gcttacattt cttttgaatg
17160

tgaacggtgc catgtatgat ctgtaagtgt gtgtgtacgt tctgataaat tttcactttt
17220

tataatagat ttgtgtgtat tttgcagtaa ataaaaaatt agattagtat ttacatatat
17280

tttatacatt catgacataa cttcacttaa ccttttttct tttttttttt gatatttttg
17340

aggctctgag gtttgtcttt gaattttttc aaattgtttc aaacctccaa aattttttcc
17400

aatatattta ttgaaaaaaa tctgtgtata agtggaccca cacagttcaa acccagttgt
17460

tcaggagaca actgtacata cgtgtgtgca tagacatatt ctgtatgtga gaatagaaac
17520

atgtaagaaa caatgcacta ttttcaatga cttctctatg gagaaataaa tatcaaccta
17580

gactgtcagg aagctattct cctttcttcc ctgctccctt gcttccccca tcacatcctc
17640

ccagccacca gccttgacag tgactctatg gtttgattct gtacctcacc tcatcctgtt
17700

aggcggtgga aggagggctt cccttccacc aagcccctac tggggtccca tggagaggag
17760

gagctcagca gccctgacag agtgagttgg gccaggcacc cgagtcctga gcaactagaa
17820

aggggctggg gagagggcgg atgggcgctg ttcctgggtc ccttcccact gcctgccgta
17880

ccagccctgg tgtgagtggg cacctgggca agtatctcag gggcagcagg aacgaaacag
17940

gatttccact ccctccctgc ctccttcctc ctccttcaca gagcagagac ttcctatggt
18000

gcggaagggg ggccgtgggg gaggcctctc ctcccttttt ccctcttcat ttgacctcga
18060

gaagtaagga ctgaggctgc tactccaaaa gagggaggag aggaagaatt tggagaggag
18120

aacagggaag aattatagcc ataaaaagga agaaaaagag gaagagaagg aaaagtccca
18180

ggggaataga aggcgccaag gccggctccc atggccacag caaagatggc tgccggtgtg
18240

aggctctgtc agggccacct cctcttgttc tttgtccttc atcacatccg gtcccgccct
18300

cacctcggta agtcacagat gaaggggcag ctgacttccc tatccacata ttctcataga
18360

gcctgactca caaaaccgca gaagaaagtc tgtagctctc tcggctgggc atgcctgtaa
18420

ttccagcact tcgggaggcc gaggtggatg gatcacttga agtcaggagt ttgagaccag
18480

cctggctaac atggtgaaac cctgtctcta ctaaaaatac agaagttaga caggtgtggt
18540

ggaaggcacc tgtaatctca gctgctcagg aggctgaggc aggagaatca cctgaaccca
18600

ggaggcagag gttgcagtaa ggcaagattg caccactgca ctccagcctg ggtgacagag
18660

caagactcca tctcaaaaaa aaaaaaaaaa aagaaaagaa agccagcctg tagctttcta
18720

gcacatgctg agagttttta tttagatgca gacaaatgac gttacaccta atgacatcac
18780

acttcccctc cattattggt ttcgttgccc cattttccat tccatcatca ccagtatttt
18840

taatattaag atctgttaga aggtgcgtaa aaggaggatt gtcctcaaaa gggagatgat
18900

atcaagagtg gaagtaactg aaaattttta aagcaaaaag agaatatctg tattacactg
18960

atattgtcca ctttcttcct tttttttctt tttttttgag acagaatctt gctcactgca
19020

acctccgcct cctgggttca accaattctc ctgcctcagc ctcctgagta gctgggacta
19080

caggcacacg cccccacgct cagctaattt tcgtattttt agtagagacg gggtttcacc
19140

atattggcca ggctggtctc aaactccaga ccttgtgatc tgcccacctt ggctccccaa
19200

tgtgttggga ttacagttgt ccacatttgg cagaggccac agttggccac atttggcacc
19260

ttatttttga accacctgaa aagcaacaga aacaagtggt caattttaag tgtttttgaa
19320

ttagtgtaga gtagactggt gtatgctggg cctagtgtat tctacctaga attttaaatt
19380

taagctgcta tttcaatatc agagttgtat agcctaatgg ttcaagtgtg ctcagggcta
19440

ggagtttggg cagagaagtg cccacagata acagctgaga actctagcca gccagagaga
19500

ggttcttaag tctctaaaac agtggtcccg taaccttttc agcaccaggg accagtttca
19560

tggaagacaa tatttccatg gatggaggtg ggagggaagg ggggatggct ttgagatgaa
19620

actgttcctc cttacatcaa ttagattctc atcaggagca cgcaacctag atcccgcgca
19680

tgtgcagcag ttcgcaatag tgttcgtgct cctaggagac tctaatgctg ctgctgatct
19740

gacaggaggc ggagttcagg tggtaatgct tgccccccgc cacttacctc ctgctgtgca
19800

gcctggttcc taagaggcca cggactggta ccagtctgca gcggggtgtt agggatgcct
19860

gctctaaaaa aactgacatg ttggtaaatg aggataatgc cttagataaa tgaggcccta
19920

aagacctagg gtggttattt aagtccccta cctcctccga ctcattcatt cattcgttct
19980

ttcattccat atatactact tgaggatcta tgttttagga tttcttaaaa ttaggcatgg
20040

gctttatctg agccagaaca tatttgtttt ccttctttct tttttttttg atggagtcta
20100

gcactgttgc ccaggctgga gtgcagtgga gccatctcag ctcactgtaa tctccacctc
20160

ccgggttcaa gcaattctcc tgcctcaggc tccccagtag ctgggattac aggtgcctgc
20220

caccacgccc agataatttt ttgtatcttt agtagagaca gggtttcacc atgttggcca
20280

tgctggtgtc gaactcctga cctagtgatc tgcccacctt ggcctcccaa agtgctggga
20340

ttacaggtgt gaggcaccat gtttggcccc catattttgg ttcttttaaa agcaatgaga
20400

tagaaatttt aggacagtga cacacaaaga ttgaaaaacg gttttcatat cctttttaaa
20460

tccccttcaa gggcaattaa tgcctatact acttggtggt gtggagaaga caggggtatg
20520

gtgaggataa gggagggaat caggggttct ggttttccat attcaatctg agacacctgt
20580

cagacatctg agaggtgatg tcaagctggc agttatccaa gatcaaggga gagatcagga
20640

caggagctat aagtgtggta accatcaatg tacgaataac atggaaccca tggcagtgac
20700

tgaaattgcc ttgggaaggc tgaggactga gtcctggaac acagcaaaga agattgtgag
20760

ggagagtcca gtgagatcaa aggaaattag tgcaagtgtg gggacctgga agtcaagtgt
20820

tttaaggaga agtgatcaac tggttaaccg ctactaagag gtcaagtaag atgtgactga
20880

gaattgtcca tgggatttgg atgaaaatgg tccaggacat tgtcaagggt agtgtctgtg
20940

gagagttgca ggtgcaaacc tgattgggat aggccgagga acaaatgaga caggaagaac
21000

tggaaacatc aagtgtgaaa agtggagtgg atgctgcctc caggagatgc ttctcttatg
21060

gggaatgatc ctttaatttt ctcgtctaaa tctgagtgac tattttctct aaagagcttc
21120

ctcctgttct gtctgtactg cagcatagat taaggcactc tgaaattcct ctctttttgg
21180

tggttatctt tgaggttgga tccacaccaa acaataaatt cctccctaga cagaaatctg
21240

gacactgtga cactccctga acaaggctat gtgttcatcc cctgcctggc tgcagagctt
21300

gacgctggag cactttcatg cttcctatca cagggcctaa ggtaaaatgg ggctggatta
21360

tatatatgca gtgttataaa ctgccagatt ttattttgtg tgaatttaaa tattttccta
21420

aattctgttt gaggtagaca gttgagttca ttgcaatgcc taaacataaa gtcagtctat
21480

agattttatc ttctagaaat agggaacaaa tctggttgtg cattctactt cagaagagga
21540

caatagagtc ttaaggagcc tgtgaatgct cagggcgagt taggggtttt tgaagtgctg
21600

tctctcctgt ggataaggca acaatcccaa gtgagatcct caggttttga ctgacaaggc
21660

tcctggcacc tggcaggaac ccctgcagaa ggcagaccac ctggagctat ctctgcccca
21720

caccttcctt ttcttcttct ttcttaaaag agacagagtc tcactctgtc actcaggctg
21780

gagtgcagtg gcaaaatcat agctcactgt aatcttcact cctgggctca agcaatcctc
21840

ccgcctcagc ctcctgagta gctaggactg cagatgcgca ccaccatgtc gtgctaattt
21900

taaatttttt gttgttgttg ttgagacagg ggtctcacta tgttgcctag gctggtcttg
21960

agctcctgcc tcaaataatc ctaccatatt ggcctcccaa agtgctggga ttacaggtgt
22020

gagccactgt gcccagatgg tgcttgtccc ccacactttc tttacaaaca taggtaatta
22080

aagacatata aataggatac tcttctgact tttgtgcctt tgaccctgtt agtgttactg
22140

agcaatgggc tcactgtcta acacacatgg aaaccaatac catgcagagg ctaatgagaa
22200

agaaaaagct ttcttgtgaa tcaactggca aggacagggg aggcgatgct caaatcgtct
22260

ccccagttgc aggtgtgggt caagcttctg tggcctttct tactggtccc aggtgatgtc
22320

aatgcagctg gtctgccagg ctggtggtgg taacaataag gaggttaaca cttttcccat
22380

tgcacatgcc cgggcaattt tggctctgca ttacctgtga caacttaagc aatggctaat
22440

cagtctcagc tggtcccaca gttacactgg cataaaccca agtactattt aggagtaaaa
22500

tgaaaactca gccaaaagta agtaaaggca taaatacata aatagtgcca aaaaggagct
22560

gagagcctca aatcatagaa gactattagt cggggttgcg ggggattgcc ctcagaaatc
22620

atccagccga gcccccatat tctgcaggag agaaaccaag ggccagggca ggaagccctg
22680

agctcagccc tcctttgccc catgatctgt cttccgcaga aggcagcatc tggcagagaa
22740

aaatgagaaa cctgcccaga gagatcttgt gtaaaacccc ctgattgtca tctttccttc
22800

tttaattttt ttttcagttg tgaaagatac atattctgaa gaacgtatat ggcaggtatg
22860

agtagttccc ccctcctgct tctggttcta catttttact gggaaaataa aatcctcagc
22920

tgggtgcggt ggctcacacc tgtaattcca gcactttgga aagccgaggc aggcggatca
22980

cctgaggtca ggaatttgag accagtccgg cctacatggc gaaaccccca tcactaataa
23040

aagtacaaaa aattagccgg gcgtgatggc atgtgcctgt aatcccaggc actcgggagg
23100

ctgaggcagg agaatcgctt gaacctgggg ggtggaggtt gcagtgagct gaaatcacgc
23160

cacttcactc cagcctggga aaaacagcga gacatcgtta aaaaaaaaat tcctccaggc
23220

tgcaaagaat gaggcttttg aatttctagg ctaggaagtg cgtgctggtg aaatgagacc
23280

cttcatgaca tcatgcaaat attaatgccg acccacccgg ctggcctgtg tagaaccacc
23340

acctcccagc ttgggctggc ttctggccac tcacagcact gctggcagtt cctttgcagg
23400

tggggatgag gcccagggct agcttggccc acccaagccc aatgccccct tgcagccagc
23460

tcagcaacta actggcaggg gccttcttgt cattgctgtc gctgcagcat cttcctgcaa
23520

gaaattccac aaggttacat cctgcaggca tgtctgctaa ctctacacag cctaatccaa
23580

acatttaaag gtgccatgtg catgtaaact gatcattcaa ttcattgggg aaaaaaaaat
23640

tttaaaccct agatcctgac tgtcagtccg catagtagaa gcccttttat ccaaacatta
23700

ttgactaagg gatcattatt gattagctgg tcatctaatt agaaaataat tcaaatgagc
23760

ggatggtata atcctaatgg catgacagaa gtgacccttt cattgattct taacaagctc
23820

catggttctt attggcaaaa tggagtagtt atccaaatgg gtggggtgcc cacacagctt
23880

ccttttcttc cctggggttg gcacagatgc caaccttctt gcccagtgct cttgagtccc
23940

agagcagttc gaggctcctg tctctcaaac ccacttgctc cttctctgtg gctaaacttt
24000

cccttagaat ccacctatga cccagcctcg atggatcaac tccccccttt cactcctagg
24060

cccatttcca ctcctaggcc cagcagccag gccaaagccc agacctgagt tcactggcct
24120

ggggtggggc agacaattca ctcctcacgt gctagactta gggaagcact attttgcccc
24180

cagataatca gcctggcctc ccaggctcaa ggttggccca tggcctggtc ctgctgctcc
24240

cctagatgcc ctccaggggt ggagatgcaa gggatgagac ctgtgatcgg agcagtagaa
24300

taggtctttt tggcaagcag agctctaaca actagaggaa ctgtgtctgg gcatcagaac
24360

ccaccagaac cacacgcgcc agctcaccgc gggcaacttc tgcagcttct accacactga
24420

gatgggcaac acggcctaag gccttcctgt acttcctccc cactggtggg cttatatcag
24480

ccacattcat ggccaagttg gcgaggatga gctgggaggg aagtgttgcc tctggtgggg
24540

agtccaatcc tgagcggggg acacactgtc ctcctggctg cactgtcatc aatctggatc
24600

attctcgttt cttcctctgc catgtgacag tgtgctagat gggggtggag cacctgctgt
24660

cacatctgtc tttgagcatg accatctgaa ttactttggc caatgggatg ttagcaggca
24720

caacatgacc agaggcttga cttacacgtg cttgtgggga ttgacttgtc ctcttgcaca
24780

ttgcagggtc tgctctttct cttcatctta ggctacctct ctgttgcaat agcctggcct
24840

aggagtcgag gtaggtgtgg gaatgtttgc ccacttaggg cctcagctta ttttctagtt
24900

ggctgccgct ggaccctccc tctccagctt ctaccccctc taagagtaga gctccttgga
24960

gttgtttctc tttgtggagt gactcctctg tagcttcctt ccccatttcc agggctgctg
25020

cttccttttt aagtctgatc ccacatccat gttgactttc agaatgtgaa aggtttgcct
25080

tatctactga acttttgtct tgtagctaaa atcacactgt ttgcccctcc tactcgcctg
25140

tcaaataact tcagtgagac agtattccac agaagcacct aagcagacca aacttttgag
25200

ggtcatagca gacagtagtg aatagattcc tactaaggga gaaagcaaca aaggaagtga
25260

aattttaaga aatatatgta ttttcaaaac tccacatacc agtttttcta cacaaaggta
25320

atataattcc aaagagtgtt aaatccttct gacatcagga cgtttgcaaa cacatgtgtg
25380

gtactcagtt aggcactgac aactcttccc ccagcagctc actgacaaga actggtttga
25440

accaattaga catgtgtgtt ctagggaaat tcactccttt gcaagcttaa agaaaaatta
25500

tagacagttt actcttgctc tacttagaca agacaaattc ttagtaagtg aatgaactct
25560

tttagtgtaa attagacaca tttggaaagt tgctttggcc aagcacagtg gctcatgcct
25620

gtaatctcag cactttggga ggccgaggtg ggtggatcac ctgaggtcag gagttcgaga
25680

ccagcctgac caatatggtg aaaccctgtc tctactaaac atacaaaaat tagccaggcg
25740

tcatggcatg cacctgtagt cccagctact tgagaggctg agacaggaga attgcttgaa
25800

cctgggaggt ggaggttgca gtgagccaag atcatgccac tgcagtccag cctgggtgac
25860

agagcaagat tccatctcaa aaaaaaaaaa aggtgccttg aacttccatg agcatgtgat
25920

ctatgtagct gcgcagagct ccagaatggt ttaatgcttt gggtttaatg ctctgctctc
25980

ttgagtatag aattcttaaa ttttgaacag gggggctctg catttttgtt ttgcactcgg
26040

cccctcaaaa taggcagccg ttcctacttt taggtcacga ttaagaagag aaaggttcta
26100

agtgtacttt catagctttt gctctctgtc taatctcttg ttaattcaca tgcatggcta
26160

gaatctactt tgagactgga accgtcttcc acagtaaact gagcttgtaa aactttgccc
26220

cttttaattg tggtgggaag ggtcatggga gttgctctgt cttagtggtt tttcatggcc
26280

agtggaaata taatattccc tcccacctcc cctactctat tcagagcagc ccacagccat
26340

gggtttatgg ccaccactcc aggcctgctt ttcacctctg tcctcccaga aataaaacaa
26400

acggtgagat tttgctcttt taggggaaat gacaagttca gcaaacttaa gactgcattt
26460

gtggccatgg tttgaaaaga aagcccaaaa agtgaaggaa tatgaacatg gtgggcagtg
26520

gtccagcccc aaggatggtt aatttgagac gtggggtggc tgggacctga gatggccacc
26580

agcaaactga ctccaggtga gatggatgtc ccgtccctga atcacaggaa gacatcccac
26640

ctcccaggat gcctgctaag gatgtgggac tgtgttatga atatttaatc agtgactttt
26700

aatggtttgg tttttatcat ctctcttatt taaaaataat tctgacttca ttcctccccc
26760

caaatataaa taaaagcaaa atagcgctga gagagcattt gctgtggctt cttaatcatt
26820

tttctcgttg taatgcccaa gactcagtgc tccctgggtg tacttgaggt tagaaagatt
26880

ctctaaagtg ctccatcctt cctcattccc ctccctcagt tcctggcctt tgcaggaact
26940

aaagttcgtg cctcttttac tggaagattg tccaagtgat taattaccat ataagaatag
27000

agttagccct ggatctttag ttcccccacc tcctgcaaga cattgagtat aattatctag
27060

agagttcatt aaatgcatga tagttatgct agttctccct gaacagagac aaatggtgag
27120

aaatgattaa actgctcgcc taactaataa ctcatggcga caattttata cggaaagtca
27180

cataaggagt gacacagcat gaatcaagaa taacctctgg aataaactgc aaatagtagt
27240

aagtattgaa gaaaagacaa catttaacca tctaaactgt cctgcagtga cctaccccta
27300

attttgcctc agttgatcag taacagctga catttatatg gcacttcact ggttataagt
27360

gtcttgtagg tacacaatca tatttttcat gatctgagaa aatccaaaag ggtaacttcc
27420

cactggaaat cctcattgtg gacttggaca ataaataatg aagatgagac ttcatcatag
27480

gaaccgagtg actggaatgg cagtggggac atggcagtag cgcctggcac aactgcacct
27540

acatattcat cctctgcaaa actacccaac acgcggagct tcttcaccct tcctggcttt
27600

tctctcttct acctctgggc ctccctcccc tcagcctgtt aaatggccaa cttgccaccc
27660

ctcccctcct cagctttagc tttcagttct tattgctgcg tctccctgct ttccagagct
27720

cctgattaca caggagcgac atctaagcaa ggcccatcac tctccggacc acatggcccc
27780

tttggttgcc tcggccttgc tgtccaacgt cctcttcctc accctccttc ctctgagaac
27840

actgcttaaa gcaggcagct ccatgcacat accacggatg tgtcttctca ccttgtgcct
27900

ctgcccagag caccttccta tctggagcaa aagttggcac acttttgctg tcaagggcca
27960

gacggtaaat atttgaggct ttgcaggcca cacagtctct gttgcaacaa atcaactcac
28020

aaaagcagcc acaaacaatt cagaaacaaa tgagcatggc tgtattccca tacaactcga
28080

tttatagaaa ctagagttgg aatttcatgt tgtcatatgg cgtaaaatat tcttcttctc
28140

ttgatttttt ttcaattatt caaaaacgta aaaactattg ttagcttgtg ggctggatgt
28200

ggtccagggg atgtagtttg ccagcccgtg gcctagacta ttactagtgt ttcacagcca
28260

tgtcacatat cactttattt ggtaaacttc cctgagtgtt gggcatttta agcaaaaggc
28320

cccctgtcta tgctctgatg gtgttacggg atctttgggg tgttactttt cttgctggaa
28380

acctgtggcc ggtggtgtct ttgcccgagt tttgctttgg gcctgctggg tttgttccac
28440

ccacttggcc atgtaggctg tgctcagctc atgctaccgc cctggatccc acacctccaa
28500

gggagactgt gagtcaggca tggagcagcg agggctgtgt gagcaagtga ggggtccagc
28560

cactgtgcag ttggacacac cggctgctgc catgatgcgc gcagctccag gtgccagcat
28620

gggtgccagc tctctgcaag gctgcagctg gaccaggcac accacaagca gcttcccctg
28680

atggcaccgg gaattgcgat ggtgcccaga agcttggaga taccaggaac cacagggttc
28740

cagagaggga atcacagccc tggctcgggg agctcacagg tctgggcccc ctgaagggcc
28800

acagctcttc tccccttctc ttcacttgca acatggtgag caaggggcat atttcaactc
28860

tgtgtgtgtt acagctcttt tagccctgcc atttgatggg tcctgagttc ttgtcctgca
28920

accaggaaga atgagtgatg cagaaaagtg gagggtgtgc aagacaaaga ggagctttgt
28980

tgagcaatag aacagcttag aggtgacatg caggggttag gccctttcta cagccagggc
29040

atcccaacga gtgttcagct cctagcacag aggagaccct ggagtgagaa gctcctctct
29100

gcaggcaggt catcccatca tctctgcagc tctcagcaga ggcagcactg gatggggtca
29160

ttcctgtcta catgcaggtc gtcccatcat cttcccagct ctcagcatag aggaggtcct
29220

gagtgggtag cttctctcta cagctaagtc atcctgatgt ctgctcggct ttggcagagc
29280

ctggggcttt tataagccac agaggggaag aagtgaatgc caattggccc atgaccatgg
29340

gtgggcctgg aaaaggaacc acaagttccc actccagttg gcgggactgg cagcccagcc
29400

cacagccttc aggccctccc tgatgtgaag gtaaggcctc accagggact tgcccccctt
29460

ccgccaagga acctgtctgc ctcctgtcga tttttatggt gccaggctgt aggtgccaag
29520

gggcacctgt aggccagtgc cgagctgccc tcagcacccc ttggcttcac acctatactc
29580

gttggcaccc aaagtccaga gggggccaag gtggaagggg ccttgtgtgt cagcactgcc
29640

ctaagcgtgt gcacacccag ccaggctgta acagccctgg actcagccct gactttgctc
29700

catgatcaga gcaggcactg acaacaggga gaaaccaagc agcaggagca gatattttgg
29760

agcctgtgag agcagggttg gccttcatgg gcctccaaga gcacagagat gtctgggtct
29820

gcagattgat ttgggtggct gcagctgcac cctgaggggt ggggctcctg cctgctccat
29880

ggagcaggag gcctgggtct acagctgcaa cttgggtaac tgcagctgcg cctgggaagg
29940

tggggttcct gcctgctcct ggcccctcca agagcacagg gatgcccagg tccacagtca
30000

tggcttgggt ggctgcagca gcacccaggg agctcctgcc ccaacttaga aggggtgggg
30060

cacctgcttg tccccagttc ccactgactc catggagtgt gcatacctgg ccgcacctcc
30120

ctgctgcagc cagcatgatg gcagcggctg ctccagaagg cctgcccctg ccatcaatgg
30180

gacccaattc atacttcttt gtcagctctt tccattcgat actgataatc tgcttacatt
30240

tctgactgtc taccagcaac taaggccctt acgggtaggg ctggtgatcc aggcctggcc
30300

tgctgctccc agcagccact gagtggaatt gggcactctg ggatggtgtc tgagccatct
30360

tctctgattc gcctggggcc ctagaggaca acagatcact gatcgtgaga aaggggcatg
30420

tgggccacag tgggggccag acccagtcac ctgatctgga gagaaggagg cagtcattat
30480

ggaaaacatg taccatggtc tgaatgagtg tgtctcccca aaattcgcat gttgaaacct
30540

aatccccaag gtgatggcgt taggagaagg ggcctttggg aggattagag gccccagaga
30600

gctaccctgc cccttctacc atgcgggaac aatgagaagt caccatccat gaattagaaa
30660

gtaggccttc accagacaca gaagtttcta gcactttgat cttggacttc tcagcttcta
30720

gaactgtgaa aaataaattt ctgttgttta taagccatcc aatctatggc attacagcat
30780

aggagcctga atggattaag atggcgtaaa acgatgcttg aaacatgaaa actccgggaa
30840

ggagagtaag tcctccaagg ggggcatgtg aagaggaggg catttccctg ttgtgggagt
30900

cttctaaggc ttggaggtag tttcctgcct cagagtaacc agcactaccc caaacccata
30960

ggctcagaat ttcccacagt tcctggggat ctgcatttta agtaggtaca ccaggtgatt
31020

cttctacaaa tcacagcttg agaatgaccc tgccctgctt gagttcagac tatttggcgg
31080

tctctgaaga ttgcttcggg ctgcatcaaa gttagggaca ttggggtaat tgcctgttca
31140

gcactcttat agctctagca ggaagcacag atatcccaag gactcccagg ggtccaagaa
31200

cacactggcc accatgtgtg catccacaga gcagtagaac acagtgcatc catgcaggtg
31260

agctcagcaa cggtgggaag ggccggagca gcggcaagac accggtggag aacaagacat
31320

gaggaatgtg aggagtaggg acagcagtgg ggtagacact aagccagcag atataggaca
31380

ggcctaactc attaatcgag ccacctggaa aggtcagaca catagactct gcagtcagcc
31440

tctctggttt gagtcctagc tccacttctt actagctggg tgggtgacct tggacaggtt
31500

atttcatctc tctttgcctt agtgcctcat cagtaaaata gcgacaataa cagttcctac
31560

ccatggattg ttgtaggata aaatgggtta acacatgtga atatatcaga acagtatctg
31620

agatgcagtt ggcatcatgc tgtcattgct gtcattgtta ttaccatcaa gaatccacag
31680

ccagtaggac ctaacaagca tttatgctac caaattagtg tttggcaaac agttgttcaa
31740

ctgcactgaa ctatgtcaag ctgcaacctg ggtctctcct tgcccagcat gtaccaggct
31800

ccaaaactaa ctctaccctc ctcttggggt ctcgactctt cacagagaaa aatgattgac
31860

ttcaggtagg cttaggaacc aatatggatg aaacgcagct tctagaattg tactaccccg
31920

actctgccat ttatagtata gtcaaactat ttaaactcca taagacttca ttttcttatc
31980

tgtaaacatg gcaataacct ccagcatatg gttcttgtga aaatttaaat aaatgatatt
32040

ctatttaagt taatgatctt ttcttctcaa aaagaaaagc aatctagcta gggttattta
32100

tatggtaatg aacttaatct ggttgaagtg ccagtatttt tatgaacatt ttaaaaagca
32160

gaaagcgaat ctatttaaat catatatatt tctaattggt gggataagat caggggtcc
32220

cctcttttgc tttttgtctg ctgttaaccc accattccaa aacccacaac aggagaacag
32280

attgtctgga tatactgtat ttattttagc tgttcctatg gaataaatta cttctgaatt
32340

tagtggctta aaacaacgat aaacatttat tatcttacac catttctatg gatcaggaaa
32400

ttgggagcag cttaactgag tggttctggt tcagggtgtc tcaagaaatt gtagtcaaaa
32460

gtcatctagg gctgcagcca tctgaaggcc taattagagg tggaagattt gattccaaaa
32520

tggtgcactc acatggctgg caagtcggtg ccggctgttg gaagaaggct tcatttcctc
32580

atcatgtggc cctctctgca tggcttcttg agtgttctta caatgtggca actggctttc
32640

ccaagagcaa gtaatccaaa agaaagcaag ggaagctgca atgactttag tgacccagtc
32700

tcaaaaatca cactctctca tttcacagta ttctattggc tgcacaagtc agccttattc
32760

aaagtgggag gagatgacac agggtggaat atcagcaaca gaatcattgg gggccatctt
32820

gtaggctggc ttccacattt acaaaggact taaagcctgt tttcttaagg actagattaa
32880

atcaactttt aaaatatgct gttttacttt aaaattccct tggtcaacac ttcaccaaga
32940

ttctttagtt taaacaaatg ctggaaatag gaagttaatt cccaaggcca tattttgctt
33000

attcctgact gggtatggta tgagtgggcg gattcttctg ggggagtaca tcaaggagtg
33060

agacatggca tttctcaccc tgaaacaaat agtttagtat cttctcaaac tttgtattgc
33120

tggcacatgt gtgctaaaag aaaaaactta gacaaattaa atttaacacg gtttagttga
33180

acaaagaatg atttgcaaat tgggcagcct cccaagccag agtaggctca gagactccaa
33240

cgcagccatc tggtggaaaa gttatggtca gaaaaaggaa agtgacacac agaaaacaga
33300

agtgaggtgc agaaacagct ggattggtta catcttggca tttaccttat ttgaacatgg
33360

cttgaacagt tggccccctt tgattggcca aaactcaatg attgtcacag aaataggtta
33420

cagtctgttt atacctccat ttaatgtata gctcaccata tagggagaaa cctttaggct
33480

aaatttagaa tatgtaagga ggcagcttta ggctaaactt gatttaacgt gtgtgtatgg
33540

tagatatcag aatactttgc aacaatatcc atgttctctt cttcttgaga taggtccttg
33600

ggactagttc ttatgaatgg gatgtaagca gaaattatgt atgtcatctt cagactgagg
33660

cggttgagtg gaatgtgatt tatccatttg ccctctcttt tgtcacctgt tgataggatg
33720

caaagcattg gtagagggaa ccatggtact agaagatgac agagcaacca gtttgaaaga
33780

gcctggatcc ctgaatgaat gtatggagca gggctctctc tctgttgaat ggtgatgaga
33840

gtgagaaata aaccttcatt gtacaaagcc actgaaatgg tttttgttta ctgcagcaag
33900

tattacttgc tgctactaat gccaagtata aatcatattt aaatctttgc catcctggga
33960

ttcaatttaa ttatacaaaa ctaaattcat gtctacttta atattcatgt tttctcatag
34020

aagataccat tttttaaata ataggagcgc caagccccag aacatagctc taaggtgaaa
34080

atgtataatg tcataactta ttacatgaca tctgggtcaa gagaatagag aagacggtgg
34140

ggtttaaaaa taaaaagtaa ataagggtta taaaaaatgc aatactatat aaaaattgat
34200

ccaatgtatt caaaaattaa agactctcta tctcttaata aataccaaag taccacaaga
34260

ttctttatat acaccaagga ctaggcaagt agaaaaaaca aacaaacaac tttaacctta
34320

taattctaga atgaaggatc atctccagag tcctcagtag ggcgcttata aacagctcct
34380

aaaatcttgt cagcacaatc aagggctgtt tctcaatctc tttgtataaa aaactagatt
34440

ctgtattagt ctgttctcag gctgctaata aagacatacc caaggctgcg taatttataa
34500

aggaaagaag tttaattgac tcacagttcc atgtggctgg ggaggcctca caatcatggc
34560

agaggacgaa gaggaaccaa gacacatctt acatggaggc aggcaagaga gcgtgtggag
34620

gggaactgcc cttcataaaa ccatcagatc tcataagact tactcactat cacaagaata
34680

gcacggaaaa ggcccaccct cccaattcaa ttacctccta gggggtccct cccactgcat
34740

gtgggaatta tgggagctac aattcaaggt gagatttggg tggggacaca gccaaaccat
34800

atcagacttt attcattttg gttcttcctt tgaggaaact gagtcccata aggaaatgga
34860

ggaaaacatg gccagcagtg ccaactctca ccccattcac ttcccgccct caagggtgtg
34920

ctgccctttc cttagcacca cccactccag ggccgtcacc atcgcagtgg gggctctccg
34980

tgtttcctga gatctcaaac ccaagcgttt cttggggcag cagcgggggg cctcgccatg
35040

gaactcccgg ctcccacgag ggcttccgtg cctgctggct cttccggctt tgagcgtccg
35100

cacgagaagc cccctgctgg ccacattcat ggcgtgggga cctcgggcta aataaatttc
35160

atttctctct tctccctcct aagtgtcttc cactattttc ctttgttggc tctggacttg
35220

ttctaaatgc agccctacta aactttcggg gggcctgagt gtttctggca ttaaggggtg
35280

cagtggggcg gggggtgcag aggggctggg gctgcgctgg gagatggcgt tgtgttaagt
35340

gaacgtggac acagtggtat ttacagcctc actaacgtgc cttgtcttgc tgccctagcc
35400

tctgactgtg aataaaacac ttctcctatt ttttttttta agacaaatga caaataaagc
35460

tgggtgaaga aagtaatcat ttactttgtg tcttcggaca tgacatttct ttcctaacat
35520

ctttcggaaa gctggagttc cctaatgccc tgctaaagaa aatgattgac atctttcaga
35580

gggctaggaa atttatgcgc tggaaaacaa tttccaggac gttggagatg agtaatttcc
35640

ctctctatca ctatgtgtac aaaagaaaag ggacccaggg cgctgatgta actgtatgaa
35700

aaataaatgt gtggcaaagg aatagtaaaa atacttgaac tgaaaacaaa gagaagtaat
35760

cattttttat agtttggctg tagtcactga acaattgatt tagatgacta agtctaacac
35820

atctgtattt acacgatctg gaagacataa attactaaca cgtagaaaaa ttttggagag
35880

gggaagtaaa ctacagctta agtatctgat gaagtaatct agcaagactc actttttcat
35940

ttctgctgaa gtcttgatta catctagcat gactgtgcca tctagtggtt tcacagtaac
36000

tgcctcgagt tggaggatac agttcggaaa attgctttga atatcccatg aactcttggg
36060

taggtgcaaa agtaattgct ggttttgcca ttaaaacagc aatttctttt gcacaaaaat
36120

ggcccaataa gaaagagatt gaaagagtat ttccacggca cccacacgca gctagttctg
36180

aattacttaa gccaatcatg gcatgaaagc tcatgaactt cacacaaatg cctcaattca
36240

aaccactgat ttcatcatct aaaagcatct actcggtgtc aaaatgcgta ttgtggctgt
36300

atatcaaaga caccatcagg gtctgtgggc tttgtcactg tccccagatg tttatctgct
36360

gaggagagga gatgcaggct tctctttcct ctgtttacaa ggtgctaaga gatactgaga
36420

atatctgtag caactaataa gatcacagaa gaacccccaa actcaatagc catccatcaa
36480

ccaaccaaag gctgatgtta ttcacacaat aacgttgact tccccatatt tcttgctctg
36540

aaattttggg gatctttgat cattttagct ttaaagttgg agcccagcca caccagcttg
36600

tgatctctct tcctctacct ggaatcccct gctctctcat ctgcgttctg gaggccacca
36660

gtgcccacct aaggttgttc ccagctcgca agtatcccca gttctgtgca gttccctgct
36720

gagccactca cattctcaac tgcggggacc tcaatggcaa tcacagtacg agagccacaa
36780

gaaactcagg gaagaaaaat tagaaaacag cctccagatc tccagggaag gaatagtcat
36840

agtttatgtt acaagttgtt ttgaataact gcacacttca tgtagatcta taaaaacgtc
36900

aatgtacaga gtggtaaaac agctaggcta agaggggagc aaggcggggc cagaaccctc
36960

tataaccttt ggaaatgtta gagaaatgga aaagtgccat aattttttaa acataattct
37020

acttattcca aatcattgac acgattttga gatataattt acattacata aaattcacca
37080

ttgaaaagag tgcagccgag tggtttttag tatattcgca aagctgtgta accatcacct
37140

caaactgatt ttagaacatt ttcattactc ctaaaataaa cccatatcca cagcaatcac
37200

tcccaatgct cccttcctgc tccagccctt ggaaatcaca aatgtacttt ctatctctat
37260

ggatttgcct attctggaca tcttacacaa atggaatcat acaatatgtg gcctttggtg
37320

tctgacttct ttcactcagc atgttttcaa agcttgtcca tcttgtagca tcacagtact
37380

ttctttcttt ttatggctga atgatattct gttgtatggg tataccaaac tttacacatt
37440

cattaattga tggacacgtg ttgtttctac ttttactatt ataaataatc ccactatgaa
37500

cattcttaca caagtttttt tatacataat tttttttcag tttttgtaca tgaatatttt
37560

caaatgctct tgggtatgta tatctaggag tggaatttct ggtttacatg gtaattccat
37620

gttgagaatt ctatgtgaca attctttaac tctgaaaaac tgtccagttg ttttcagcag
37680

catctgcacc attttacatt cccaccagca atgtatgagg gctctaattt ctctatattc
37740

ttgccaacac ttgttatttt ctgtgtttaa aaaatatatt accattatag ccatcttagt
37800

agacataaag tggtcattgg ttttatttta aagagcaaaa attaatgcaa atacagacta
37860

gaaaacaaat caatgccctt tggagattga aaagatccca ttataataaa gtcaactaca
37920

tgcttctgtc tcacgtagca cagaggagaa taattacaca gcctgttcag gtacagactt
37980

tgctactagg gcatttgtag gatgagggtg tgagtcagaa ctgtcttcat tcatcccact
38040

gatgcaagaa gtttctctac aatttatctg acagttaatc atttcattcg atttatccag
38100

tggcaggaaa ctcttttctt accattttat ttataatttc tagaatgccc ttcttgacat
38160

tttcaatttg tcatcttata acctcctgta aagtttaaaa ccaagtgaca aattttaaag
38220

tataccaagc cacctcccat gtcctcagtc tcaatatttc tttccatgaa gccacctccc
38280

atgtcctcag tctcaatatt tctttccatg aagccacctt cacgtattaa ccttttctat
38340

aaagtcatgg tttccattct tatatcatct ctgacacctt cctctagata ctacaatgtg
38400

tcaatatcct tctaagcagc tatcctcaaa tatgaacaaa atgtgctgtg gccagtgggg
38460

aataccatga gacctttatc ggttgtggta ctgtgtttct cttaataata cagtgcaagt
38520

ctgcaggatt ttctgcaact atgtaagata gctcaaatag ggcttccacc aaatctgcta
38580

aaatcctcaa gactttttca aatgacctgc tgttaccctg ctgctcttgg ctagttgctt
38640

tttgaagttt atatttctcc atattaaatc tcattttaaa aattttagtc tataattcca
38700

gcttgttata atcttttgga ttcacctcac catgccagct ttatgtcagt tacaaattag
38760

aaagatgttt ttattgatgt ttgatcaatt ccttattatg aaccatgtgg aaagcctcct
38820

ctccagataa gcatctattc taggaggcag caccatccag cagcaatgac agaatagctg
38880

ttcacttgtc tgcctactat tacattgaaa tcttactgtc catgttaaat actagtcact
38940

taataaatat ttgtttaatt gaattgaata atttcccttt caaatgtcag cctagagctt
39000

tgttgatgtc ccttgataaa aatcccactc tttagctatc ttaagacatg gttaaaaaaa
39060

aaaatcaatg acactttctg tacagctaac aattcacttc ctaatatatc ctccttcatc
39120

tttcaagttc tcataaaaat cacttacatt ttttcaagct tttttatgtg ccatgagtta
39180

tgccaagcat tttatgtaaa ttaactcatt tcatccttac atcgaacctg taagttccat
39240

ttttattttc attctaggta tgagaaaact gaggcacagg gaatttaagt aaatcatcca
39300

gggtcacatg gtcaattgct tagcagagtc agaatctgaa cccaagtccg ttccaagcta
39360

gagctagaaa gcctttcatg aagttgtgac catcccctaa aaaaggagag aaactcctat
39420

tggtgatatc ctactgacag cattcacttt tttagccaca tcccaaagat ttctgccagt
39480

aattgacatc tttcattcaa aagtgaccca caggggagtg gttgcctcag aaagaggagg
39540

gaatgaattg aaactgaggt aggaggcaag actttactcc aaactgggtt gaaggctggc
39600

tgaaataggg aggaagtgaa aacacctctc cgtaagacat gtattactca gggttctcta
39660

gagggacaga actaatggaa tatatatata taggagttta ttaagtatta actcacatga
39720

tctgtctagg aagacagaac taatggaata tatgtatata tatgtgtata tatatgtata
39780

tatatgtata tatatgtata tatgtatata tgtatatatg tgtatatata tgtatatatc
39840

tgtatatata tatatgtgtg tatatatata tatgaggagt ttattaagta ttaacttaca
39900

tgatcacaag gtcccaccat aggctgtctg caggctgagg agcaaggaga gccaatccaa
39960

gttccaaaac tgaagaactt ggagtccaac gtttaaggtc aggaagcatc cagcacgaga
40020

gaaagatgta ggctaggagg ctaggccagt ctctcttttc acatatttct gtctgcttat
40080

gttctagcca cgctagcagc tgattagact gtgcctgccc agattgaggg tgggtctgcc
40140

tttcccagct cactgactca aatgttaatc tcctttggga acaccctcac agacacaccc
40200

aggatcacta ctttgtatcc ttcaatccaa tcaagttgat gctcagtatt aaccatcaca
40260

catgtccacc agtgccatgt caatttatcg ttgctatggc aacacccaga agttaccact
40320

cctttccatg gcaatgacca ggaatttacc accctttttc tagaaatgtc tgaataacct
40380

gccccttaat ctgtatgtaa tgaaaagtgg gtataaatat gactgcagaa ctgcccctgc
40440

tactctgggc accctgccaa tggggtggcc ctgccctgga aggagcagtc cctctgctgc
40500

tgctgtgcac agccgcttca gtaggagttg ctaacaccac tggctcatct ctgaagtttt
40560

tcctgggcaa agccaagaac cctcccaggc taagccccag tttgggggtt cacctgccct
40620

gcatcaaaac caagaagaaa ctaatgaggt attacccatt cacatgtaat gtcttgtcac
40680

ttttattttt aaaagaatgc aaaattacaa aatgtaaata tgcatttatt ccaagtgctg
40740

gattcctatg tgttgttatg ttctggatac ttgccttcat ttttccagtt accgaaataa
40800

tatttttaaa agcgaatctg aagatgtgtg gacattttga cgcatgtgaa attgctgatg
40860

aggctgcctt tttgacatag ctaaaggagg taggtctgca gatttttaca ttttatcaca
40920

acaaatttaa catatgtcag cacaatgcaa ttgaaatccc tttagaatgg ttataatctt
40980

catagtttct tctagaagat caatttacct tgcaacacta gggagaaaac acccagaacc
41040

ccttcaggtc cgctgattcc tctcagtagc tccaacaatt agcttctatt taaaaagcaa
41100

caaggccagg gcattaggga atggcctaat gatgagatct taatgtatgc aaatttactg
41160

cacatggtgg cagcaggagg aaattaaacc attgttgact gattatttct aagatacaag
41220

atcatgacgg ctttttttca tatacttttc atacatgttc caaattttcc acaggaaaag
41280

aaattatttt aatatatcct cctttgtctt tcaatgtttt aattcacttt gaagtttatc
41340

actgaatgca ttgacagcct taaacactat tcaattggca ggtacccttg aaagtttact
41400

gtgccctgat tgcagtgagt gcatcttggc ttctatcgtt ctgtctgttg cctacctagt
41460

acacagtcag cttttacatc tttctttatt ttatttactt gttagagagg gagccttact
41520

aatgttgccg aggctggact tgaaatggac tcaggcgatc ttcccatctc agtctctgga
41580

gtagctgggg ctacaggtgt gagccatcat ccacttgggc tcacatcttt tgtaatgttg
41640

tttcttcgtg tttttttctt cttctctcac cttctaaatt cctgtttcta tgtttctata
41700

cactgggaaa ttaaataagt gtgcttttta gaattgatgg taaaatagaa taaactaact
41760

ctgagtaaat gtttgaagaa tgttttgttg tggaggattg ttctgcatct ttaggatggg
41820

tttcgtgaca gttattttac ggatcttaga gggaaaaatt acatgggggt ttggagcatc
41880

tgaaccagag ggctgaggtt tctacagatc caaatatctg tcaagtgagt cattcttata
41940

ttgatctaaa ctctgcacac tatttgtttt acccttagaa agcctatgaa taaactatgt
42000

cttgctgctg aaactagtgc cttgaaaatc aaaactaaga cacgtgagcc attgtgaaaa
42060

aaatagattt ttttccattt ctgttcttta ttagatgatg aaagtcaatc ttctgatctt
42120

ttcagtccaa caatttatct ttagagcaag ttcacagaac aatttgtgtc ccatggttaa
42180

gtcatttgga ttcaacctta tagactgatt tttgaagagt gctctgtttt acaaactaca
42240

actaatatat tataattgta gttgttgggt tcttaagaca cacgttatat gattgcttat
42300

attaatattg tctcccaaga agcctgtaat cagatctatg ttgttttgag actgaagatc
42360

attgggaaaa gctttttttt tattttcaga gattattgaa ttgtttcaat tccattttta
42420

gagtggctgt ttatttccct tgctgtccat tgaattagag agagatgaca ctgactccat
42480

tttatagaaa catactttgg gttatttttt aggaataaga atagtttgaa ttgaatctcc
42540

aatatttctg ttcccactgt gactatcaca tctaatatgt aaatggtgct ttatggcttc
42600

aaaatatacc tgatctcatc tggttcttaa atgaagaaag caggcatggc tgatgttacc
42660

caaagaggag gaacacaaag gttttcttct gactcctgta gaggaggctt ctcaccactc
42720

tgagtggctg acagcattag tctttgagat ggctggtttt tccctcatta aagaatcttg
42780

gccaaggttg acttagcagt ttttatctgt atttgcttcc taatttttag gtctgataaa
42840

gaagtctcac ttcaaggctt cattttatga attaatgatt ccaaatgaga aacagtgaag
42900

agcacacata atttattata tagaagaaag ccaatgagct gtcagaatct ctccattcta
42960

aacttgactt tgaaaaagat agatctgaaa attggctaaa tattccagct ttctttttgt
43020

gatcaatcat tgaaatatgg ttcataaaaa ggaaaatggc atttttaaat catatctatt
43080

tgtatgtaag acttggagaa gttttaatac ctcaatataa tttatcattt tgaagtgagt
43140

catttatctc catggctatg atctatggct tcttctatgt ctggaatgcc acatgtcata
43200

ttaaaattaa gactgcagct ttaagtgatg aagaacaaac actggaatat tctttagaag
43260

agcctttcca cactcaggct attttcaagt gagcttatgt ttagacaact taacgaaatg
43320

tgatcctctt gaattaaggt ttatatattt acacacacaa gtgccagaaa aagggaaata
43380

gcttattaga acttgccttc cttattgttt ttaactcgcc tgttttttgt tttttaaaca
43440

agttttaata gtgccacctc ccttaattct tgaaagtaga catatttttt aaaataagaa
43500

atattcatgc aattaacaaa tactcatagt acctcaagta tggacttagc cattaaaacg
43560

catgtttaga tatgaatgaa gtcttaagga attaaagcta ccaccattag ctttcatttt
43620

aatgtcctgt attagtttta attaatggac atcctgtctc ttttaagcta tattttctct
43680

ccagctcctc ccccacaatt tcccgcttct aaggtcactg ccagagttca tgctggctct
43740

ggttgagttt ctgcctctaa gctaggaaac tctgggtttt gctttcaacg tgggaaaaaa
43800

atcacgcatg gatgaaaact ggcataagtt aaatcaccta attcccttag tccttaatta
43860

agggaattgc agaagtgttc atttaattac atttaaacac ctaactgaaa atcagaagtc
43920

ccctaacttc taagttcaca aagagtctgt gataaaagtt tcataaggcc ccatttcaat
43980

tttatgaact gaatccttgc ttgatacgtt ggggatctcg tatatttcat agagtcattt
44040

tttgttttga tggtgaatat atgttgttat gggatcacta gcccaatttt cattctttca
44100

gaaattaata ctataacttt gaaaggtaaa gaagagacca cacaggaacc agggaaaaag
44160

ctacctgaga aaccaatttg tagtgtcatg ggctttggag gagctggccc gacctgccaa
44220

ccaaatgact gggccaatgc actcaaaccc cttcaagcta ccctggcctg gtgctgggtg
44280

gccatgtcac aagctggtgg cagggccctg attctgcagg tgcaggacat taaccctgat
44340

ttgtgctggg gcagcagctg agcctggcag tctttgaaat gacattttga gacaaagtgg
44400

gggggcttat tttgtctttc ctacttcctc cttctagttt aattgagtcc ttgccagatg
44460

acacttggca ggatgccaag ccagcacttg actcttactg ctagaaatag tgtgtgtttg
44520

attaaaagct aaaaattgat gatatttgat ccctaagtcc aaatactgcc ccatggaggc
44580

atgccaaagc aggttttcta ttctgaggag ccttgctgga agacggctga gtacacagga
44640

ccatttagga atcttttcaa aatcacagtc ctcatattgt ttgccaagtg ctgttctaag
44700

atccctaaat gtattaactt cataatcctt acactgattc catgaagtag ggactattgt
44760

gatcactttc ccctgtctac ttgtagatgt agatcttcga tggaggtgtt gaaacacttg
44820

cccaagatca ccatttagga gtagggccaa gttgtctggc tccagggccc atgctcctac
44880

ccactagtca ctgtgtgcca gtgtcccacc gcacctccag tgagatgggt tcattcgtct
44940

gcagttcaca tgggtgcttt gaaatccggg aggaaaagac atcattcctc gttgcagtca
45000

gggctgcaag ggcactctct tttttacttc tttattgtaa atgtgctact ctcttttcta
45060

gagatactgg tgagctctag aaaaatgttc ccttctctta aaaaaaagtt tttcttaaaa
45120

caaagacaga gaaagaaaga agggaaaaaa ggaaaggagg gaacggggtt aattcagtca
45180

atgggaagca ctggtgggag gaagagaaaa cttaaggtat gcctcctccg cctctgcctc
45240

gggcggcgtt gttggctgtg gctgcattgc cctggcacca gcccttcttg gcaagctgtc
45300

tatggttcca ccttcctcca ggtgctccca gaccttgggc tctagtgagg cccctccagc
45360

cccgtccctc tagtgctggg taggaggggt cagggctaat cactcttgcc tcactctccc
45420

cacagtgcaa tcaattcctt gttttaaatt ccccttctga aaataattag catggtatct
45480

ctctacctgg ccagatcttg tctgagcggc cctgtaatct cttagagtgc agcagcgtgg
45540

ggtgcattta ttccttccaa gagcagaaga tgggcttaga aatagccttt caattcgttt
45600

tccagtccag aatttcacaa aagcataatg tttagtcagc tcaatgctac cagtccgtga
45660

ccaaaactat gttacgaaca cagggcgcca aagagtaaag aggtcacact aagcgtaacc
45720

tcttcctgct gccccaaact cctcacatcc cccccacccc acaacactcc ctgtaattac
45780

gtgggttgtg ttgtaactgg ttcatccagc tactaaatca gagagattca ggcactcttt
45840

tttcccaaga gttctccagc aaaaatgcag aaaagctttt gaaggcttgt aaccatccag
45900

tggtgtcatt caaattgcaa ggattctctg caatgacaac cttaccagct gatacctacc
45960

atggccccca ggagccccag gacacagagt tgaggtagac tggtctctgc tctctgagag
46020

ctgagagtct agcagaaggc ttggtccaag gagaagggca gccagcgtca taggcaggga
46080

gcccaagatg ctcagaggaa agagatgagc tcagccttcc tctttccctg ctgaagtctg
46140

atgggagaat ccagggaagc tttgacaaag tgacataacg cttggccttc aaggatgggg
46200

acgtaaatct gggagaaaga catggaaggg gattcttgat ctgagcaaga agggcaagcc
46260

gggggcccaa cgggcataca gaaggttcaa gttgattcaa tggaaccccc tggtgggaag
46320

cagacactct gagtgatggt cagtttccct gaagctaggt tcatgggctt gttctatttt
46380

atactccttc tgcacacatg ctccttatag cttctcctct ctgcctctct ctccaggtac
46440

cccagttggc tgagctgatc cttaggagac gaggaggcac aatcacagct cacgtcctca
46500

gcttctctct gcctcctgcc cgcccattct ctccctcttc acctttatcc agttgtcctt
46560

cttcaaaccc actttggggg ttttcttttc catcctctgg tggccctgat gtttgcttgc
46620

tagttaagtg atttatacat tccaggctgg atccaagaca gtgtggaatg aaaaccaaat
46680

gcaaattggc tagaaggaag ggccagaata aacacaaact gaagacccca aaccaacggt
46740

gtccaagctt gtgcatgtat ctgcgctgtc cccatggggc cttccaactc tgctcaaagc
46800

aatctccagg gctgttctcc aaggacaatg ggtgagttag agagaagatc tctttgtgct
46860

tccttctttg ctttttaaat ttaaggtgga catgatctca gggctttgta cagaaaacct
46920

ctgagggtca gttgagtgac ccacttaccc tgccattccc caccctgctg gggtcctcct
46980

gctacttcaa ctccatcacc acagagactc aattcctgcg ctctgaggtt caaatctgca
47040

tctgctgaag aatcttccag aactgcaact cccatctgca ctgggagcat gtctggccca
47100

caccagctca agcactgtgt gaagggtccc agttattcag tgtgaaattg tgtaagactt
47160

tctcctggag acaggacctt tgcaatcaca gcttggggaa ttgtctttac aataaccact
47220

tcctcttagg gagtcagcag cctgggctca aatcctgctt ggtcactaag atagttttgt
47280

gactctagac aagtcacagc acccctctta gcctacattt cttcatccat tcaacaaata
47340

ttcacatagc agctaccggc accaggaata gagaggggag catgattgat aaatcatctt
47400

agaatttatg attgcattat gggagttact cagtaaatga gtttttaaaa atcaggggga
47460

gggggaaaac ctttgaagaa caatttggca agatctagaa aagctgaaat ttgacttctc
47520

tttgacccag taagtctact cttaggtata taccctagag aaattcttaa acattcccac
47580

aggacaaaga atgaatgtaa aagaatttaa atgacaactc ataagaaaat gaaaaatgtg
47640

cgatatgttc gtacaatggt atagagcagt aaaaatgaat gacctagagc tgcacatttc
47700

aacatggact atctcataag cataactgaa gaatgttgca gaaaaatatg taaagtttaa
47760

tgccatctgt gtagagttaa aatggacaaa tattagatac tatttagtgg caagagtatt
47820

ttttacatgc atgggcatta ataagaccaa attagattag tgctttcttc tgtggaggga
47880

ggaaagagga tggaactggg gagtaataca taggcggtat taactgtatt gataatgttt
47940

tgtatcttaa cctacattgt ggttacatag ttttcattat aatattctta ggattttgtg
48000

tctatatttt aaatgttgcc ctataaaaat tttaaaattt attaaataat gataagtgct
48060

ctgaagaaaa taaacatctg aagtgattga gagtgactgg ggtttggttg gggtactgg
48120

agattgggtg gtcaaggaaa gtttctcagc agaggtactg ggttgagctg aatcatgact
48180

gacatgaagg gagcagccat aacagtatct aggggaggag cattgcaggt ggagagaaca
48240

tctaatgcca agggtcgtga attgtaaagg acctaagatt ttaccctcct cgcaagatag
48300

caaggtagcc tgacttagta gtgtggatgc tgacagaaga cacaagacta ctgggtcaga
48360

gcaagatatg ttattattca cagcagcagg cagcatgctt ttcctgttca cactgcttct
48420

ccttgttccc ctcccatggt caatattcag agataaagtc agtataactt gctcatggat
48480

ttggatgtgc agaataaaag aatcacagaa atttaagatg atgatatggt ttggctatgt
48540

cttcacccaa atctcatctt gcattgtagt tcccataatc cccacatgtc atgggagaga
48600

actgatgggg gataattgaa taatgggggc agtttccccc atgttattct catgagaatg
48660

agtgagttct catgagatct gatggcttta taaggggttt ccccattcac tcagttgtca
48720

ttcttctgct tcctgtcacc atgtgaagaa gggcatgttt gcttttcttt ctgccatgat
48780

tgtaagtttc ctgaggcctc cccagccatg ctgaactgtg aatcaattaa acctccttcc
48840

tttataaatt actcagtctc aggtattctt tattagcagt gtgagaatgg actaatacag
48900

aagatttctg gatttgaggc tccatcccaa aaagtatgaa ttgaggggcc aagtaagggg
48960

tggaaaggac tcaaaagtag tattttgagc attaaaaggt tgtggtgcct actaacatct
49020

aagtggaggg acagagaaag cagctgggag taagagtttg gcgtttcagg gcaaggtcaa
49080

ggctgaaggt ctagaccagg gagggcctgg cagatggaag gtgctaacag ccctgggact
49140

gatgagctca cttgagaata cttgccacat ttaccatact cacctggtca gcagaatcac
49200

ctggaacacg ttaaaaacac agattcccat gtccctttca gatcttgcat tctaaaattt
49260

tatgactaga ttgagatata tttctgcaca cattcaatat gtactgaata tttattgagc
49320

acctattatg ttccaagcac tgtgcttgtt gtgattggtg aagctataac agcatccggt
49380

ggttatgaag aaagaccaag agaaccactg agactttggc actaacttca ttgaagcaat
49440

agaccagcat ctaatatcta cctccagact tctcactagg agggaaaaat aatcccttat
49500

ttgtttgggt tatagtgagg tttttttgtt tgtattactt ctgtcaaaag cattctgacc
49560

tcatacacac attttattat cctatttata aaactagtaa acaaataaat aaaacaaatc
49620

tctttgggga agggggcttt taattaatgt ttgattaact caagttgaat aagccaaact
49680

cccttaaagc atcagtctta gttataggct ttgtttatta aaatctttta aacattctaa
49740

acttcttgtg taatttatca tatttaattt tctattttag cacttcaaat cattgatatg
49800

acaagattat aaccaattaa gacaaaacat tctcatatat ttaagcatct cttgtaaatc
49860

taatacatta aacttatagt aaatctagtc taatactttt catgagctaa gttaaatatt
49920

cttatacctt tcaacttcaa atcacaaacg taaaatcaat ttcacatttc aaattggaat
49980

cataagcttt ttaaagattc aaatcactta aggagcatac acaagattag tcctaaactt
50040

aacatgaaag tatcaatatt atggatttga attattgcat tatttccata atgaattcta
50100

cacattccca gagttggaaa atcatttacc caataaggga acagaggtgc tatttactat
50160

tgtcaaggac tcatgcccac aatatggtgg acaactcaga tgagcagagg ttgcttagga
50220

tctctggtca taagacagta ctccagcgct gggagacagt gccctgagtg ctgtggttac
50280

tatgggaaca ggaggctccc agttttcacc cttcagaggg ttttgatttt agaaccttta
50340

ttccagttac ctcccaagta ttatttttat ttttaattat tagagtagaa ggaagttggc
50400

gactccacct aggttagcta acaacttcct ttatgatctc attggctttt gcgtttactt
50460

ggcactctgc aggcattgct aacatatttc catcctgctc taaagacatt taaatgctgt
50520

tgaattcaat cctgaacatt tggctctgag ccatgactct caggtcatga taataacata
50580

aaccaatccc ctgcctcatg actttttagg aatttcccaa atgggattat agcctctcac
50640

atttcattag aactactgta tttagcttgg caggatctta gctttggggg ctgttctccc
50700

ttgagcctgc ccctggatgt gctggacgtc atttgctgag gccacccaga tagggagcag
50760

acctggcatc agccttagat gcgtcagccc attccctcca ctaccctgct gggagttcct
50820

taccattccc acctccagtt ctctgctatt caccctctct aaaaggtaga taaacgcaac
50880

gttttttgag tgtccagcct attttgtggg ctgagtgggg caggagaagg gaggcacagt
50940

agcagcagag ctgaagccac atttcttctg gacctctcat tacttgtata tatgtgtgaa
51000

ttgaaaacac gatgttacct ttgtttccat ttcacacaaa ttataagagg gcactctgct
51060

gatacctagc aaaataaaat tatatttctc atgcctctct ggcataatat ccactgataa
51120

ctatgtgttt ttcttccact tactgatatt cctaaaagat gcttacaaaa tgtcttttaa
51180

aatatcacat cagtttgaat actctttggg aactccaata tttagaaaaa ctagcctggg
51240

tgtggtggcg tgcacctgta tcccagctac ctgggaagct aaggtgggag gatcacttga
51300

gtccagaagt tggaggctgc agtgagctat aatcacacca ctgcattcta gcctgggtga
51360

cagagcaaga ataaagtaaa tagagaaaac tattggtgga tcatctttgt aagtttgtaa
51420

atttggctta aaatggggtg cactttgctg gggtttgagc aaatgtgcaa taactattta
51480

cccccaatat catgcagcag agaagtcact cttcctttca tagggaggag caaataagaa
51540

gaaatactga actgtcctcc agtactcctt gatggaagtt tccattttgt ggacagaggc
51600

agagatgtct aggaagaagt caatcttagt tccactttat gtatgtttat atcaattttc
51660

tttggcccaa agtgatactc tatttaaagc tgtattagac aaatatcaac cgatcttagc
51720

gtttcagaag aaaatcttta tgcctttcac tgtcctattc tgatttcacc aatttcttca
51780

actttccttc tcccctcctt ttcttttctg ttatatccag ttttgttttc caaatccaga
51840

gttatatgtt ttggggaaga gacatccagc acctacacac atctgggcct ccttccttgc
51900

aggtgcaggg ggatgataac attgacgaca ctgctccacc tcctgtgagg tcacatgact
51960

agttctagcc aaagggttgt gagactgagg aggtcactcc taggccaagg tacgatggaa
52020

ttgttggggt gacctcttcc agtgttttct ccccctgctg cctctaggaa ggaggcagcc
52080

atgccctaga agttgcatcc cacagtatta ctgggttacc ccatgcaagt aattgtctcg
52140

gagggtttcc aggcctgcag aggactttgc ctgcccaata aatgaacttt tatcacgtca
52200

agtcacaagc attgcatttg ttgattgcca atgccccatg gttctcaaaa tgtggtaccc
52260

aatcaccagc atcagcaaca cctgggaata cattagaact ggagcatttg ggcaccaccc
52320

agcaatactg aatcacacaa tcacacaccc tgagggtgcg gcccagcaat ctgtgcttta
52380

acagaccacc agatgaggga tgccagcaag tatgaggacc atggctctac cccgtgctgg
52440

ctgacacaac tttgagttaa aaattccctc ttggcaaagt agagtcttca ggtgagtcct
52500

attagtaaga cacaaaccat tttcaacttt cttaattaag tatacttcct cgcagtgatg
52560

caatcattcc attatttttt tggttttaag gcctcagaac agcaagagga tctataagcc
52620

tccccgacca ctatttctaa ctttgaacac agttgtcaca tgactgcatt taatgaacac
52680

ataaaagaag gttaataaga gcttggggct gcagtgagaa ggaagaaaat tatccttact
52740

tttctagaca ccactgatgg ctggggggct gtgctgattt gccctgatcg tgatgctgca
52800

gcagaaatta gtgtggggtg tgcttggggc agaagaagtg tctggtgttt ctggtcccta
52860

gaagaccctc cattccaagg ggcggcaggc tgaaatggat tttacgttcc ttccaagggc
52920

ttccttcctc ttcttcatta ctcagtcggt tccctgcaag caatgtgagc ctctggcctt
52980

atttctcctc cgattctcat ttccattcct caaactctcc aagtggggga cattccaaaa
53040

caacccaatg aaataagcat gatatctgca ccttcagtaa atcgcttaac ctaatgatgg
53100

acttagaggg catttttagg caaattactt atatgactga aagaaatgct tatttaaatg
53160

tatttccatt cagatttgac tgtgttcaaa tataaattta aaataagagt ataatgagaa
53220

ctgtatatgc tccacctaac attttaccat gttagttctc gctcacactc tccaaacaca
53280

cacctataca cacacctttt ttcctggagt aagttgtcaa tatgatgctt catctctcag
53340

tacttcagca ggcatcactc acatcactaa caatatcacc taccaccaga tccacattca
53400

acattcccag ttgtctttta tacctatgag atttttccta aaccaaaatc caatcaaggc
53460

ttacgctttg cctttggttg ttgggtctct ggttatctgc aacagagctc agactttaac
53520

aggcatgcag atcacctggg catcttgtta aaatgcagat tttgattctg gatgtctagg
53580

tgaggcctga gagtctttga ttctaacaag cgcccaggtg gtcccaatga tgatacaggc
53640

ccaggaccac tctttgagta gcaaggacaa gacagtcccc ctcttattct tcccacagtg
53700

acactcactc cttaaatagt ccaggcctat ttttttgtag aatgatacac attttggtta
53760

ttccaccatg ccattaactt ctttatcctc tgtatttact gtaaactgaa aattaggtgt
53820

aagactcgat tagtttccat tgacatcttt ggaaggaata cttcataatt catcccagca
53880

ggaacactta atgtcagtta tcctaccatt aatggtgtgc taggactttg tcacttggtt
53940

aaggtgatgg ccagatgttt gcattataat ttttaaaata aaaatttgaa gagtgcctgc
54000

aaagagtgag aaccatgggg caatggcaat cgataactcc aatgcttcag aagtaaataa
54060

cctggctgca gatcagattg ataacacgaa ctacacagag aaaaaaatct ttcaagttgg
54120

tctctgatgg ggtaaggctg actccctgca ggccaactcc atctggccct gcagctgccc
54180

ctgagccaca ggagggcttc tctgaagagg agggagtccc tcacccaccc acgttacatt
54240

tgttcacctg aatcccaagc agggctgcct tgcagtgaca atggtttcta ccacagtaag
54300

ggcccccacc atttatgaat gttaaccatg taccgagcag catgccaaac cctatgaagt
54360

aggtgccatg gacggaatgt ttgtgtcccc cgcaattcat atgttgaaac cctaaccccc
54420

aatgtgatgg tattaggagg gggtcctttg ggaggtgatg aggtcataag gacggggcct
54480

catgatggga ttagtaccct tataaaaggc atgccagagg ctctaaccct ctttctacca
54540

cgaggacaca ataagaagtc agcagtctgc aacccagatc agggtcctca ccagcacctg
54600

accatgaggg caccccgacc ttggacttcc agcctccaga actgtgagaa acaaatttct
54660

gttgtttatc agccacatac tctatggaaa ttttttatag agagagtttt ctcaaggaaa
54720

atgaggacaa gaagaagaaa cgcacaggga aaggggaaaa agaggtgagt agagttgctt
54780

gatgagaaat catccaagct gtaagagttt gcctctgtaa atatgacacc cctgggagga
54840

cgacagaagg tgatgggtga agactacatc tagcattccc aacctggcaa ctgtgaccct
54900

atcagtccca aacagctgtg ctgtcatctg gagaataggg gtcagtgtgt ttcaggggtt
54960

ggggtagaag agaagggaga cactgtgggt gctatgtatg gtaagcagct ctctttaaga
55020

agaagaaagc aagtgctgat ttgtagtggt tgtgatgtaa actctcccac catgggcaat
55080

ttcaagctat gaatgtttaa taactgtctt gcaagattct tgagtattga gcagcttcta
55140

tgaactgctt acaagtcaga tctaccaaac cactaggcag tgcaactgac cgtttacatg
55200

ccagccaggc ccgtgcgacc caagggtacc ctgtcctgtt ctgtgaaggt gacattttag
55260

taactttgtt tttgtttttt ttgagacagg ttctcgctct gttgcccagg ctggagtgca
55320

gtggtggcat ctcagcatct tgactcactg caatctctac ctcccggatt caagcagttc
55380

tcttgcctca gcctcctgag tagctggaat tacaagtgca tgcccagcta atttttgta
55440

tttttagtag agatggggtt tcaccatgtt ggccaggctg gtctcgaact cttgagctca
55500

ggcaatctgc ccacctcggc ctcccaaagt gctgggatta caggcatgag ccactgcgcc
55560

tggccatgtt ttagtaactt tgatggttac ttattcaaaa ctagcagagt gtccctgaag
55620

tttggtaaaa aaaaaaaagt agttctcaaa aattagtgtc taaaagccac cagaaatcag
55680

agaacaggag accatttgat taatctaaat ttaaattcga gtcatcatgt agctgaattc
55740

ttgggactat ggtaaatttc tgattgtctc ctctatgtga gttttttaaa aataaaagaa
55800

aaaagccaat agtgattgtc tagaataact tttaaaatgt aaaaacccgg gcatgttctt
55860

tgtaaagtgt tcatgcattt ccaaagtgga taagaaacag gatttatggg actggaagtg
55920

ccacagagtg tggagaggct tcttgacata gtgtggccac ctgagaagag caatgagggg
55980

acctgacacc tggagaaaga gcagccaaga gacccaagag gagcaggcga gaggctcagg
56040

gaaaagcagg ggcgccatct ccctgccact cccttggggg gccctgccta cactgtcacc
56100

atgatctggt ccagaggttt gcgcttgagg tcaggcaccg tggcctcctt gctggggtac
56160

cccacgggga gcagcatcag cagcttttca tgtgcggggc ggcccaggag caccctcagt
56220

cgagggccac agttgagagg agtggtagtg acagtcacca gacctgcatt ctaaaataag
56280

aggacaagaa agcattttaa agtcagcagg cagaatgact gctctccctc taacctgggg
56340

accttcttgt cctgattata cctgctcttc ctatcatttc cctgaagttg ggcagcaaag
56400

aggctgccag ctcaagtaat ggcatcgtga acctccctgc ccccatcaat ctccccaccc
56460

cctcccttct tttaaccaag cagtggcaag cttttggaag aagaaaaata ctattatttt
56520

tcattgatgc ttaagaaata ggaatggtta ttttacagaa aaatactgta ggaatccaac
56580

tacagtagag gaaaggcaaa aacaacaggg agttcctttt agctaagggg ttggtctgtg
56640

tcctggtaca agaaccaaat gataagagag tccaccaaag cagtgtccta gcccaaatgc
56700

gacataattt tcctactaaa tatacatatt cggttttgtt ttttgttttt tttttgagac
56760

aaagtatggc tctatcaccc aggctggagt gcagtggcat gatctcggct cactgtaacc
56820

tctgcctcct gggctcaagc catcctccca cctcagtctg ctaagtagct gagactatag
56880

gcatgcacca ccatgcctgg ctatattttg tatttattta tagagatggg gttttgccat
56940

attgcccagg tttgtctcaa actcctgagc ttaagtgatc tgcccgtctc agcctcccaa
57000

agtgctggga ttacaagtgt gagccaccat acctggccaa ttttcctgct aaatattctt
57060

tcctgaactc cagtgatctg cctggaatct atgatttgtt atcaataact ctaatattgg
57120

catttgaacc catatatatt tatgaaccca tatatatatg aacccatata tagatacatg
57180

cacacatgca catatatgaa tctagagtga ctcaatatag tttcttgctg aaggtctctt
57240

atacaacatc atcatatact ataccttgat taacgaagat ttcacaatac ctgagagact
57300

gttctcaaga atacatgcat caaaaaacct aactgatttc ttctttctgt cattagctct
57360

actttatcat ggcgtttctt ctccttgatc atatcttcac atagtatttg aacttgcaat
57420

ggataaatct taattttttt tttttttttt tgagacggag tctcgctctg tcgcccaggc
57480

tggagtgcag tggcgggatc tccgctcact gcaagctccg cctcccaggt tcacgccatt
57540

ctcctgcctc agcctcccga gtagctggga ctacaggcgc ccgccaccac gcctggctca
57600

ttttttgtat ttttagtaaa ggccgggttt caccatgtta gccaggatgg tctcgatctc
57660

ctgacctcgt gatccgcccg ccttggcctc ccaaagtgct gggattagag gcgtgagcca
57720

ccgcacctgg ccaagtctta aacttttttg agaaaatatt tacaccttaa tttagtgtca
57780

ttttatatct tggtcctttt caagacatag cttttactcc atactgaatt ttgtatgtct
57840

ctagcagtag aaacctgcaa atacacagac catattaaac aattcctctt attaatatta
57900

ttaatgttaa gttgacttct attattattt tatctttcac tctctcgatg tgagtacttt
57960

ggtcttatta taggtcagga tttttttaac tggaatcctc agacccccaa agggccaagg
58020

attgaattta gcaggtccat gaacttgaat ttgaaaaaaa aagttttcat attcactaac
58080

atctaactga aatttagcag tttcttcctt tataaatgta ggcagcaaac cacagtagtg
58140

ttagcaatat ctatgacttt atcaccaatg aaatccacag ttattttcat atcattttac
58200

agttgttata gatattttaa aatatcatta cattcatcac tactttaaaa ttatagagct
58260

acgactagct cttggtattt aatatattaa taaaggcttg tatattatat tctaaacaac
58320

aaccaccacg gcaagttcct atgactacag agccaaccat ggaagcagag catccccacc
58380

cagtgagatg ctgatgcccg ttgatagggg actcagtggc caaggggtac cccatcgacc
58440

tctttccaag cctccaaata acaaacatct aagtaaaagc aatgagtagc ttccagacac
58500

ttccccgcct gcccctctcc agcttttcca atcagatcac cacctctaaa cctgaccagg
58560

aagttctgga agctgcctct acaagacatg catggcgcgc ttatcagacc cagtcaccat
58620

actggagatg cgggcttcat ggagattttt ctttcctcca tcactaatca taacctactc
58680

tcgggattat gccttatgaa agctaggggg cagcaaactc aatggcaaag tgaaaaagac
58740

tgcctgctgc agaggcacct gggcagcctt gtgagctgag cttgctgcag gcttcgctgc
58800

ttcttccagg accctcaatc aggtggcggt gcctcgcagt ctgatgccgc atggtgattc
58860

cattatttac ctgcttacag attcaggaag gaaatcaatg ccagctcctt caggataatt
58920

tttccaaaaa cctaaaacac atttcaatga ctacattttg ggaggtgtta ttcaacatac
58980

atgtttttct ttgagatagt gaagaagctc tctttttttt ttaatccatt gtctctcaaa
59040

agtatggtta cttgctctta taatcttgtt ttctacattt attttatggg tttaccatct
59100

ctctctatcc actacccagc ccccagtctc tgctagaata tacagttcat cagacaacta
59160

tccccagtgg ctcccaggaa ctgccacgta gaaggagctc agaggccagg catggtggct
59220

tacgcctgta atcccagtac tttgggaagc tgagaagagt gaatcacttg agctcaggag
59280

ttgagactag cctaggcaat atggcaaaac tccatctata caaaaaatgc aaaaattagc
59340

atggtggtgc ctgcctatag tcccagctat tcaggaggct gaggtgggag gatcacttaa
59400

gcccgggggt caaggctgca gtgagctgtg atggtgccac tgcactccag cctgagtgac
59460

acaatgagac cctgtctcaa aacaaaacaa aaaggagctc agaaaatatt tgtggagtga
59520

atgcactgtt gagtgaatgc atgaatagtt tgaaaactat acagcaatcc tgctccattt
59580

catgttagca ttgctctcgt tcctatgcag atggaatttt tgaaaagcac atgaccttct
59640

tcctaatgcc atgcaacgca aacttctcag aaaagggcct ctatgaagag cccttctcat
59700

ggctttttag tcattccatc atctctgaga gcaacagtaa acgtatccag ggagttagcc
59760

tctgtgactt ttaggctcca aagtacttca tgtgggtctg ccaaaaccct tgaaacttca
59820

tgttgttaca gatgactgca aacacaacac caatttttct aacttgaaat gatgttataa
59880

gtgaattccc agcttgctga accatcatag tcaccaatcc aggtttccag agtcagtttt
59940

cacatcagag ttacctcact taggaattct actttagatt aaaaaaaaat ttccaaatgt
60000

ccctgaattg aaaactcagc tgaaatgttc taataaggcc accctgtaac ccttgatgtc
60060

tcaacatacc tgcagggcag ctagcaggat gccacaagcg atggaaacac tgatctcatt
60120

gtagtagtgg acttttttct tgccatttgc ggcgaaacca tgtacttgtt tgaaaatgag
60180

aatcaaaata ggggcagtat ccaagtactc tttaatccag ttggttctgt gaagagaaaa
60240

gagattcagg ataatatttg cccatatttt ttgttctctt ttactacctt agcttcttta
60300

tgataaaaat aaaaacatat ttactcatct tttatattta aattgaaaag ctgcagaatt
60360

tttgtttttg tctcattcca ctttttctga ccaggatcat tcaatttcat aaacatgtct
60420

taataagtgc ctagtatggc aagacatatg aaatgctgaa agatacaagg gcaggtccta
60480

ttacccagca tgcactgggt caaaattcag caagggagaa ggcaaggatg gcagtgaggt
60540

ttagccaaaa tcacaggtgg gcagatggca atgctgttca cccagttata gaaaaaagga
60600

agggaacttc tgtgggagag ggagatcatt catgtaaggt gtaaatgcag aactgaattt
60660

ttgttttcca gctcagaata tctttcttga tttcattacc ctggcccaca cattttggca
60720

gaagttttat tctagcacag atagtcaaag gacagagagt catataatcc catatattgg
60780

gattttacct taggatatct tatatcattc attagcaaga gacaatcacc attcatttca
60840

tttctagctt ggttatgctt cctcattcca aagcacacga aactcacttg tgtatttctc
60900

catggctcct cttacgctat gaatatctgt atgttacact ttcaaagcta cttaaccata
60960

cttcaaccag cttagtcaca tttgaccatt atcatttctt ttctccctat aaagagggaa
61020

aatggtaacg ctttagaaga tctgcactgg agctgggctg tcttggccac tgggcaagct
61080

acttggcctc ccctggagct gttacctcct ctataaaatg tactcttgtg tacccacccc
61140

ctgtggttat tgcaagggtg aaaatagtta aaatacataa agtggcttaa agccaagcat
61200

cagaatttca aatcatggaa atatcaacac agaggagcca cttgtaatcc actttcctga
61260

ctcactggat gaaccaacac tctccattcc ctccacagag cacgcggact gtgcttcggc
61320

gcccggagca gaccccgagg gctgcatttg ttgagggacc acagagaaac tacttacact
61380

gaattcccct ccacttcttt cctggagttg aggaagccaa aaagttacta aggcctcaca
61440

gataactaca cttgtgttaa acttgtccta aaactaaaac ctgcttagta aaatcatttg
61500

gaagacctga tcactttagc aaaatgtaat ttgcttcatg caagatcttg agaagaattt
61560

taccatttta tgttccagac gagtgaaaag tgaagtaatt cctcaattat ttactaagca
61620

aataattgag tcagagccta tttaccaaac gctcacatgg caatacagga gtgagcccgg
61680

acttaacttc cgtaattcct ccaagaggca gaatagaatc aagctttttc acgcttacaa
61740

caaagccagg acatggtggt ggtggtgatt ttattttaag aggccaaggc aaacatcccc
61800

agttccacca ctgtttgtac ctcagtttct tgaggtctgt gacccagcga tgtcccatcc
61860

ttttcatgta gttgatctcc tcttcctcct caatgatctt tcgaatcttg tgcttcacgt
61920

ctgggtcctt cacaaccacg aaggtccagg gctctgtgtg agccccactc ggggctgttc
61980

ctgtcaccca ttccattaaa atcagaaatt aaaactgcag gaaagtgtga gactgctaat
62040

taaggagagg ctcatccctg tagtccaagc actttgggag gctggagctg gaggatcaaa
62100

caaaattttg cagatttttt tttttttaac tagcagggtg tgctggtgtg tgcctatagt
62160

cccagctgct tgggaggtag aggtgggagg attgcttgag ctcgggaggc agtgagccat
62220

gattgcacca ctgcattcta gcctggcaac agagtgacat cctgtctctt aaaaaaactt
62280

tttttttttt aaggataaac gtagtaattc atacttggcc aggggcagtg gcctatgcct
62340

gtaatcccag cactttggga ggccgaggtg ggtggatcac ctgaggtcag gagttcaaaa
62400

ccagcctggc caacatggtg aaaccctgtc tctactaaaa atagaaaaat tacccgggtg
62460

tggtggtggg cgcctgtaat cccagctact tgggaggctg agacaggaga atcgtttgaa
62520

ccctggaggc agagattgca gtgagctaag atggtgccac tgcactccag cccgggtgac
62580

gagaatgaaa ctctgtctca aaaaaataaa aacaaaaata attcatactt gctcaaaatg
62640

aagttagata atctggaaaa caaatgcctg gcagtgaacc ctgtcatgct gctgccaagt
62700

ttgttttgtt cagcgtcagg ccaactgatc aatgatcaag ctctgtagtt ccagggttgt
62760

gggaactcac ctctgcttct ctaattacat ggaagaaaat gtccccaagt aactgcacca
62820

tgcctactaa ggaaagaaac tcaggggaaa ttttgacagc cagaattctc cagactgtca
62880

tttgttttag taatcttgaa aaagaataaa gttgtgaagg ttattgaagg aggcaaaatg
62940

tacatggtaa gatggtttta aaaacactct tcaaaaggca acttagctat acagtgtttg
63000

gtccctgtat caaagctatg agtgtggcac tgtgaggttt gtccttgggg acacacaaca
63060

gttggggaat tctttttttt tttttttttt tgatggcgtc tcactctgtc accaggctgg
63120

agtgcagtgt cacaatctca gctcagtgca acttccacct cccgggttca agcgattctc
63180

ctgcctcagc ctcccgagta gctgggacta caggcacctg ccaccacatc cagctaattt
63240

ttctacactt agtagagacg gggtttcacc attttagcca ggatggtttc aatctcttga
63300

cctcgtgatc tgctcgcctc ggcctcccaa agtgctggga ttacaggtgt gaaccaccac
63360

gctgggccca gttggagaac tcttagacat gggtactcaa tacgcacggc catgtgtcat
63420

tctactcccg gggggacttc gggaactctc agtccacacc ttgatcagtt ccagtgtccc
63480

agggcaaagc ttcgcagtgc atcatctttg tccaaggagc ctcaattcct gtgggattct
63540

ggtgagagta ggggccagct tctgatctgg gtgtcgtccc tcaaccctag agatggccca
63600

ctgttcccaa ggggagacca cagtgactca tgggtgggat atttcagaag aaactgttcc
63660

ttccttccca ttggatagtt taatggaata agcagcacct atggagggag gaagggctgc
63720

agactcttgg tggggttctt gcttgccaca caccactccc aacactagaa gtcacagctg
63780

gcttgggaaa tgcaagtcaa aggctaaaca tgaggcccaa atttctgcac agagtgattc
63840

cagttaactt tgtacccatt ttatttgttt actaaatatt tgtatctcta aacataagaa
63900

ataaattttt ccctaatgtt tttagttgtg tcaaatttac cttaacagca tatgtgtacg
63960

tatacatata caaatacaga tacagataaa ggctaaattt agtaactgct gttagttagg
64020

ctaaggactc aacaatctaa tgtatttgta tttctaactg aagtaaagta ttttattctt
64080

gcttcctttc acattggaca atagattaat aacgggcatg tgataatttt tgcaaataaa
64140

cttaaatatg gtttatgtaa gaataagact ccaatcacag gagattctca agtagggagc
64200

tttctcttat ttctcctccc cattttctgt agctctgatt ttttttcctc agtggaaata
64260

ttaatttaca ttaaaaatgg gttttttctt gctttgtttt actgtgcaaa tttacataag
64320

taaactttgc tagttaatga gaatactgta ctacagcatc atggtgtgta atataagaaa
64380

tttgcttcca aaaaaaacaa atataactgt gagtatgcga gtgtgtgtat gtctggtgtg
64440

ttttaaggag agggaaagaa agaaggtgta tctcctccaa tatattaata taatgatgtg
64500

atcagaaatc ttggttttta attaaaagat catgaactga ataatttcag aagttttgaa
64560

ctattgtcac atcttcttcc aagaagcttg taatagtcaa aatgtggaaa gtagaagtca
64620

ttgtgtttct ggcactcact cagcatttgg gtaagacttt atagataaac atatcactct
64680

aggctttatt cgctatgttt aaactttgac atttttccat tgttggagtc acaccagtaa
64740

ggtgactaac atttccaaag accccatctg caattacaaa cctgccgttc tgatgacatt
64800

atcaatgact tccattggga cttgctcatt acttatgaac ctgactgacc gtctcttatt
64860

gagaagttca taaaattcct gagacctctt aaccatttcc ttctcaggat agtggttatg
64920

agagaagggg atgtgttcaa cattttcttc tgattcttgc cattcatcag catctgcaaa
64980

taagaataag aaaggtaaca aactaaatga tcccttggtc ataaggtgac cgctggggag
65040

aagaagataa agtgcttaaa tgtgtaaggt tatgaactca aaaaaatact ataaaatgaa
65100

aaaagaatag atttacagaa tccttaccac tgatcttgac tatccttaaa actccagctg
65160

ggtgcaatgg ctcacatgtg tattcccagc tgctctggag acagaggtga gaggatcgct
65220

tgagcccagg agttcaaggc tgcagtgagc tatcatcaca ccactgcact ctagcctggg
65280

caatggagac agacccagtc tctttttaaa aaaaggaaga aaagtccggg cgtggtggct
65340

tacgcctata atcccagcat tttgggaggc caaggcaggc ggatcacttg aggttgggag
65400

ttcgagacca gcctggtcaa catggtgaaa ccccgtcttt actaaaaata caaaaattag
65460

ccaggtgttg tggtgcacac ctgtaatccc aactacttgg gaggctgagg catgagaatc
65520

gcttgaacct gggaggcaga agttgcagtg agccgagatt gcaccactgc actccagcct
65580

gggtgacaga gtgagactcc atcaaaaaaa aaaaaaaaag agaaaggaag gaaggaaaga
65640

agaaagaaag aaagaaagaa agaaagaaag aaagaaagaa agaaagaaag aaagcaaaaa
65700

ctccagacta taaatctaga aaaagaaaaa ttttaccaaa aaaagatgag gaattcacat
65760

ttctgttaat gatggactag gttatttgaa acaatctcct gtcagttgaa aaccactcag
65820

atctctaaat aaagtatttt tcaaagtctt ttttaaaatc gttgagccta caacatagca
65880

agaaatttgc aggccaaagt gaagagaaaa tatgaactca cacaggtaga taagtgcaga
65940

aggtacattt ttttctctac tgtatttgcc aatccctaca catttgaatt ccatttttat
66000

atcttcaggg attaagggtg acagatatca aagcccaggc ccacacagaa tgtgaagtct
66060

aataagctag gaccccaaaa ctacatcctt aggggaagtg tcaatcataa gtaaattatt
66120

cctcaacggt ttatatcagt ttgatgagtc ttgtctcaaa cttgaacttg gaataaactt
66180

gtcctgcctg aaagtacccc agttgtctag cagaagaaaa tgccaatata caaataattt
66240

taagtacaat catcagcaac ccatcaaatg caatcaggca cataaagaag aaaacatatt
66300

ggacaagaaa cagcagaatc aattttaaaa aaataaagtc agatgcccaa agactccaaa
66360

tactggaatt atcagaccca gaagataaaa caactatgat tactgtattt agagaaaaaa
66420

gacaagctta tataaatctg tagggtacag gaaagaataa aaggaaaaaa attaaaaaga
66480

agaaaaaata aaaacaacag caaaaataaa gaataaaagc taatataaac aatctgaaaa
66540

aatagagaac ttctggaatt gaaaaataat agaattaaga acttaaaagg atggttgact
66600

agcaggttac actagctaca gaaaaattaa tgaactggga ggtaagtcag aagaaaacac
66660

ttagaattgg gcaaggagag acaaaaagat agaaaatata gaggaaaggg caagaaaaaa
66720

tagagcagag tgaagagtga gaatgtctaa tatacatttg atcatttgat tcaaagagta
66780

tatggtggag gcaatatttg aaaagatagc atctgagatt tttgcagaac tcatgaaaaa
66840

taccagatct acagattcaa gagggctggt aaattctttt tttttttttt tttttacaag
66900

aacttttttc tttctcttct tttaaaatta gaaatggggt cttgctatgt tgcccaggct
66960

ggactcaaac tcctggcctc aagggatcct ccagcttcag cctcctgagt agctgggagt
67020

acaggtgtgc accattgcac ctgactattt ttaatgacat taaacacagc tgctacaagg
67080

ggaaaaatgt gattataaag aaggtgccaa acttcaggtt cataattgat cctgacaaca
67140

ataaaagtaa atcaggggct gcgttagttg caaaactaca agttatttca ggcttcttgc
67200

ctacaggtaa gctacgtaaa tggcactttt ttactctgag gcctagtaaa ctcaaagagt
67260

tatttttaaa aactatagtt tttacaataa aatataaaaa tattgaaagc aactagagaa
67320

aaagtttacc tcgggaagaa cacagaaata actgaatttt taaccatggc aagaatagtt
67380

gcattgaata gaaatgaact aaatgttccc gataaaggcc aatgattatt ttaagtccaa
67440

gtatgttgtt tataagggat acacctaaaa cataagcata tagaaaggtt gaaagtcata
67500

ccatgcatca cataactata ctagttgaaa aaaaattaag gccaaaagca ttattgataa
67560

taaaaagggt cagtttataa taatacaatt taatttactg ggaaaacata attttaaata
67620

tgtatgcaat tacattacat gacctcaaga cagactttta agaagaaatg taaaaatcca
67680

caacacagtg aaagatctta atgcatctct ttcggtaatg atagaataag cagaagaaaa
67740

aaatcagtaa ggatacagaa gattttaaca aggtaattga caaagataac caaataggct
67800

tacataggac actgcaccaa acaactacag aacacatttt tttttaagca gacttattgg
67860

accagaaaat aactctcaac acattttaat ggcccaatag ataatgatct ctgactgtaa
67920

tgcaattaaa ctagtaacaa aaagataact gaaaaaaaca aacaaaatac cttaaagaaa
67980

attagaaaat atttcaaaat taaaatatga aattattaca tattgaaaaa tctatgcaat
68040

aacattaaca tattatattt agagggagat tccaagcctt acacatacat acattagata
68100

ggaacaaact ctaaaaatta atgaattaaa aatcaatttc aagaagttag aaaaaatacc
68160

caaataaaac caaagatagt agaaaaagta aatattaaat atgacatatt aaggaaaaca
68220

acaacaaaca tgcaataggg agaaacatca aaaccaaagc tagttctctg tagaaactga
68280

ttaaattggc aatcgtctgt caagattgat caaggaaaag agaaagcaca agtaacaaat
68340

atcaggaatg aaaaaggagg catcagtaca gatgttactg tcattcaaca aatagtatga
68400

gaatatttat tataccaata tattagaaaa tttatataaa atagacaaat ttcttaaaaa
68460

tataacctgc taaaactaac ttcaaaatag aaaaactgaa aagtctgctg actattaaat
68520

aagctgaatt agtaagcaaa gattttccta aaagagaaag caactctagg tccagataca
68580

atagaaaaag agtaaacacc ctccaactca ttttatggaa tgaccacaac cttgatacca
68640

aacataagta gggcattaag agaacaaaaa actacaggct caacttcctc acaaacatgg
68700

tacaaaaatc ctaaataaaa tattaataac agaattcagc aatttataaa aaaaaataca
68760

tcttggccaa gttagaatta tcccaggaat atgaggttaa tttaacatta gaaaaacaat
68820

cttttttttt ttatcacatt aaaaagctga agaacgtaaa ccacaaaaat cattacaatg
68880

cagaaaaggt gtttagtaaa acttgacatt tatctcttag taaatttgga gaaaggaact
68940

ttcttaatct gatagtctaa ctatgggggg aaaaacccca ccacatccag ctatggtaat
69000

gatgtatctt tgaaaccctt ccatcagtga ttgagaacaa ataggatgtt ctcaccacaa
69060

ttctaaatat gaaagattaa tttttaaagc ttttagaaga gaatataaga gaatgtcttc
69120

acgaccttgg agtggagaag gatttcttaa ataagacaca aaaagattaa acatgagaaa
69180

agaactgtta catttaatta cataaaattt ctcattatgt ctgcctgctt ctgccacttc
69240

ttctgttttc cccattaccc tcctgttcag aaagttctgc aagctcatta tcagtagttg
69300

aagatcttag agaagatgaa gatgcaacag aagggtcaat cactggagct aaaaatgcca
69360

aaactgagga gaaagaaaat gagcccacag atatggcaga agataagaag gatgatgtgt
69420

ctagtgcact aaaagcttca ccaagggcac acccagttat gttattttag aagaagattt
69480

tccaataaat aactttgtga agttcaagat agtctctaca aacaagagtt ctgaagactg
69540

tattgtcaaa ttcccagata ctacattcca atattctcaa aattgttcgt tatatatcca
69600

aaatttcaca gcttttctga atactgtttc ttcatttcac ttaagctcat gaaaagacat
69660

acttctgaac tacaaagatt tcaacagaaa aaaattctaa gatgctgtct tcaaacagtt
69720

aaaattattg aggggaaaat gtttggatgg caaaaatata ataataataa tagatattcc
69780

ttgcttgtct taggctggtc ttgggcttcc agttgttgct gagttttatc aattcctaga
69840

acttaggagg caaaatatac caaaatgagg ccagtacaga aagtaaacct cagtatatca
69900

aatcagaatg aattgttatg tgttggaatt ttttcagtga aatttgaatc gcataaagaa
69960

agcttcactg agaaggctgc ccatggagta aacactgaaa atattgttgg gatctaatga
70020

gtagatggtt cttttacaca ataattcagt ctttaacaaa cctgccctaa tattttgcag
70080

cctgatggga atgggtgtag agaagccata tcacacagaa agcaactcct acttagataa
70140

gaacagatca tcagtctgct gtgatattgc tgaaaatgct cagcactcac tttcctaaaa
70200

taacaaattt aagatgtttt aaaccattaa aatgtatgtg tgtgcatgtg tttgtgtcct
70260

acctgaatca ttattgtact acctaaattg tgtctttagg aaactgaata taatgttgtt
70320

atatatcttc ctcattgaca acctccccaa aacccaatta tatgtgcttg atatgatgtg
70380

tatcccatgc cttgagctgt gtgatggaag aaaataactt gttacttttt tcttttagta
70440

gatacaaatc cacttcaagg gttttttttg ggaggaggtt ttttttaaac attaattttt
70500

tttgtttttg ttttgttttt tttttttttt tagagacttg gtctcattat gttgcatggg
70560

cctcaaactc ctgggctcaa gcaatcctcc cacctcagcc tcccaaagtg ctgggattac
70620

aggcatgagc cactgcaccc gaccataaat ccattttgat ctcattgatt agctgatgca
70680

gctgtggtta tagttgctgc aatttgtggt tgtggcatct gttaaaggat tttcactttt
70740

agtagtggtt aattagagaa tttttatgaa cgccgcaagc tagaaaacca aaacaactga
70800

aatcctttgt aagatatatt ttaaaacatt ttcttgtatg cattggaatg ctcatctgtt
70860

attgtaggaa atgacggtca aaatctagct gaaggttagt accacagagg tagaccagca
70920

ttcaaatgcc cagagtatta tgtattttcc aattaccaag aaacagctgt gaggttgatc
70980

tgagataaag gatgatgaca aaagccaaaa tcccaggtct acccatggag acttgacagc
71040

cccctacact ttaagctggg accctgaagg tctacagtct catagtaagg ggggatttta
71100

aataaaccag ccctgatatg tgcttgcagc ctaagataat atcatctgag tgatccaggg
71160

aaccacaggg tttgacctta ggttaaagtc atcccagagt attagtgtca caccatgtaa
71220

gcacaaaagt gcattttccc tggaaataag taccaatatc ctagacctca caaagttttt
71280

ataagtactt ttccaaaaga atgactagca aacaatgaaa aataacaatg tatttaggaa
71340

aacacaaagt tatgaataag aaccattaaa aaactatggg catcagaaac agaccaacaa
71400

tgacttatga tactggagtt atcagccaca gatcatgaaa caactatgct tactatgtcc
71460

aaagaaatac aagacacacg actttggcag aaaaaagtga cagcaaattt ggaaaaaaga
71520

actaaaaaga tcctctagaa acaaaaatga aaacaaacta aattaaacat tcaattaata
71580

ggtttaacat cagtgtaggc acagctaaag aggaaatgaa tcaataggaa gatagtttct
71640

tctaacctat ccaaaatgta gcatggaaag agaaagacag aaaacatagg tgagaatgta
71700

aaagacatca gaggatacag tgagaaggtc taaccattta attggagtcc tgaaaggaga
71760

agagagagag gaaatgggcc agaggcaata tctgaaaagg caatggctga aatttttcca
71820

aaattgttga aaaataccaa tccgagattt taaaactcag tattgtctag taaaattgga
71880

tagaggtata ccctacatgt ctctacttag gttctccaag aagctgactc agagactgag
71940

tcgtgtgtgg aatgtttata ggaagtagct ttgggatcaa aacctatgga agggagagga
72000

aagaaacaag attgggcaga agaagccaag ctgcaatgca agctgagaga cggcctcaac
72060

cacagcaagg gctattttga aactaaaatt gtccatcaga gttgtctcag attaggccat
72120

aatggctgga cctttatacg accactgcaa tcagtcatta gatgtgggcc atcctgggag
72180

gggtgggatc tcgggagaga tagctatcta cagcagaggc cattcctgca agaggccgac
72240

agtactcgca gcaatagaga cagcaagccc ttcctcaagg ggggatctgc gtggtacatc
72300

atcaggccct tcacaccatg actctgcaat tgcttgcaca cgccagaaaa atccatgcac
72360

atatgtacaa gactagtatt aaaatgttta cagaaatagt tgataatagt cctaaactag
72420

aaatagacca aatgttcatc aacagtagaa taaattgtga tatagtcata taacagaata
72480

ctatacaata gtgaaaatga ataagcctca gttacatgcc taacctgctt tgcattatat
72540

ttgaccctta caaatataaa acagcaagtc aaaagaatat aaacagtata attttaatgt
72600

ttaggaatgt ttagttgtgt aagagtaatg caactaagaa gaaaagcaaa gaaataattt
72660

tcataaaatt caaaatagaa gttacttctg ttggaaatag aagaggttgt gactggggaa
72720

ggacatatgg gtgtttatga aatgttggat gtgtatattt cttaacctaa gtgataattg
72780

tataaggttt tgcttaatac ttatttgtta agctgttcat ctgtgtttta tacacttctt
72840

cagtgagtgc atttcacaat taagagtttt ttcaatgaga agaaggccat agagattaat
72900

taccagagga atcaggaata agtcaactgt tcagaacttt taaacaaaac ttctaaaaaa
72960

ggagaaaaga cacttgacat tttctcaatg cagtgaaagt tcaggttcag caaaagtagg
73020

taaagtctag atacagtgga aacttagatc cttagagatg aatttcttgc catgttacca
73080

tttctgtaag tacaatagca aaaccaatat ttttaaattt ctgtttcgtg tttaaaatat
73140

caatactttc aagaaagatt tggagtttaa tacaatgagt aagaacttaa aagtgtaaac
73200

aaaactggcc ataggtactt ttgccttttg agcaaagtag catcatttgt ggaaaataaa
73260

cttcctgttg ctcacatccc ctcaccaggc tgatgaggga agaagacagt gtaaaattgt
73320

aaacctctga cgattttcct gtgctgccat gtccctggat tccatggacc actctttttc
73380

agagcatagg catgagtctt tgggggagag gagctgagca tgatcaagtg aggggaacag
73440

agtttgggga ggggagccca aagggaggcc tatgacagtg gctcaaaaga cctttagaag
73500

gaagaagggc tgccccaaat gctaacatgc tgtttgagga gttgggaagt gagagaccaa
73560

agaaacaaac agaaacaaag gttgatttcc acagataaat tggtgtgagg tggaagaaga
73620

aaacacatag aggaaatcgg gggtcccttg ggcatgaacc tcagagccag aggtggctcc
73680

atagacacag gttctgtggg gattgtcaaa tctttgagag ggaattggcc tcctcaaagc
73740

atgtaggatt gaggattcag ccttttttaa atcttatttt tagtttattt atttttttct
73800

aaataggatg agctcagcta acccttgggt tatgtaagta ttacacatta gctaaaagaa
73860

caaagcatga agaagcagcg caagtcctaa agttgatctg ttttatatta gtggaactaa
73920

acaactttct ttttttagac atctgctcag gaaatgccaa tagcaatgat tttgcagtgg
73980

ttgttgttgt ggtagtggta gtggtagttg tggtgtttta gggagagaat ggtggtacag
74040

tggaataaaa atctctggtg ggtatgatta gttgggaaaa aacaagtttt caggttacaa
74100

ttgtaatttg caaaacacca aatactatgt ataatgcaaa ttacatttgg taaagttctc
74160

aaaatcttcc tggaagatgg aaatatacaa gtcattcaac aaggctttta ttgagccttt
74220

gatatatata ttagtgaaca agacagatgt gacttttata ctataggggt gaagggggaa
74280

agcttcctcc ctcactctct gaagattcac tgaaaatgaa ttcacaaaag acagattaat
74340

aggagaaaaa gccatacaaa attattctaa catgcatagc atggggcaat agcaagagaa
74400

tgatcactca ataacccaac ggggtccaaa tgcttataca cccttcttta tagaggaaga
74460

ggagatggga ggtgtaggag taatgaattt cagggggaat gaatggaccc aatgatcaga
74520

taatggatag tcaataattc cctttgggaa ttgaatggga cctgagaaca aacaatggtt
74580

tatgacaaag tttgcctggg ctctaggtgt ggtgtttaat tttcactctc ttcctctatg
74640

atatgaattt taatcttctc cgattagtaa cattttaggg aaaggattga aggtaacaat
74700

tttcctcttt ggaacaataa ccttattcct tatatattta aacaaagcag caaatgtatg
74760

tatgcataga attacaaact gtaatcactc ctatgaggac aattaattct ctatgaagtg
74820

acagagaata agaagggatg gtacaaagag ggaggacaca aagtgattcc caaggggatg
74880

gcatgtaatg tgaaacttga ggaataagaa agaggctggg gattcaagta tatttagcag
74940

acaatgtgaa aaacgcagat ccatacagac gctgtagaca tagcttgact gggggacgat
75000

gctggcttgg acccttggag aagcagaaag aatgacaggg gtgtgcagat tctcgaaaca
75060

ccttagaaat agaaatcgaa agactggcta acatgggtgg ttgagagtgg ctgagagaga
75120

gagggaggaa ccaggaatca atgccaggtt tctggcttta gtgcctggtt agatgatgga
75180

accatccact gagccaggag gactaagaga tgaactattg ggggtgggca ggaaggagga
75240

acaggagtca acagtctgcc tgattccaga tgctgcaagc cattcaagtg caggtgtcaa
75300

gtacaaaagg aaatgtgtga gctttgaagg tgagatccca tttggagccc tctggaagcg
75360

atgagcatga ccaggaggaa tgaaaggaag agtggcacat tttcttaatc cagtctatca
75420

ttgttggaca tttgggttgg ttccaagtct ttgctattgt gaatagtgcc acaataaaca
75480

tacgtgtgca tgtgtcttta tagcagcatg atttatagtc ctttgggtat atacccagta
75540

atgggatggc tgggtcaaat ggtatttcta gtcctagatc cctgaggaat cgccacactg
75600

acttccacaa tggttgaact agtttacagt cccaccagca gtgtaaaagt gttcatattt
75660

ctccacatcc tctccagcac ctgttgtttc ctgacttttt gatgattgcc atcctaactg
75720

gtgtgagatg gtatctcatt gtggttttga tttgcatttc tctgatggcc agtgatggtg
75780

agcacatata caccatgcag ccataaaaaa tgatgagttc atgtcctttg tagggacatg
75840

gatgaaattg gaaatcatta ttctcagtaa actattgcaa ggacaaaaaa ccaaacaccg
75900

catgttctca cttataggtg ggaattgaac agtgagaaca catggacaca ggaaggggaa
75960

catcacactc tggggactgt tgtggggtgg ggggagcggg gagggatcgc attaggagat
76020

atacctaatg ctaaatgacg agttaatggg tgcagcgcac cagcatggca catgtataca
76080

tatgtaacta acctgcacat tgtgcacatg taccctaaaa cttaaagtat aataataata
76140

aaataaaata aaataaaaag aaaggaagag tggaagaagg tcaaagaccc gccctgaggg
76200

acttggccat tctccaggtg gaattcaggt agagaattca ccttctgagg aacaccaata
76260

acaacgttag caggcgctac ttttggctta tagctcttag tgaggaaact tccttttata
76320

tttctctagc agggacagtt tgggcctatt tagaagaaag tatgtgtgat gggggagtgg
76380

taataaacat ctatcaactt tctaacaatc agtcctggcc cagcgaaatg ccaatgtcat
76440

gaacgtcaca gtgacaactc tgaactttat caaggtgtca ataaagactt tcaacaccaa
76500

tgatactttt gacaccattt aagatgaagt ctggcagcct ctgtagttaa taagtggtat
76560

ataagcaaat gcattaaatg cttccttaac tctttgaaag gtaagttact aagtcttaag
76620

acccattcag cacttgtaaa aactgaaagc agtctaaatg catgacttgg cagtttccac
76680

agcttctaga gtccaaggca gactctccta attagcagaa ggaagcaaac tgttacctca
76740

ttatcagtgc taaccattat ccctgactag ggaatggtca aacaaatgat cctcatcacc
76800

tcctgatggg attgtcctga gtgcactctc ggagcagtgc acagccgggc tgccattcct
76860

ggagcctttc acagggcaga aggtctatac tatggctggg gaatgagact agcagggctg
76920

ggctaggagc acagtgagct tccacttgtc cccttctctt gaactcctga ctgctcccga
76980

ggaatgtggc cctcagccag aggcaagaga gcagactgtg taaccccttt gttttcctgc
77040

cttcactcta gagcagtgga gccactaggg attattcaag ctcgaaggtc cacacagtgc
77100

aaagcaaatg tatactatta tggtctgctt tttgaatgaa atcttgtgct gccacacttg
77160

gaacttgcta ttgataccaa cagtaacttg ccttgtatct ttgcactggg ggtctgcaaa
77220

caatacccta aaatactgac acttggcact tgacctggtg aactgaagaa gcacctccag
77280

gtctccctct gacctgcctg gtctctggtc tctctgatcc tttttcctga agcactgaga
77340

gagactcttt ctggaatttc gctatctgac taggggcatt tctttccaaa agaaatgtaa
77400

ttaggatgtg cccagtggct catgcctgta atctcagcac tttgggaggc tgaggtagga
77460

ggattgcttg agcccaggag ttcgagacca gcctgggcaa catagagaga ccctgtcttt
77520

acaaaaagtc taaaaattag gcatggtggt gtgtgcctgt agtcctagct actcaagagg
77580

ctgaggtggg aggatagctt gagtccagga gtttgaggtt tcagtgagct atgatcatgc
77640

cactgcactc cagcctgggc aacaaagcaa taccccatct attaagaaaa gaaaagaaga
77700

agatgaagaa atgtaattgt cttaagatcc cttgcctggg aatccaggca gacagagaca
77760

acctggggaa ctttacctgc ataatgagaa gacttttgtt cacagtgaag ttccacccct
77820

cgccttccca ctgcctctca taagctcaag gaacttcatc ccaaggcatt gttctttgga
77880

cccattcatt ttccctgaaa gtcatttact cctacacact cccaccatcc cctcatcccc
77940

tatgaagaag gatatataag catctggacc ccactgggtt attggcgatt ccccagtgct
78000

tatacatgtt aaataaatct gtatgccttt ccttctatta atctgtctct tgtcagttca
78060

ttttcagtga accttcagag ggcagaggag aaatttcttt tcatatttcg ctagcaggag
78120

cagttttggc ccaaactgta tacttttagt tagaaggaag tagatgtgaa gggggagtgg
78180

taatacacct tatcagcttt ctagcaatca gtcccttggt ccctacaatc gctttaatgc
78240

caaggaatcc gttaattgtg ccataattag gtttagccag ccaagtaatt ctaggtcact
78300

tctatctttt cacatttagc atggaagtgt ttttctggtt acatctccaa atacattgct
78360

ttagtgtttt tacagttcta ctacacagtc tttgcaaatg gaccactatt ttctaatcaa
78420

gaaaccaagg acaatttgtg tcacatcatc cttgttccct tcttgcaatt ttggaagcct
78480

tgaggagtga tgttactaag gaggagggtg atgcagattt tgaaggtgag gatgggagcc
78540

acttcagggc tcagcatctg ccccgacaat gcctctttcc cagctgcttc accattgctg
78600

tgctccctac ctgggcctct cagtcatccc acttcctgtg aggccatcct catagggcta
78660

acaagaatta tggacagaag tagttataat caagcattca tcaggctgct ttgacccact
78720

tccttgttct gaaagtctca tagcactagt agatactgac cgtttgcatc cccgctgttc
78780

ctatgcatag aatttctgac atgagagtca caaggctttt gtttgagata gataggatct
78840

atccatcacg atcataaggc ttttatttaa aaactactta aaatgttttt cagatccgaa
78900

ttccagtgaa atagctgatg ccaaccagtt tgaagactcc cataatgcag tggaatcagc
78960

atgagaatac agtttcttca tctccctgtc ccatgacttc accctgcgct ctgaccaatc
79020

agtgacctcc acactttggc ccaccccaaa actctaaaaa atcctagctc caaactcctc
79080

agggaaatgg atttaaggtt tcctcctgtc ccttcattca gcagccttat gattaaacct
79140

ctttctctgt tgcaacctga tgtctcagtg tattgacttg ctgtgtgcat cgagcaacag
79200

aactattaag gtgacatctg gctgctgtat agatggatta tttcattagt agcactgagg
79260

tgggtaggga ttgggaaggc tgaagggtgc ccagaaagag acaccttgaa tagggaagtg
79320

ctttcctaaa gccagtgggg ttcagaacat attaccccaa agcatgccaa cttcagagca
79380

aaaagggctt tctgaccttc cactgccctt ctttcctgaa gcaggtcata aaatctagga
79440

agaatttcct gaccctcccc tgaggcaggt catatggcac ccaagtaaga ggtgcccacc
79500

cagaggaaag gagcattctc atctctgagg atgttaggga cacagagaag aatctgaaca
79560

aatacatatt gcaaagtgat atggtttggc tgtgtcgcta cccaaatctc atcttgaatt
79620

gtagctccca taattcccat gtgttgtggg agggacctag tgggaggtaa ttgaatcatg
79680

ggggcagttt cccccatact gttcttgtgg tagtgaataa gtctcaggag agctgatggt
79740

tttataaggg gaagcccctt tcgcttgatt ctcattccct ctttgcctgc caccacgtag
79800

gctatgcctt tcaccttcta ccatgattgc gaggcatctc tagccacatg gaactgtgag
79860

tccattaaat ctttttcttt ataaattact gagtctcagg tatgtcttta tcagcagtgt
79920

gaaaacagac taatacacta agtttcccca gtttattgcc attagaccac gttagaccat
79980

agcccctctg tcctcttgta tgtctccacg actgttcact ttttgccaaa cctagcatga
80040

aaacacacag gtttaactgt tcctttggat cttcatttcc ctatgaaggc tcctgtgtca
80100

catgaaactt atgctaaata aacatgtata cttttctgtt attctgtctt ttgttacagg
80160

ggcctcagcc atgaacctag attgggtaga ggaaaaggta tttttatctt cctctccaaa
80220

gcagtggagt gaagacagga tttgtcttct gagacccttt atctctttgg gtaccccagt
80280

gtcccctgct gaccctaact ttcccctagt aggaacccac ccagacttga tctcaagaac
80340

aatgtcttca ttcaagtcct tttcctttcc tactcttctc tcatctaaac ttgcctgtct
80400

cttattctaa aagcttaagg tcttacagga gtctagcaaa ccaacctctt aaattagcca
80460

tgtgaaagta ttaatgtcta ggtgtgtcta agacaaagct gattgcattg cttcccatag
80520

taaaagacaa ctatgttgat caagctcgac tctctgagca gagtagaatg ctgacttaat
80580

atagaatttt tggcagtggt gggctggtaa gccaactctt tgtggggtgg aagggagaaa
80640

gcactgattc atagcacttg ccagtatcca ctgatgtaaa taatcacacc atggcgtgac
80700

ttcaagccac taatgattta acaaaaaact cctaaatatt taacagttgg ctcctgctaa
80760

tccccagaga ctcgccctag tatgctactt ggttggggag catgaagttc aggcactggt
80820

ggactttcag agctgcaagt ggcacacatc atttgtagga tcagtgtcat ttggttgaga
80880

ctctgacact cagaggagct ggccctggag tgagtgcacc cctggttggc tggctttcag
80940

aagcatgagg ctgacactga ttggttagct tgccaagttt attctgaaat gagttgtcat
81000

tgattcactg ctcaggcttg gaaccagttt tgaaagctac ttgttattat gggcacataa
81060

caatctgttt ctcaagtttg taggaacatt tttgttctca gatgttactc acactggata
81120

aaaaatttta actcaggcct ttcttagaaa agactggaca agttgggtca cttattattc
81180

agctagtgac ttcagttaga ataatagctt ttggatggcc agtgaagcta agtgcagaag
81240

aatcccccac taacactaaa acagattcat cttgatgaga tgtgtttcct atctttttct
81300

tcttttccca tttctatgga cttctatgaa tctgagccta actagaggat ttgtgggtag
81360

gattttcagt tcactttact gggacaatct gagacttatt tctttgagat aaacttcagc
81420

aactcttctt tcttggaccc taactgtaga caaacaaggt attaacagga caccaacctt
81480

ggctggtttg caaagggaga ctgtacccta catctaataa gcatggtcac acaatgttcc
81540

ctcaccaccc ccaccccacc cacacacaca tacacatgta taacaagcat ggtcacacaa
81600

tgttccctca ccacccccaa cacaccccca cacacacatg tctaataagc atggacacac
81660

aatgttccct caccaccccc accccaccca tacacacaca tgtctaacaa gcatggacca
81720

acacaatgtt ccctcaccac cccccacacc gcccacacac acacatgtct aataagcatg
81780

gacacaacat gacccctcag cactccccac cccacccccc cacacacatg tctaataagc
81840

atggacacac aatgttccct caccaccccc caccccaccc acacacacac atgtctaata
81900

agcatggaca caacatgacc cctcagcact ccccacccca cccccacaca cacatgtcta
81960

ataagcatgg acacacaatg ttccctcacc accccccacc ccacccacac acacatgtct
82020

aataagcatg gtcacacaat gacccctcac caccccacaa cccacccaca cacacacatg
82080

ccgattctct gacctgtgaa catgcagtag gaattctttg tgaatcagag ctgcaggagg
82140

ttggatgggt acccatattt tccttaactc atgctagaat ggcctacaat acttatttta
82200

ccatttaata ccttgttaat attaaaaatg gtttcaatct ggtatctatt tgcagtcaac
82260

aacaccaact ccatgtgtct tcagtaaagg agtccgttct cttctccagt tagctcctgc
82320

tcaatccctt ctggacctag agtaatatcc ccacgtagat catgtccaac caccctgtga
82380

ctgagagcag ggacttgggg ggctgagaag cctgagttta agcattggtt ctcactggtc
82440

acctgtgtga ccttggcatg caccacttat gtaaacaaca taagagatgt ggagtaaatc
82500

atttcatctt catgacaata caatgaggta ggccctatta gaatccccat acaagagatc
82560

tgtggacacc acactcaggg cagactttgt cccagctgct aaggacgaca ctaaggctgt
82620

cctgttcatt cctctgtata aagataagac aaggatgagg atggcaggga acagagaatg
82680

aagaggcaaa gcattttgca aaagtaagtg atcaggcaga gagccagaga aaggccctcc
82740

agttctgctc tatgttccca gcctccaggc ccagccccag gacctgaccc tcaacagcct
82800

gcccgagctc tcctgtgtcc tcaaacctct tgggtcttca aatccagtgg cctttccttg
82860

atgcacactc tttctggtct ctcagagaat gttggtccct gctggctatc tcctgcctcc
82920

tccattctag gtcattctcc caggggagag gcagcccagc aaacagctgg cttgggtcct
82980

atctctgcaa tcaccttcct gagcttaccc tattggggcc tcaatttcct tatctgtaat
83040

atggggattc taatagtgcc tacctcattg tactgtcatg aagatgaaat gatttactac
83100

acataatatg tggctgtgat tgcatacatt aagagctatt aatatataag ggcttgttac
83160

tgttatgatc ccagagcccc ctttcccaca gctcctccct ttctctagta cattttctt
83220

ccacagttcc tcttaaccgc atgtgtccct caagttctat cctcagcctt ctctctagca
83280

atgcccactt tgtggattca ttatttctcc aggttccaag tatcttcccc atactggtga
83340

ctctccagtc agtatctgtg gcctgggccc ctaacataga ctgctgtctc ccttttctgc
83400

tcaggtgtgt cactgtcgtc tgaaatcaaa catgtcctcg atggaactca acatcttccc
83460

ctttaagctg gatcctcatt ccctcaccca cttatgatag cttccagtct ccaactctcc
83520

ctctctcctc cttttcccaa tatccaattt actagcaaac cctgttgctc ttccattaac
83580

tttactctga tccacgcctt ccactgcatt cccacctgcc cccaccatcc aggccatcct
83640

taccagcaca aaccattacc cagcttcaca gcagatgttc cctggattaa gggaagtgct
83700

tctccgctgg gtagaattga cgttctgagt ggaagaggtt tcaatgtagg agagcaagag
83760

gcagccctgc tgagaggcta gactcctgta cggtgcagtc agcactacag gctgttagca
83820

ctgagagcca gctggacacc ctgcgccccg gtcctgagat taggacacac actcatgcac
83880

acactctctc acaaacacac tcatgcgcac gcacacacac attcatgcat gcacacacaa
83940

actcatgcat gctcacacac actcatgcac acacgtgcac actcacacgc acactcaccc
84000

actcgtgcac acacactcat gcacacacac tcacacatgc acattaatga acactcacat
84060

gcacgcgcac acacatccac tctcatgcac acacacacac gcacgcgcac acacacatgc
84120

acattcataa acacacactc tcacacacat acactcacac acactcacat gtccaggtgg
84180

cctctgtttg gggatggggg catcattagc ccagctgaag atcacagagc ctcttccaaa
84240

tcgaacccat cccagacaaa gcttctcaaa cacagatggc tcaagcttcc tccacgctct
84300

ctcccttcct acctgcctcc ttcatcacct cttcttctca aggtggccat aaccttcccg
84360

ccctcaattc tctacttcca ttcttaccct ctccctccac cctcaattct ccacttcctc
84420

tcttacccct gccccaggcc tccaggtgct tctgctcctt tggctccttg ttgcctgaca
84480

ggcctcccag cacttgctat ttgtacccta ttcatcctcc cagcctggtt tgtcctttcc
84540

ctgactgcac cgtcaaactc ccaggcctac tctaaacacc ttagaatcag ttccaccggg
84600

tctgtctctt tgttactctc tctgattcta gtgtatcatc ttggcagctg aactggacat
84660

tcagtttgtt gaggacagag gccatcccag gctttcactt ccagggcctg gcacagggaa
84720

aggtagcagc ctcctaggtg atgccacttg gtagatttca aagttcctga tcaaaacctg
84780

gcctcttagt ccctgcccac tgccagcctg ggatgaattc ctgcagaaag attttaacaa
84840

ccagaacaga attgtagcca gggaaaactt cagcctctca acagcttttt cactgggcat
84900

caaaaataaa aaagggttgg tcccagcagg tgacaagctc atcactggag agagatgacc
84960

tgtctgtgcc aagcactgat tcattagcac aatagaggtt gatgttgaag ctggcctgtg
85020

tgtgtttctt cctctccata agccctgcca ttgactcaca ctcatcatca acacaacacg
85080

acagcgaagc taagcagcat agctggtgtc tttaccttct tctgcttggt gcaggtcact
85140

gctgtctttt aagtcttcat ccacccaggg gcgagcttcg gccctggttc taggctcccc
85200

cttctttttc tccatgcttc tgtcggcatt tttaaagatc cacacaacca aaatgcagag
85260

aatggctacc aagatgggag tcaggaaata catggtctgg agtctgacgt cacaggcgtg
85320

cacacttccc acaccggttt ttaaacacac agagacagga gtggagaatg ccgggagata
85380

gaaggaatgc agacacaggt gtgccaggga aggaacaaag tgcagaactc ttcaactctg
85440

tgaggacttt gcaaaggagc aaagatcaca gtcacatgtg tggggtgttg tggctttggt
85500

tatacaccag tccattaact ttttaaaagt atgtactctg tcgatatatt cttagacaac
85560

aattgccaga atctgagtaa gaacttgcat gtgctagatc tagctagatc acctgaagaa
85620

cttgtttttg tttttgtttt ttttaaatag atttccacac accaactcag atctgctgga
85680

agaaaatgtt caggaaagag gtttggcacc atgcatttta aatggcatat ttgtgaaaac
85740

agccctaggg ttttgatttt ttttttatga tacctgagcc aatatatgga gtaaagagaa
85800

tatcatgttt tgcatcagag aaaaactttg atccaggaaa agtgacacaa aaaattaaag
85860

tgacaacttg attatgtaat tacttcagta gggcttttta tgtgttagtt tgtctgtctg
85920

ttttagaaag tgccttagtc agttatggct gaaataacaa aaatactatg gaccgggtgg
85980

cttaaacaat ataaatttat ttctcagcca ggtatggtgg ctcatgccta taatcccggc
86040

actttgggag gctgagaggg gcagatcact tgaggtcagg agttcgagac cagcctggcc
86100

aacatgatga aaccccatct ctactaaaaa tacaaaaatt agttgggcct ggtggtacat
86160

gcctgcaaac acagctactc aggaggctga ggcaggaaaa tagctccacc caggaggtgg
86220

aggttgcagt gagccgaaat tgtgccattg cactccagcc tgggcgacaa gagtgaaagt
86280

tcctctaaaa aaaaaaaaag aaagaaagaa gaagaagaag aaaaagaaaa gaaaagaaag
86340

aaagaaagaa agaaagaaag aaagaaagaa agaaagaaag aaagaaattt atttatcatt
86400

tatttatcat agttctggag gttgagaagt ccataattaa ggtgctggca aatcagctcc
86460

tggtgagggc tcccctcctg gtttgccaac aggtgccttc ttgctgtaac ctcacacagt
86520

ggatggtgga aacagatcat ctctatcatc ttttttctta taaggccact aatctcatta
86580

atgagggctc cactctcatc acctgttcac ctcccaaagg cccaacctcc aaataccatt
86640

gtactggggc ttagggctcc aacacatgaa ttttgccaag acatattcag tctttagcat
86700

caaggtgcaa acgtatgtat aacatgatgc catttgaata aaacagagtg gaaaatatga
86760

tacatttata caaacttata tatgtatttc ctggaaaagc atactctaag gagcggttat
86820

atcaggaatt ctctttatta attttgcaat ttcttgtgag tctatattat ttcaaaataa
86880

acagtgaaaa aaacagtggt tacttgtttt ggagggagtg tggaaactgg atagatgggg
86940

gacaagggtg agagaatttc tactgaatac tattccataa tttaagtatt ttgagctgtt
87000

gtaaatatat tatctattta aaaaattgaa tatgtgttat ttatttcaat actcattttg
87060

atggttttat tgtatcatat attgtaatac tcctttttgt gatagacagt tgttttttaa
87120

gatgtatatc atattggttc tctaactcca gagaactctg tagaagataa taaacaaagt
87180

ttattatgga agcactggcc tccacagacc attgaatttg gtggtgctgc ctgccatgcc
87240

gacagctttt gtacaggaaa gtgccctccc tgtgcctgct gaaagggtgt cagaggggac
87300

atgtccagtc catgtgatcc aaccccacgc aggcagctga aaatggcaga gcctgtgttg
87360

gggcccagct tctctttaca tcccactaaa aacacagaat aaagaaacca ctccatagca
87420

agagaagagc aagacaaatg tgcagagaga agcaaggcct gcaaggcagc aatgcctcac
87480

agctcagggg aaggacaagg tgagggccat gggtggcaac tgcctctgct cctaggaggt
87540

cccaggcctg cctcggtttc agcccatgtg tctccttacc ttcgaagaat cagaaagatc
87600

ctcggtgcct gtaactacaa aagcacaatt catatcagtg tcaggaacag gattaagccc
87660

aggaaaaatg gggtggtggg aattggtttt agtgcactag tgggtgaagt ctgggaggca
87720

cccccatctc agaagggaat gctaacagct gtggcatgca gcagctggct ggctaattga
87780

gtattgcctg agtctcttgg gtttcagacc atgtgcccag cagaatgagt ctgtagggga
87840

ttctgaagcc actgagagcc ccggaggagc tgaaacataa ggacaaccat gccagaaggc
87900

agctttccat catgtttggt gacttgcaat gggaaagaaa agattcttgc tctgaattgc
87960

ttggttcaag atagagaaga aaagcaaaat gaccatgcat tccttcattc attcattcac
88020

tcattcattc aaaatatatt ttttgggatc catgcaaaag gcattgcatt tggggccaga
88080

aatagaacgg agggcagtag gggctgcaca tgctcatgga ggcaggagcc acagagagac
88140

aagcggatcg aatctcatat actaatgcca cattccaact tccctaagag ctgctaggga
88200

gctgactgga gtgtgactgg gctgagttgg tgaaatcaga ggcttcccag aggaaggaat
88260

ggctgagttg aggtcggagc tgtgagtggg aatcagtcat ggggcaggag agagccttcc
88320

aggcagaggt catggtgtgc agcaggataa ttaaggcatc agagatgggt tgaggaggaa
88380

ttatttaatt atttaggtgc actgacccag tcagattaac atccaaaaga ctgagccccc
88440

aacaaagagt caagctacct tttaagcatt ttgtggggtg gggggagatt tgtgcagggg
88500

gaagtgtatt acagaaacga gaaacaaaga cagttattca attaagacat gcattacatt
88560

atttcttact tttcaaggaa cagcatgttt tgtgacttga gattatctgt ctagtgacct
88620

tgcagctgca cagctagaga aacagggtct tcacaatgcc tgggaaaggg ggagataagg
88680

ctcactagcc tcagaaaaac aggcagtcaa tttttaaagg atttcagctc tttctcttcc
88740

tcagggggaa ttggtttttc ttacatacaa ttgagttttg cttacacatt tttaaatttc
88800

ttttaattcc gttccaggtg tgggcaaagg cctatgcctt ggaaggaaca ggtacatatg
88860

gagaggattg acaggagctg agcatttcag gagggcataa acaggggatg caggaagcca
88920

gagaggtagc aaaatgcccg atgaggcagg tctctctagg tcatgctggt aaattctttc
88980

ttcaccctaa atggaatgtg gggagggaac agtgacaggt tgagaattgg ggacacatct
89040

agcacacacc atgtggtacc aggttctctt cttagtactt ctacatatta tctcatatgt
89100

gcctcacaac aaccctgtgc aatagatccc attagtatcc tcattttttc aaatgaagaa
89160

aatgaagtac aaataggcct agggtcagtg tcagataaat gggcagaaat agcccctgtg
89220

tgtatccccc tggaatggct accccgttgt tcacttcttc atagcaagcc agagtccttg
89280

gccagagccc gtcagtgcca caccggcctg gctggggaat ggaaagtggg aggtgcaggt
89340

ttcttgcaca gatgcaagag gtacaggtac ttggcatcgt gtggctccag cttagcttag
89400

gagaaataca gaaaagaggt ttaaaatgca tatagcatag aggaaaagaa ttggcagatg
89460

ctaccagggg gcaaagggta gcaggaacag gtagtggaga ggggcagggc aggtcacggt
89520

gatgccagga cgggaggcac acagccccgg agctctggtg tccacactgc tttatattcc
89580

aaagcacctg cagggccagt gaagtgcttc cttcccagga ctctcgctct tggatttcca
89640

cgatgccatc cctgcctcct tcttgatgtg gattcccata atgctaacct gagctagacc
89700

cctagttctc catatccaac ccatctccat ggccaggcct ctctttcacg gtcaagtcaa
89760

gatagcaaat gccttgtggt ccccaccaag cttgattggt aaaggcctct gctaatatca
89820

gggtttccat ttggttttac cttagaagaa gagctgtggt tgggggcata gtaagttgcc
89880

acactgctga cttgtggaag gcccaactat ctctctactc tgagtagctc taaagcatcc
89940

tggtcaactt gtcttcccaa tacgtttttc tcatcagctg aggaaaagga agggtgtgct
90000

acggatggat gctttggaag atggtatgaa acactttcac actggttgtg tttcattatt
90060

tacttcagat tggttgtctg gtttaatcat ttcatttcca ctgatgacag aaaatttaat
90120

tttgctatca agacaatttt ggctaggcac ggtggctcat gactgtaatc ccagtgattt
90180

gggaggccga ggcaagagga ttacttgagg ccaggagttt gagaccagcc tgtcaacata
90240

gcaagacctc atctctacca aaataaaaaa taaaaaaatt agccaggcat ggtggcccat
90300

agctactcgg gaggttaagt gaggatcact tgagctcaag tgtttgaggc ttcagtgagc
90360

tatgatggca ccactgcact ccagcctgag tgacagagca agatcctgtc tgatatggtt
90420

tggctctgtg ccctcaccca aatcttatct caaattgtaa tccccttgtg tcagggaggg
90480

atgtggtggg aggtgattgg atcatggggg cggtttcccc aggctgttct catcattgtg
90540

agtgagctct catgagatct gatggtttaa aagtgttgta cttcccccca taaattaccc
90600

agtctcaggt agtatcttta tagcagtgtg agaatgaact aagacattgt ctcaaataag
90660

taacaaataa acaaataagt aaatgaccat tttacccctt ctattttaat acagagacaa
90720

atatcattct gtctcttgcc cttccatttc cccgggtgcc cacagagtgt cctggtactg
90780

agagagagag aagcgggtct tggtgtggcc ccatcttggt gggtcccacg gaagggtcta
90840

gtcctggtgg tcttaggcaa caggcagtgt gcaggctgca cctacaccca gacatagtct
90900

gcctcccacc acctccaaac ccagatcaca gggcccttct ggctggtcca tcatcaacca
90960

atctccatgc cccttgggag tagtttccaa gtctcttggg atatttgttc ccttgcattt
91020

ttggagacta ggggaagggg aggagggttg ttgcctatct caggcctctc tcacctttgc
91080

tatttatata tacatacaca tatatataca cacacacaca tatatataca tatatacata
91140

tatatacaca tatatacaca tatatataca catatatata cacacacata catatatata
91200

taatttgatt tttttcaact agcatgcact taaatttttt taaaaaatat tttgaaaatt
91260

tttgggggta cctagtagat gtatatatta acatgcactt tttgtatttt ttattgtggt
91320

aaactatata tagcattaag ttgatcatct taaccgcttg taagggtaca gttggtggca
91380

ttacagttac attcacaatg ttgtgcaacc atcaccagta tttccaaacc ttcttcacca
91440

ggccaccaga aactcgagac ccattaaata gcccctttca ccttccccca gccccaggta
91500

acctccagac tgctttctct ctctgtgaat ttgatgactc taggcacctc gtatcatgtg
91560

aaactacaca gcattggtcc ttttgtcacc agcttatttc acttaccaca atggcctcga
91620

ggttcatcca tgttgtagca ggtgtcagaa ctctcccaaa agattgcaaa aaccataatc
91680

tcacacaaag gtcatcacaa ccttacacaa aaaaaatact tctgggagga catctgccca
91740

gcaacagcct gtccaatctc gaattggcat cattcttatt attggtcctt gtagccaagg
91800

ataattatct caaaattttg agatagttat tttatatcaa aataaaatac gcatgccatt
91860

aataagctat gttcatattt ggggaggtaa gggaaaataa taataaaatt taaattagaa
91920

attaaaaata ctaataataa aagaaagaaa tgattttccc ttacctccct gaatatgaac
91980

atagcttatt acggcatgcg tattaccttg caatttccta atccttaagt aaaaattatt
92040

ttttttagag agcctctctt tgtttgttaa actgtgggct gacaatagaa tcgcacacac
92100

acagagtccc tgatttaggc taagagggga caggacccac ggaggagctg aagcttttat
92160

gtagtcttga actcattgtc ttctgggaca gtgctgctca aggtgccagt ccagaaagga
92220

gcttgtgcca gaatgtaatc aacgcagctt ccttcctcag gaaagtcttg tcccctctcc
92280

cccacaaaaa ggccagtgga actaaacagt gtgtgtagag atgtggttgg tgtctctgaa
92340

tttacattct agtacatgtt tcttatctca tttggggact ggcaaccaag agtcatattc
92400

agaagcccat ctgcagacca cattttgaat agtactattc tagaagacac atttccccaa
92460

gacacacagg ttatttttaa ctctcaacgc tgacgttaaa tctaatgatc cccgtgttca
92520

ttttcctttt tgctttttga atatcctaca tgtgtattca ccaaagaagc caaatgtgtt
92580

tgaaaaactt cccacaaaag agaattcttg gggcattgag tgagttaact gttctatcca
92640

ttcacacaag caaactggtg tcaagcattt aaatcaactg ctcatttagc aagttcaagt
92700

tgaagacttg aaataggata tgcctgtgtt gtactccatg atcttatctc tattccaacg
92760

caaaactgga aaaacttaag gaattcacaa atgctttaaa acctaattgg ccttaccctc
92820

actaatatca acccaccaag ggtatttatc atatagctaa ttgctcagag ggtttataat
92880

gttgacattt atcatgtatt acctactatg tgccagcact gtattaatcc tttatctaca
92940

ttaccccaat gaattctcag gatgaggcat cctatgagaa actccactgc tctgaaaaga
93000

aaagtaaatg aagaactacc taccgcggcc cagccgcagt gagtgagtca gagctggagt
93060

cctcagagta tggtcctgga ttagagcact ggcagcacct gagaacttga taaaatgcag
93120

gttcccaggc ctcactccag gcttactgaa tcagaaactc taggaccatg gccctgcaat
93180

ctgtgttcta accgccctcc agtgattctg atgctggcta aagtttgaga gccactgggc
93240

tacatgaaag gctctttctc acatgccctg ctttattcca aattcagatt ctccaaaccc
93300

aagcccctgc attatccaga aaagcaaatt atcctaaaga taaaacaatt attgaaaatt
93360

gttcttacat ggtgtcttaa taaagcacca ttttgctttt ctgcaaataa aaaaataatc
93420

atcagagcta gtaattattt ctcactctta tttttcctct aagaataaat tattgggaca
93480

ctatttttgg taaacaagat ggctcttgac aaaggcatgg ctgcttggca tttaagaaga
93540

tgcctggtgg catcagtttg ctggagctgc tggggcagag tattgtaacc tctatggctt
93600

aaaacaacag aaaactgtac agtgtcttgc acataataaa ctcccaataa atagatattc
93660

aaagcttcag taaagaatgg aagtgatgtt tttaaaacta ttttaaggca aaataatctg
93720

gtcacaaaat caggggccaa taaaagctgt ggcaggaagg tgagtcaaag aaccattgca
93780

gcagttcaaa tgaaagctag caggggtctt atttggaaag aaaaccatgg cactgggaag
93840

aggaactgtt tgcataaaat ttggcggtga tgccatggaa tactatgccg ccacgaaaaa
93900

gaatgagttc atgtcctttg cagggacatg gatggaagct ggaaaccatc attatcagaa
93960

aaataacaca ggaacagaaa accaaacact gcatgttctc attcataagt gggagttgac
94020

caatgagaac acatggaccc agggagggga atatcacaca ctggggcctg tcggtgggtg
94080

ggggacaagg ggagggagag cattaggaca aatacctaat gcatgtgggg cttaaaacct
94140

agatggtggg ttgataggtg gagcaaacta ccagggcaca tgtataccta tgtaacaaac
94200

ctgcatgttc tgcacatgta tcccacaact taaagtaaaa ttaaaaaaaa aaaattggtg
94260

ttggtggaag tgacatggtc ccaatgtgga ggactatggg gtgggaatta aaagttatat
94320

cccaccttct ccaagcaaat cacgtattcc catcctttgc cttactcctt actttccctg
94380

gggaaagggt ttcggatttc cccagcttca tgtactattc aacctgatga gcatttaagt
94440

ggaggcatga tgggtttggg gagaggcttt tcatagaaag agctttcagg aaaaaaaaaa
94500

aaagctcaaa agaaaatatg ggcctcagaa agttgaagtt ggttctggaa cgtaactcat
94560

caagtgagtc tgtctctgtt ccaatcaaag cattaatttt gaggttgagg ggttttctaa
94620

gtgtctcttt ttcttaccca aacaatatct gaacatgctg cttgggtctt gggtaggggc
94680

ctacaaaata aaaattatag taattctcca accaccatat tacatgattt tttgaatca
94740

atgcctttat ataaaaaagc ttaaccacaa acaacatgac atgcattcaa gaaaagagga
94800

tagaaactct gtagtgctat tagtcacaaa tggttttaat aggaaaatgt catgttccag
94860

agcaatatta tgtaagagtt atcaatagta tattcgcttc ttatggagac ttaattttta
94920

caaaacaaga tagttttttt tttaagacag agataaggtc tcgttctgtc acccaggctg
94980

gagtacagtg gtgcaagcat agctcgctgc agcctcaaac tcttgggctc aagcagtcct
95040

cccacttcag cctccctagt agctgagact acagggtgtc tctcaataaa ggcacttgtt
95100

gcttggcatt taaggagatg gctgattata ttagtttccc agggttgctg taacaaagta
95160

tcagagtcca aatagcttaa aactacggaa atgtattatt ctctcacagt tctggaggct
95220

ggaagtccaa aatcgaggtg tcaactaaga ggcagggctc tctcctgatt acactggggc
95280

cacccagata atctggagta atttccctaa aacaaacaaa gttccaaaac caaccaaaaa
95340

tcagtgacta tggttaaaaa aattacaaac attatcaaat cattttatgt gaaaagaagt
95400

atcccagctc agatggtgaa ctttttaaag attcaaagaa cagatattac tcctaaataa
95460

aaagtttcag agtatatata accatgaaaa gcttcccaat ttattgagca aaattagtat
95520

aatcttaatt ttaaaatgtt aacaaagata gcagaccaga aaataaaatt agtctcattt
95580

atgaatccta ctcataaatt ttacaaaaag aattggccat ttaaatttat tagcatgttt
95640

aaaatatatt ttccatagtc aaatgagatt tacccaaagg atacgggata attaaacaag
95700

aagagacctg ttacacgttg agaatcccca atttgaaaat ccaaaattca aaatgctcca
95760

aaatctgaaa ctttttgacc caacacggca ctcaaaggga atgctcactg gagaatatca
95820

gagtttagac tttcagatta gggtgctcat ctataacaca aatctttcaa aacccccaaa
95880

aataaaaatt ccagaacatt tctggtaaga ggcatttcag tagagatgtt caacctgtaa
95940

taaatcacaa ctgttggtca aatttttaaa ttatataata atatccaaag atttccagaa
96000

gttatctgat atttaacatc cactctgatt tttagaaact tctttagaag ccagtaattt
96060

taaaaatcta tcttaaactc aatcaacatt gttactgata aaacacaaga agcaatttta
96120

ttaaaatcag aaatcaacat acaaaagaag gaaagaaaga aagggaaaaa agctcaccct
96180

agcatttttg ttcttgaaat tctggccaaa aaattaagaa tgaagtatat aatagaccca
96240

ctattataat gaaatgcact ataagaactg aacattttta aaaatcagat ggcctgtatc
96300

ttaattttaa atcattttga agtggattag aacagagatt tgcaaagtca actgagagag
96360

acactggctg gctttgctta gccaactgta atgataatta taaaatcata actcatgttt
96420

tcataagcat ttcctctgtc aggtactgtg atactcactt gacagcattt aatactcact
96480

tcagatctac aaggtagatg tcaagaagca ttttcctccc tcactcctta cattcccacc
96540

tagaaagaaa gagatgtcca ctggctccca ggcctctctc tcaacctccc cttgctcact
96600

gcttcgcaga ggactcctgc aggactctcg cccctctgcc atctctattt ccttgaggcc
96660

tgcccagatg cacgaatcat ttgttcctcc caggagtaag cctactggaa cagaattggt
96720

tgtgggattg gcgggtgaga gagatggaac aagacagtgt tagttgtgag gaggaagaag
96780

tggaaagccc gattctaccc ccaaatccac ctgcattctt caactgagtt ttctcagtaa
96840

tgaaactagt gatttgggtt gatccatcta acttctgagt ctacatctga actctgagga
96900

gcagagggaa aggtggtaac aatcccattc aagccccgca gcgggcactc ttactgtcct
96960

tattttatta aagaaatcga ggcctgaaga gtgtattagg ctgttcttgt gttgctataa
97020

agaaagatct gagactaggt aatttataaa agaggtttaa ttgtctcacg gttctgcagg
97080

ctgtacgaga agcatggcat caacaactgc ttagcttctg gtgagggcct caggaagctt
97140

acaatcatgg cagaaggcga cggggagcca ctgtgtcatg tggtgagagc aggagcaaga
97200

aaaagaagga cgaggtccca gactcataaa cagccagatc tcatgtgaac taactgagca
97260

agaactcact catcaccaag gtgatggtgc taagccattc atgagggatc catccccatg
97320

atccaatacc tcccgctaag ccccacctct aacactgggg atcacatttc aacatgagat
97380

atggagggga cacacatcca aaccatatca gaggggttaa gcaacataca tgagcagtct
97440

tgatatgtct ccaagacagg ccactgagaa agacaggagg cagccaaatg ccaaatagat
97500

gggggcgggg ccctggtgaa accccagctc caaacaaaga caatctaaag cctgaaagcc
97560

aagctacaag ttaaatcctc ggaccagatt gagaacttgt cttcctgttt ggcatgcttt
97620

cctctgattg atccccacac gtcacctatg ttgcatatac ctacactttc ctaattggtt
97680

tcctatgcta ttgtgtgcac ctttgggtgg tgtctttgct ttaacctttt ttgtatactc
97740

acaaaccaat cagcacactc ttcccatcct gtggctataa agaccccaga ctcagtcagt
97800

agaggaggag atgacctgac ttcaggaaag agacaacctg actttgggga agacgatcta
97860

ctcttcctgt cccctctcta gctcccctct ccattgagag ttgttttcat cactcagtaa
97920

aattctctgc cttcaccatc cttcaatgat ctgtgtgacc acattcttct tggactctgg
97980

acaggagctc aggacacacc aaatgcgagt ccccagaaag gctgtcacac tggccctttg
98040

ccctcactgg tggagggcag ctgccccatg caatgataca aggggccaat tgatctacta
98100

acatgctgct gtctgtggat ggcagaatta aaggagcact gtaacgcccc ctcttgggcc
98160

tcagggttgc aggcaccctc acctgggtgc tgctgcattc ccctcaaggt gacacagctg
98220

gtctggccac aagccctgca cggagtttgc tcctgtgtca gtgcccagag ccatgggctg
98280

gattttgcac tcactagctc acatgcacct acccacaagg ggctgagcac agtgggctga
98340

gtagatgggc accccttctg tgagtccggc aaaggggcca agaaaaatcc tgcatcacca
98400

ccatgctcat accagctaga cacctgtgat gtaaaaggca ccttcctgaa gttgcacagt
98460

gcccaacttc ctgccccacg tggtggctgc agtggtcctg gtaccccttg agaagagtgt
98520

cctaggtggg agagtgtggt catagggtag gcaaacactc atctttacaa agtaatacca
98580

agcaggcttc caaattgttc atcaatctgc agtcctcctg cagtagtcta caacattaca
98640

gaaaaattca tcctgaaatt tgttggatgg taagaaagac tattcaagtg tcacctaaac
98700

aaacaaaaaa atcaaggttt taaagaatta aagtagtttt attcagaagt cttgaggaca
98760

gagactgagg ccaatagccc aggagcattc ctgttagact gcccagaaca gtgtttcaac
98820

ccactgctta catagagatg gcagggtgtc agagtgtgca aaagcacatc agacttgctc
98880

agaagtcaca ttatcacaga actacatcaa agtctgggtg taagagcata tctggttata
98940

aattacagag gtacaatcgc taactctgtc agacttttta tctcctatgt aggaagaggc
99000

aaggattagg gttatttatc ttttaaggaa tacagtgagt cagggaagag atgtgggagg
99060

ccgtgttctc tatcttggtt tgtcttcaaa gctgcacatc atcacagagg caggggtttt
99120

gtgaaattat gtgagcaagc agaaatgaac aaataaggtt tcttatgttt gctactttgt
99180

ctcacacaag aaagtattgc aatgggggtt ttgctatagg ggagagagat tgggctcaac
99240

tccaaataca acaaggacaa gtggagattt acagccaagg agtagagcaa ggggtcagta
99300

gatggaaaat tactaagagg atacctcaag gctaggggga ttattgctga aagcaggcca
99360

gggtgataag acatcaaggg tggggaagag gaatttgatc agatatcaaa gatggtcaga
99420

aaccaagagt gaagtttgag gaatttggtc agatattgag ggtaggggga atcttagata
99480

aactgaccca gcagtattct tgctaaacct ggactaagta ggccaagggc agagtccaag
99540

gtcaggactt agttgaaaag ggggccctga ggagctgact tgagtgtggt caaggagaga
99600

gtctttgtca atgagtcttt gttaaaagag gtatagctgt agcacatctc agctctgaga
99660

gtggctgagg tgggaggatt gcttgagccc aggagtttga gaccagcctg ggtaacatag
99720

tgagaccatg tctctactat tcttaaaaaa cttaaacaac attttttagt gaaaaaaaaa
99780

tgttcaaacc caacagcaaa gaaatgtaaa ttaacaacag aataccatgt taaattactc
99840

taatcagtat accaactcag ctagactcat ggaattatat taaaatataa tagtcaagtt
99900

tggcaagagt gagataaagt agtctttctc atttcataca catctggggt atgtaaattg
99960

atacagcttt tgtgaaaaga aaattgccaa tattcattaa atatcatttg aaaggtaatt
100020

ttaaacaaaa aaaaatttag aaataaacct caaagagtct gaaatgcaaa agaagctttt
100080

tgcataaaag tgctcatttc agcactactt ataatagcac aaaattgaaa acaacaataa
100140

acagcaatac caaaaaggtg attaaataaa ttgtgatgta ctattaaaaa gcaaaatact
100200

gttttgtgaa atatttaata atatgaagaa ataaatggga aattataaaa gtaaattgaa
100260

aataacatta gccataaaac tttacatttc aaaattttat ataaaaccta taatacaata
100320

ttaaatttta aaggactata tgagtattag aaatgtgcat agaacaactt agggcatgga
100380

tcaaaatatt aatactggtg atctctgagt catacttgat tttcaatttt cctatttata
100440

ttgttcaaca cttttcatat tttcaaaaat caatacataa tatttctata aattttaaaa
100500

gttaaaaata tatattattt aatatattaa aactccctta ctggtgttca agggtctaga
100560

gtcattagtg aagaaacata atatcatgta atgtagtgca tggtctgggt caagctgcct
100620

gtgtttcagt cctgaatttg ccacgtgcca gtggtgtgaa cttaagcagg ttacttaact
100680

cctttgtgcc tcagttttct cctctgtaaa atggaacaat cacagtaccc agcccataag
100740

atgtaactga attaatatgt gtaaagtgca tagaacaagt tttatgaaag caatgttatt
100800

attatatata aaaagaaaga gttatcaacc agattgcaca tttcttaaaa acaaggccca
100860

tttttcttag ctcttttata tcttttatat ctttattacc accttccccc caaaaaactc
100920

aaaagtagtg ctgggccaat agtgatgttc cttgcctata aaataagagg ccagcccctt
100980

cttgcccatt cctgttattc attttacttc agtattacaa atgtcagaag ggacctgccc
101040

agcatggacg gactgtgaaa atatggctct atcttcaaaa atgtcatcat catattacca
101100

aagcacagta tcattattga acattatagg caatgagatg ttaaggaaag caaatctcaa
101160

gtagccactt ggatttttat caaaatgctc tatacaactt ttgcccttag agcaacaaca
101220

acaccttctg gcagcacaag gaactgggtg gttgagggcc tcctgtggct gttgagaatg
101280

aagagctatt ggcctttggg taaggggcaa ggaatcctag aatgtggccc aaagaaggtg
101340

gtgggagagt ctgatcttga ttttaggagt tggtgagtag taggggaatg ggatcccatt
101400

caagggaaga tgtagacatc aggagagaat caagttatcc aagcagagtg actttgaggt
101460

tggtaatggg aaactaagta ggaagctgaa gggagagggg aggtcagagt gtaattgccc
101520

aacaggttct tcctgcccac tgcaccgaga aaaccaattc actgagatgg cagtatggca
101580

gtaaagaaag agttaaatta acatgaggtt agccacgcag aagatagagt ttattaccca
101640

caacatcctt cccaagaact cggaagctag ggtttttatg aataatttgg caggcaggca
101700

gggggctagg aaatgggtgc tgctgattag ctgggggtaa aatcatatgg gtgtgggaaa
101760

tggtcctcgc atgctgagtc tgcctcttgg tggggccaca ggactggctg agacatgagt
101820

catgggtctg gatggggttg gtgagttgcc agaatgcaaa aattttaaaa acatctcaaa
101880

agaccaatct tagattctac aatagtgata ttatctctaa gagcaattga ggaagtcaca
101940

aattctgtga cctctggcca tatgactcct gagcagtaag aaattataga aactatgcct
102000

acattttagc agaatgcagg cccctctcat aatcctaatc ttgtggcctt tcattagtca
102060

gggtttaagc ccctgaagaa ggaaggtgtc agttttaggg agggactatt atcatccttg
102120

cttcaaagtt aaactataaa ttaaatgctt cccaaggtta gcttggccca cacccaggaa
102180

tgaatgagga cagccagcct gtgaggctag aagtaaggtg gacacagcca ggctagatta
102240

tctcgctgtc ataatctttg caaaggcagt ttcaggagaa caatagccac caaaacagag
102300

gcaactactg agcactggta ctgtccgcct tgctggggtg atggttgtga aaaggctggt
102360

ttggaactca tggctgatgg tgtgatgggg tctccatctc cagccaggga gtgcagccac
102420

cactcactag tccagctgtc atccctgcta ggtcttacct atttgaagac acactgggtc
102480

ttgttcagca tcactccctc tttctacatc caattattgg tcaccaaggg atccaaaggt
102540

ctggctctct agccaaatct agacagttct gaagggtgat cctatcttca gaactccctg
102600

taggctgagc tgtggcatct gtggtgatga catcacagcc caatgtctcc ctctgcccag
102660

tcctgcttct tcgtctgcct cctgcagtgt tgaccccaag aattctccct gataaagtcc
102720

tgtgcagttg ataatcttcc cctcagagtc taggacagtc ccctccggcg gaggttatat
102780

cctagtggga cagctggaaa gcaaacaagc aagcaaacca gtaaatactt tcaggagcta
102840

gtaagtacta agaggaacag aaaaggggta aattgataaa cagtgatatg acatagctag
102900

ggtgatcagg aaaggccttt ctgaggagac aactttggga ctgagaactg aatgacaaaa
102960

agcatcagag tcaatggtca agattagcag gcattggcag aacagagaag gccgctgagg
103020

ctggagtggg gtggttcagg ttgagtgcag tagaaaatga agttggaaaa gggcctttct
103080

agataggagc ttgtgggcta tgataaagag tttgggtttt atgctaagtg aatgaaagac
103140

tctgaagggt ttaagtactg gagagacctg atctaattta ggtttgcaaa agatcactct
103200

tgctactggg acaaataaat tagagagaag agtagaggca ggaagattat tcaagaaatt
103260

cctgcctaca tctacactgt ttctcaaaag gcaagttgca gcccagtcat gggtagtgaa
103320

ataaaataca atggtttatg accttaaaca tttattaatg aaatagcata ggctggacta
103380

gactataaaa gaaaattgag tgcatggcat tctgtaacag taaaagctat ttcatgattc
103440

ttactgtagt gaaaaatgtt cttggctatg tgcattgctt caggattaaa agcaaacttc
103500

ttggtggggc acagtggctc atgcctgtaa tcccagcact ctgggaggct gaggcgagtg
103560

gatcatctga ggtcaggagt tcaagtccag cctggccaac atggagaaac ctcatctcta
103620

ctaaaagtac aaaaattagc caggcttggt ggtgtgccta tagtcccagc tactcgggag
103680

gctgaggcag aagaatccct tgaacctggg agatggaggt tgcagtgagc caagatcaac
103740

cactgcactc cagcctgggc aacagaggga gactccatct caaaaaaata ataataataa
103800

aataaaagca aacttcttat tgtagatctt agtaacaaaa ggttgaaaga cactggtctt
103860

gattgggaag gcacactggc gtgtacaagt tgcattatgt aaccaaggcc aggaaaagat
103920

gacttaagaa ggaactaaga gtaggctgtg gccgggtgca gtggctcacg cctgtaatcc
103980

cagcactttg ggaggccgag gcaggcggat cacgaggtca agagatcaag accatcctgg
104040

ccaacatggt gaaaccccgt ctctactgaa aattcaaaaa ttagctgggc gtagtggcac
104100

gcgcctatag tcctagctac tcgggaggct aaggaagggg aattgcttga acccaggaag
104160

cagaggttgc agtgagcaga gattgcacca ctgcactcca gtctggcgac agagcgagac
104220

tccatctcaa caacaacaac aacaacaaaa gagtaggctg caaggacaaa gggagtgtgg
104280

tgtcctacag tagtcagcgg agtcaaagac acctcaagaa gtagtaggga cagaacaggt
104340

gaaacagtgc ataggccagt ctcaaaaaga ccagaggaaa gaccagagca taactagagg
104400

gtccaggagg gtgcagagca gctgcagtga gatggtttgc acttttgaag ccatgtcaat
104460

cttggcagaa gcaaaatatt ctctcttgtt taaaaaaaca aaaaaaaaac agactccacc
104520

acaagctggc aggcacacac ttagagaggg tcccagtttc cgtttgagag agcaagttca
104580

tgagcttgga aggaacaggc caattgtgca acctcactca aaagggagga gttcctctcg
104640

tgggtggcaa gctccatggc cctctaggaa attgaaatgg aagaataaag gtgtcacagc
104700

ctgaacgaca agacagtgat gttgacagta atatttaagg tgagctggag aagtctatga
104760

gcaagcattt cggagagcca agggcatcct tctaccaata gcttccacag gtctgtagtt
104820

ccacaccatt ctctttgatc ttcgatctct gggcctggtg ccatggctca tgcctgtaat
104880

cccagcgctt taggaggctg aggcaggagg agcccaggag tttgagacta gcctgggcaa
104940

catagccaga ctccatttct ccaaaaaata aaaaaaaata aaaaattagc caggcgtaga
105000

ggtgtgtgtc tgtagttgca gctactttgg aggctgaggt gggaggattg cttgagccca
105060

ggaagttgag gctgcagtga gccgtgatgg ggccactgca ctccagctgg ggcaacagag
105120

ggagaccctc aacttaaaaa aaaagtcctc tggacacata tgtctaggaa atcttggatt
105180

ctgtattctt tcttgttacg tgttaaattg ttccccccaa aaatatgctg aagtcttaat
105240

ccccagtacc tcagaatgtg aacttatttt gaaacagtgt cattacagat gtaactaggt
105300

aaagtaggct gggcagggta gctcacgcct ttaagcctgg cactttggga agccaaggtg
105360

ggtgggtcac ttgagcccag gagttcgaga tcggcctggg caacataggg agacctcatc
105420

tctaccaaaa agtagtacaa aaattagctg ggcatcatgg catgcgcctg taatctcagc
105480

tactttggag gctgagtcag gaggatcact tgagcctgtg aggttgaggc tgcagtgagc
105540

tgtgatcacg ccactgcact ccatcctggg caacagagca agaccttgtc tcaaaacaaa
105600

caaacaaaac gaaacacaaa ataattaggt aaattgggat aaagtcatac tggagtaggg
105660

tgggccccaa atccaacatg actggtatct gtataagaga cccagggaaa tgggcctggt
105720

gttcatatca gacacaggga aacagctctg tgatgatgga ggcagattgg agtgatgtgt
105780

ctacaagcca aggaacaccc aaggattgtc ggctgacacc aacagctaag aagaggcaag
105840

ggaagattct ctctacgagg ttcagaggga gtgtagctga tgtctggttt ctggcctctg
105900

gagcttggag aggatccctg ttctttaagc cactggcttt gtggtgcttt gtcatggcag
105960

ccccaggaaa caaatataac ctcaagagtc acaacttaca tatagcgtcc aaaagtctaa
106020

gaagcccatg gtttaagaca cagagaccta aaatagtgct taagaaagtt attctaagga
106080

agatgtgctt tcatcccaat cttgacactt tggtgtgcta cttatatcat gctaccttca
106140

agggtccaac cagaaaggaa gaagttaaac tctcagacca gatttcagag aaaccctgta
106200

aagccagagg tttggactca cagcgtcagg ccaggcttct gtaccatttt aaggcctctc
106260

ggctgtaatg atgctttcac ttccccttat cacctgcctc ccgcgctgtg cccaccccag
106320

gtctgttggc agcagagaac tcacaagaga aggtgcctca gcggatgctg tttctgtctt
106380

catctcacag tcttaatctg ggtggaagaa aggcaggaga gaggccttat ctcttttcta
106440

gggaatattc ccatgcaaac aacaacaggg tgagaaccag agccatcaac attctggtga
106500

atagaagtca atcggaggag tccagccagt ggcgaggtgg ctcatgcctg taatcccagc
106560

actttgggag gccgaggtgg aaggattgct tgaatccagg aggtcgagaa cagccctggg
106620

caacatggtg aagccccatg tctacaaaaa aaaaaaaaat tagtcaggca tggtgacaca
106680

tgactgtggt cccagccgca cgggaggctg aggtgggagg attgcttgag cctgggaggt
106740

agagactgca gtgagtggaa atcacaccac tgcactccag cctgggtgac ggagctgaga
106800

tcttgtctca aaaataaata aaataaatta aaaaattaaa aagtatatca gaatgcaaag
106860

tatttcaaca agcttgatag aactgaactg tctattaact aagaggactc tggctcacgc
106920

gtctgtctgt ttagtagaac attcacatca attaccacca ctctgctgta aaacaaggaa
106980

gagctgagtg atgtgaaaag caaagttgtc ccaggagagg aggagggcgt tgctgaggag
107040

aggtggagtg ccagggatgc caggaagttg atgatggtgc tggtcttgcc caaacccatt
107100

tcctctcatg gatttgacat ggagcttatg tgatatgatg tgaacctatt catttgatc
107159

## Description

### Field of the invention

The present invention relates to a nucleic acid encoding the human thyroid dehalogenase and related sequences. The nucleic acids may be used in methods for producing a dehalogenase, as well as in methods for diagnosing or treating iodi-tyrosine dehalogenase deficiencies. The dehalogenases may be used for dehalogenation of halogenated products as used in diagnosis and in treatments as those involved in toxicology.

### Background of the invention

The thyroid is a small gland located in the front of the neck. Thyroid cells are the main cells in the body that absorb iodine (see e.g. in "Werner and Ingbar's The Thyroid: A fundamental and Clinical Text", 7^{th} edition, Eds. Braverman, L.E. and Utiger, R.D., Lippincot Williams & Wilkins, Philadelphia). These cells combine iodine and the amino acid tyrosine to synthesise thyroid hormones such as thyroxine (T₄) and triiodothyronine (T₃). T₃ and T₄ are then released into the blood stream and are transported throughout the body where they control metabolism and modulate growth and maturation of the nervous central system. The protein thyroglobulin (Tg) serves as a matrix for the synthesis of thyroid hormones. Iodination of the tyrosine residues of Tg occurs by means of the oxidase/peroxidase complex. Thyroid peroxidase (TPO) catalyses the iodination of tyrosine residues in Tg, thus forming deiodenated tyrosine residues that are subsequently coupled to form T4 and T3. Both the iodination and coupling process require Tg in the presence of hydrogen peroxide (H₂O₂), which is generated by NADPH- and Ca²⁺-dependent oxidases (ThOX1 and ThOX2). When thyroid hormone is required, the Tg molecule is hydrolysed by endo- and exopeptidases. T₄ and T₃ are then released from the Tg molecule and secreted to the blood. During this process also uncoupled mono- (MIT) and di-iodo-tyrosine (DIT) are released, MIT and DIT are dehalogenated and liberated Iodine is recycled to prevent excessive loss of the rare element Iodine. Other proteins involved in thyroid function are the TSH-receptor (TSH-R), governing the overall specific metabolism of the thyroid cell, the Gsα-protein coupled to TSH-R that triggers different signalling pathways in the thyroid cell and three thyroid-specific transcription factors known as TTF-1, TTF2 and Pax-8 that activate transcription of the thyroid-specific genes.

Defects (mutations or deletions) in genes encoding the proteins that are involved in thyroid physiology can lead to hypothyroidism. Hypothyroidism is the situation when too little or no hormones are produced by the thyroid gland. When it is present at birth, it is called congenital hypothyroidism (CH). CH is the most common endocrine congenital disorder, which, without treatment in the first days of life, can lead to a disturbance in brain development leading to lifelong cognitive and motoric problems. For this reason, early detection of CH is currently performed by neonatal screening in most of the countries.

In various cases of primary CH the genotype underlying the patient's phenotype has been identified, e.g. as is the case with TPO, TG, the sodium/iodide symporter (NIS), TSH-R and the thyroid-transcription factors PAX8, TTF1 and TTF2. There are, however, still etiologically unresolved cases of CH where unidentified genes must be involved.

One further protein of which deficiencies have been implicated in thyroid hormone synthesis disorders is e.g. the thyroid iodo-tyrosine dehalogenase (Codaccioni et al., 1970, Ann. Endocrinol. 31: 1161-1173; Rochiccioli and Duteau, 1974, Arch. F. Pediatr. 31: 25-36; Elewaut et al., 1972, Ann. Endocrinol. 33: 599-605; and Ismail-Beigi and Rahimifar, 1977, J. Clin. Endocrinol. Metab. 44: 499-506). The function of the iodo-tyrosine dehalogenase in thyroid, liver and kidney is to liberate iodide incorporated in di-iodo- and mono-iodo-tyrosine such that this rate-limiting element becomes available for re-use in thyroid hormone synthesis. However, apart from the facts that iodo-tyrosine dehalogenase activity has been established in thyroid, liver and kidney and that the dehalogenase is a reductase requiring FMN (for the bovine enzyme: Rosenberg and Goswani, 1979, J.Biol. Chem. 83: 336-347), little is known about the enzyme or the genotypes underlying its deficiencies, as is evidenced by the lack of publication in the last two decades.

It is therefore an object of the present invention to provide for nucleic acids that encode the thyroid iodo-tyrosine dehalogenase and that may be used in the diagnosis and therapy of the iodo-tyrosine dehalogenase deficiency.

### Description of the invention

In a first aspect the invention relates to a nucleic acid molecule comprising a sequence encoding a polypeptide with dehalogenase activity. The nucleic acid molecule is preferably selected from the group consisting of: (a) nucleic acid molecules encoding a polypeptide comprising an amino acid sequence having at least 60, 70, 80, 90, 95, 98 or 99% identity with the amino acid sequence of SEQ ID NO:1; (b) nucleic acid molecules comprising a nucleotide sequence that has at least 60, 70, 80, 90, 95, 98 or 99% identity with a nucleotide sequence as depicted in SEQ ID NO: 2; (c) nucleic acid molecules the complementary strand of which hybridises to a nucleic acid molecule having a nucleotide sequence as depicted in SEQ ID NO:2; and, (d) nucleic acid molecules the sequence of which differs from the nucleic acid molecule of (c) due to the degeneracy of the genetic code.

A nucleic acid molecule may also be selected from nucleic acid molecules encoding a polypeptide having dehalogenase activity comprising at least the catalytic site of the dehalogenating enzyme as defined by SEQ ID NO: 1 from positions 90-243, or by an amino acid sequence having at least 60, 70, 80, 90 95, 98 or 99% identity with the amino acid sequence of SEQ ID NO: 1 from positions 90-243.

A polypeptide with dehalogenase activity is herein understood to mean a polypeptide that is capable of dehalogenating a halogenated compound such as fluorinated, chlorinated, brominated or iodinated compounds. Preferably, the halogenated compound is an aromatic compound, more preferably an aromatic acid or an amino acid, of which aromatic amino acid such as tyrosine, are most preferred. More preferably, polypeptide with dehalogenase activity is capable of dehalogenating iodated compounds; in particular the polypeptide is capable of liberating iodine from di- or mono-iodo-tyrosine. Assays for determining dehalogenase activity are described in Phillips et al. (2001, J. Microbiol. Methods 47: 181-188) and Motosugi and Soda (1984, Tanpakushitsu Kakusan Koso. 29: 101-110). Assays for determining iodo-tyrosine dehalogenase activity are described in Ismail-Beigi and Rahimifar (1977, *supra*) and Rosenberg and Goswani (1979, *supra*).

"Sequence identity" is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

Nucleic acid sequences encoding polypeptides having dehalogenase activity may also be defined by their capability to hybridise with the (complementary strand of) the nucleotide sequence of SEQ ID NO: 2, preferably under moderate, or more preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0,1 M salt, or less, preferably 0,2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity.

Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

The nucleic acid molecules of the invention preferably encode a polypeptide with dehalogenase activity that is a mammalian, preferably a human dehalogenase.

In a further aspect, the invention relates to a nucleic acid probe comprising a part of a nucleotide sequence that has at least 90% with a nucleotide sequence as depicted in SEQ ID NO: 3. Preferably, the probe comprises a part of a nucleotide sequence that has at least 90% with a nucleotide sequence as depicted in SEQ ID NO: 3 from positions 90,000 to 45,000 more preferably from positions 86,000 to 49,000, most preferably from positions 85,433 to 49,696. Nucleic acid probes are used to analyse nucleic acids encoding the polypeptide having dehalogenase activity. Such analysis may include the analysis of the genotype with respect to sequence encoding dehalogenase, e.g. the presence or absence of specific mutations or alleles, or may involve analysis of the expression of nucleic acids encoding dehalogenase activity. Such analyses may be performed for diagnostic purposes (see below). Nucleic acid probes thus include poly- and oligonucleotides for use as hybridisation probes, as primers for sequencing or amplification and for use in techniques such as Oligonucleotide Ligation (-Amplification) assays (see e.g. US 4,988,617; US 5,876,924; WO 96/15271; and WO 97/45559) or Single-Strand Conformational Polymorphism assays (see e.g. Orita et al., 1989, Genomics 5: 874-879). For further nucleic acid analysis techniques reference is made to Sambrook and Russell (2001, Molecular Cloning: A Laboratory Manual, 3rd edition, Cold Spring Harbor Laboratory Press, Cold Spring Harbor NY). A preferred nucleic acid probe is a probe that comprises at least 10 contiguous nucleotides of a nucleotide sequence that has at least 90% with a nucleotide sequence as depicted in SEQ ID NO: 3. SEQ ID NO: 3 spans the entire genomic sequence of the human iodo-tyrosine dehalogenase. Probes derived from SEQ ID NO: 3 may thus be used to analyse both exonic and intronic sequences as well as non-coding sequence up- or downstream from the transcription unit that may be involved in the regulations of expression of the dehalogenase gene.

In another aspect the invention relates to a vector comprising a nucleic acid molecule or probe as defined above. Preferably the vector is a replicative vector comprising on origin of replication (or autonomously replication sequence) that ensures multiplication of the vector in a suitable host for the vector. Alternatively the vector is capable of integrating into the host cell's genome, e.g. through homologous recombination or otherwise.

A particularly preferred vector is an expression vector that wherein a nucleotide sequence encoding a polypeptide with dehalogenase activity as defined above, is operably linked to a promoter capable of directing expression of the coding sequence in a host cell for the vector.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues.

Expression vectors allow the polypeptides with dehalogenase activity of the present invention to be prepared using recombinant techniques in which a nucleotide sequence encoding the polypeptide of interest is expressed in suitable cells, e.g. cultured cells or cells of a multicellular organism, such as described in Ausubel et al., "Current Protocols in Molecular Biology", Greene Publishing and Wiley-Interscience, New York (1987) and in Sambrook and Russell (2001, *supra*); both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34:315 (describing cassette mutagenesis).

Typically, nucleic acids encoding the desired polypeptides are used in expression vectors. The phrase "expression vector" generally refers to nucleotide sequences that are capable of affecting expression of a gene in hosts compatible with such sequences. These expression vectors typically include at least suitable promoter sequences and optionally, transcription termination signals. Additional factors necessary or helpful in effecting expression can also be used as described herein. DNA encoding a polypeptide is incorporated into DNA constructs capable of introduction into and expression in an *in vitro* cell culture. Specifically, DNA constructs are suitable for replication in a prokaryotic host, such as bacteria, *e.g., E. coli,* or can be introduced into a cultured mammalian, plant, insect, e.g., Sf9, yeast, fungi or other eukaryotic cell lines.

DNA constructs prepared for introduction into a particular host typically include a replication system recognised by the host, the intended DNA segment encoding the desired polypeptide, and transcriptional and translational initiation and termination regulatory sequences operably linked to the polypeptide-encoding segment. A DNA segment is "operably linked" when it is placed into a functional relationship with another DNA segment. For example, a promoter or enhancer is operably linked to a coding sequence if it stimulates the transcription of the sequence. DNA for a signal sequence is operably linked to DNA encoding a polypeptide if it is expressed as a preprotein that participates in the secretion of the polypeptide. Generally, DNA sequences that are operably linked are contiguous, and, in the case of a signal sequence, both contiguous and in reading phase. However, enhancers need not be contiguous with the coding sequences whose transcription they control. Linking is accomplished by ligation at convenient restriction sites or at adapters or linkers inserted in lieu thereof.

The selection of an appropriate promoter sequence generally depends upon the host cell selected for the expression of the DNA segment. Examples of suitable promoter sequences include prokaryotic, and eukaryotic promoters well known in the art (see, e.g. Sambrook and Russell, 2001, *supra*). The transcriptional regulatory sequences typically include a heterologous enhancer or promoter that is recognised by the host. The selection of an appropriate promoter depends upon the host, but promoters such as the trp, lac and phage promoters, tRNA promoters and glycolytic enzyme promoters are known and available (see, e.g. Sambrook and Russell, 2001, supra). Expression vectors include the replication system and transcriptional and translational regulatory sequences together with the insertion site for the polypeptide encoding segment can be employed. Examples of workable combinations of cell lines and expression vectors are described in Sambrook and Russell (2001, *supra*) and in Metzger et al. (1988) Nature 334: 31-36. For example, suitable expression vectors can be expressed in, yeast, *e.g. S.cerevisiae, e.g*., insect cells, *e.g*., Sf9 cells, mammalian cells, *e.g*., CHO cells and bacterial cells, *e.g., E. coli*.

*In vitro* mutagenesis and expression of mutant proteins are described generally in Ausubel et al. (1987, supra) and in Sambrook and Russell (2001, supra). Also see, Kunkel (1985, supra; describing site directed mutagenesis) and Roberts et al. (1987, supra; describing cassette mutagenesis).

Another method for preparing polypeptides is to employ an *in vitro* transcription/translation system. DNA encoding a polypeptide is cloned into an expression vector as described *supra*. The expression vector is then transcribed and translated *in vitro*. The translation product can be used directly or first purified. Polypeptides resulting from *in vitro* translation typically do not contain the post-translation modifications present on polypeptides synthesised *in vivo,* although due to the inherent presence of microsomes some post-translational modification may occur. Methods for synthesis of polypeptides by *in vitro* translation are described by, for example, Berger & Kimmel, Methods in Enzymology, Volume 152, Guide to Molecular Cloning Techniques, Academic Press, Inc., San Diego, CA, 1987 (incorporated herein by reference in its entirety).

In a further aspect the invention thus relates to a host comprising a vector as defined above. The host cells may be prokaryotic or eukarotic host cells as indicated above. The host cell may be a host cells that is suitable for culture in liquid or on solid media. Alternatively, the host cell is a cell that is part of a multicellular organism such as a transgenic plant or animal, preferably a non-human animal.

A further aspect the invention relates to a method for producing a polypeptide with dehalogenase activity. The method comprises the step of culturing a host cell as defined above under conditions conducive to the expression of the polypeptide. Optionally the method may comprise recovery the polypeptide. The polypeptide may e.g. be recovered from the culture medium by standard protein purification techniques, including a variety of chromatography methods known in the art per se.

Another aspect of the invention relates to a transgenic animal comprising in its somatic and germ cells a vector as defined above. The transgenic animal preferably is a non-human animal. Methods for generating transgenic animals are e.g. described in WO 01/57079 and in the references cited therein. Such transgenic animals may be used in a method for producing a polypeptide with dehalogenase activity, the method comprising the step of recovering a body fluid from a transgenic animal comprising the vector or a female descendant thereof, wherein the body fluid contains the polypeptide, and, optionally recovery of the polypeptide from the body fluid. Such methods are also described in WO 01/57079 and in the references cited therein. The body fluid containing the polypeptide preferably is blood or more preferably milk.

Yet another aspect of the invention relates to a transgenic plant comprising in its cells a vector as defined above. Methods for generating transgenic plants are e.g. described in U.S. 6,359,196 and in the references cited therein. Such transgenic plants may be used in a method for producing a polypeptide with dehalogenase activity, the method comprising the step of recovering a part of a transgenic plant comprising in its cells the vector or a part of a descendant of such transgenic plant, whereby the plant part contains the polypeptide, and, optionally recovery of the polypeptide from the plant part. Such methods are also described in U.S. 6,359,196 and in the references cited therein.

A further aspect of the invention relates to a polypeptide with dehalogenase activity and comprising an amino acid sequence having at least 90% identity with the amino acid sequence of SEQ ID NO: 1. The polypeptide with dehalogenase activity is further preferably as described above. Included within the scope of the invention are mutant polypeptides having dehalogenase activity with e.g. altered substrate specificity. Based on the divers substrate specificity of (non-human) dehalogenating enzymes known in the art, the skilled person will know how to modify essential amino acids by minor amino acid substitutions and or deletions to redirect substrate specificity. The tertiary structure of a dehalogenating enzyme can be altered applying the same principle as above to adapt the catalytic domain such to accommodate different substrates e.g. proteins or fatty acids to be dehalogenated and to allow any of the halogens to be targeted by the human dehalogenating enzyme based on the enzyme depicted in SEQ ID NO: 1. (Journal Medical Chemistry 1979 Issue 40 nr 8).

Another aspect of the invention relates to an antibody or antibody-fragment that specifically binds to a polypeptide with dehalogenase activity as defined above. Methods for generating antibodies or antibody-fragments that specifically binds to a given polypeptide are described in e.g. Harlow and Lane (1988, Antibodies: A Laboratory Manual, Cold Spring Harbor Laboratory Press, Cold Spring Harbor, NY) and WO 91/19818; WO 91/18989; WO 92/01047; WO 92/06204; WO 92/18619; and US 6,420,113 and references cited therein. The term "specific binding," as used herein, includes both low and high affinity specific binding. Specific binding can be exhibited, e.g., by a low affinity antibody or antibody-fragment having a Kd of at least about 10⁻⁴ M. Specific binding also can be exhibited by a high affinity antibody or antibody-fragment, for example, an antibody or antibody-fragment having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater.

Another aspect of the invention relates to microarrays (or other high throughput screening devices) comprising the nucleic acids or polypeptides as defined above. A microarray is a solid support or carrier containing one or more immobilised nucleic acid or polypeptide fragments for analysing nucleic acid or amino acid sequences or mixtures thereof (see e.g. WO 97/27317, WO 97/22720, WO 97/43450, EP 0 799 897, EP 0 785 280, WO 97/31256, WO 97/27317, WO 98/08083 and Zhu and Snyder, 2001, Curr. Opin. Chem. Biol. 5: 40-45). Microarrays comprising the nucleic acids may be applied e.g. in methods for analysing dehalogenase genotypes as indicated below. Microarrays comprising polypeptide may be used for detection of suitable candidates of substrates to be dehalogenated. These arrays are especially suitable in the screening for non-iodine halogens to be targeted by the modified dehalogenating enzyme engineered by minor modifications as described above.

In another aspect the invention relates to method for analysing the iodo-tyrosine dehalogenase genotype of a subject, a tissue sample or cell or cell-line. Analysing the iodo-tyrosine dehalogenase genotype is herein understood to mean analysing whether or not a genetic defect is present in the sample that underlies a dehalogenase deficiency. The method preferably comprises the use of a nucleic acid molecule or probe as defined above. The method preferably is a method for diagnosing a iodo-tyrosine dehalogenase deficiency. Alternatively, the method for diagnosing a iodo-tyrosine dehalogenase deficiency, is a method, which comprises the use of an antibody or antibody-fragment as defined above.

In another aspect the invention relates to an expression vector as defined above, wherein the vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; and references cited therein. The vectors are preferably formulated in a pharmaceutical composition comprising a suitable pharmaceutical carrier as defined below.

In a further aspect the invention relates to the use of a vector suitable for gene therapy as defined above in the manufacture of a medicament for the treatment of a iodo-tyrosine dehalogenase deficiency. Similarly the invention relates to a method for treating a iodo-tyrosine dehalogenase deficiency, wherein the method comprising the step of administering to a subject suffering from a iodo-tyrosine dehalogenase deficiency, a pharmaceutical composition comprising a vector suitable for gene therapy as defined above, in an amount effective to overcome the deficiency.

In a further aspect the invention relates treatments aimed at reducing or inhibiting thyroid dehalogenase activity. Such methods may be useful in the treatment of hyperthyroidism, contamination with radioactive halogens (e.g. after a nuclear event) or toxification. The invention thus also relates to the use of a vector suitable for gene therapy as defined above in the manufacture of a medicament for treatment of hyperthyroidism, contamination with radioactive halogens (e.g. after a nuclear event) or toxification. Such gene therapy vectors are thus aimed at inhibiting dehalogenase expression and may express an antisense or an RNAi sequence comprising (part of) the above defined dehalogenase sequences. Similarly the invention relates to a method for treating these conditions, wherein the method comprising the step of administering to a subject suffering from these conditions, a pharmaceutical composition comprising a vector suitable for gene therapy as defined above, in an amount effective to overcome the hyperthyroidism or adverse effects of a nuclear event or achieve detoxification.

In a similar manner the invention relates to the use of a substrate suitable for inhibiting dehalogenase enzyme activity in the manufacture of a medicament for the treatment of hyperthyroidism, contamination with radioactive halogens (e.g. after a nuclear event) or toxification, by administering an excess a substrate capable of inhibiting dehalogenase activity. Likewise the invention relates to a method for treating of hyperthyroidism, contamination with radioactive halogens (e.g. after a nuclear event) or toxification, wherein the method comprising the step of administering to a subject suffering from of hyperthyroidism, contamination with radioactive halogens (e.g. after a nuclear event) or toxification, a pharmaceutical composition comprising a substrate as defined above, in an amount effective to overcome the hyperthyroidism or adverse effects of a nuclear event or achieve detoxification. Methods and substrates for inhibiting dehalogenase enzyme activity are e.g. described in Green (1968, Endocrinol. 83: 336-347; 1971, J. Clin. Invest. 50: 2474-84; 1976, Endocrinol. 98: 10-19), Fetzner and Lingens (1994, Microbiol. Rev. 58: 641-85), Friedlos et al. (1997, J. Med. Chem. 40: 1270-84).

The invention further relates to a pharmaceutical preparation comprising a polypeptide with dehalogenase activity as defined above. The composition preferably comprises a pharmaceutically acceptable carrier. In some methods, the polypeptide with dehalogenase activity purified from mammalian, insect or microbial cell cultures, from milk of transgenic mammals or other source is administered in purified form together with a pharmaceutical carrier as a pharmaceutical composition. Methods of producing pharmaceutical compositions comprising the polypeptide with dehalogenase activity are described in are described in US Patents No.'s 5,789,543 and 6,207,718. The preferred form depends on the intended mode of administration and therapeutic application. The pharmaceutical carrier can be any compatible, non-toxic substance suitable to deliver the polypeptides to the patient. Sterile water, alcohol, fats, waxes, and inert solids may be used as the carrier. Pharmaceutically acceptable adjuvants, buffering agents, dispersing agents, and the like, may also be incorporated into the pharmaceutical compositions.

The concentration of the polypeptide with dehalogenase activity in the pharmaceutical composition can vary widely, i.e., from less than about 0.1% by weight, usually being at least about 1% by weight to as much as 20% by weight or more.

For oral administration, the active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable colour, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance.

The polypeptide with dehalogenase activity is preferably administered parentally. The polypeptide with dehalogenase activity for preparations for parental administration must be sterile. Sterilisation is readily accomplished by filtration through sterile filtration membranes, prior to or following lyophilisation and reconstitution. The parental route for the polypeptide with dehalogenase activity administration is in accord with known methods, e.g. injection or infusion by intravenous, intraperitoneal, intramuscular, intraarterial or intralesional routes. The polypeptide with dehalogenase activity is administered continuously by infusion or by bolus injection. A typical composition for intravenous infusion could be made up to contain 10 to 50 ml of sterile 0.9% NaCl or 5% glucose optionally supplemented with a 20% albumin solution and 1 to 50 µg of the polypeptide with dehalogenase activity. A typical pharmaceutical composition for intramuscular injection would be made up to contain, for example, 1 - 10 ml of sterile buffered water and 1 to 100 µg of the polypeptide with dehalogenase activity of the present invention. Methods for preparing parenterally administrable compositions are well known in the art and described in more detail in various sources, including, for example, Remington's Pharmaceutical Science (15th ed., Mack Publishing, Easton, PA, 1980) (incorporated by reference in its entirety for all purposes).

The pharmaceutical compositions of the present invention are usually administered orally. Intradermal, intramuscular or intravenous administration is also possible in some circumstances. The compositions can be administered for prophylactic treatment of individuals suffering from, or susceptible to, a iodo-tyrosine dehalogenase deficiency in an amount sufficient to prevent, delay or reduce the severity of the deficiency as manifested by the symptoms of congenital hypothyroidism or other thyroid hormone synthesis disorders associated with a dehalogenase deficiency.

For therapeutic applications, the pharmaceutical compositions are administered to a patient suffering from established congenital hypothyroidism or other thyroid hormone synthesis disorders associated with a dehalogenase deficiency in an amount sufficient to reduce the severity of symptoms and/or prevent or arrest further development of symptoms. An amount adequate to accomplish this is defined as a "therapeutically-" or "prophylactically-effective dose" Such effective dosages will depend on the severity of the condition and on the general state of the patient's health. In general, a therapeutically- or prophylactically-effective dose preferably is a dose, which restore T₃ and/or T₄ serum levels to the average levels found in normal unaffected healthy individuals.

In the present methods, the polypeptide with dehalogenase activity is usually administered at a dosage of about 1 µg/kg patient body weight or more per week to a patient. Often dosages are greater than 10 µg/kg per week. Dosage regimes can range from 10 µg/kg per week to at least 1 mg/kg per week. Typically dosage regimes are 10 µg/kg per week, 20 µg/kg per week, 30 µg/kg per week, 40 µg/kg week, 60 µg/kg week, 80 µg/kg per week and 120 µg/kg per week. In preferred regimes 10 µg/kg, 20 µg/kg or 40 µg/kg is administered once, twice or three times weekly. In case of CH, treatment is typically continued for the life of the patient. Treatment is preferably administered by parenteral route. Alternatively, in some conditions it may be desirable to achieve higher than normal levels, e.g. 150% of normal levels, 200% of normal levels or even 300% of normal levels.

Another aspect of the invention relates to the use of a polypeptide with dehalogenase activity as defined above, in the manufacture of a medicament for the treatment of a iodo-tyrosine dehalogenase deficiency. Similarly, the invention relates to a method for treating a iodo-tyrosine dehalogenase deficiency, the method comprising the step of administering to a subject suffering from a iodo-tyrosine dehalogenase deficiency, a pharmaceutical composition comprising a polypeptide with dehalogenase activity as defined above, in an amount effective to overcome the deficiency.

Another aspect of the invention relates to the use of an polypeptide which has at least 60, 70, 80, 90, 95, 98 or 99% homology with the amino acid sequence as depicted in SEQ ID NO:2 provided the polypeptide has no dehalogenase activity, in the manufacture of a medicament for the treatment of hyperthyrodism or for use in detoxification or for the treatment after a nuclear event. Administering an excess of the inactive form of the dehalogenase that preferably is capable of binding its halogenated substrate (e.g. a halogenated compound as defined herein), by competitive inhibition the dehalogenase rate will be reduced, preferably to a degree sufficient to overcome the hyperthyroidism or adverse effects of a nuclear event or achieve detoxification.

In a further aspect the invention relates to a method for dehalogenating a halogenated compound, the method comprising the step of bringing a halogenated compound into contact with host cells as defined above, a polypeptide with dehalogenase activity as defined above, or a polypeptide obtained in a method for producing such polypeptide as defined above, under conditions whereby the polypeptide dehalogenates the halogenated compound. The halogenated compound may be a fluorinated, chlorinated, brominated or iodated compound. Preferably, the halogenated compound is an aromatic compound, more preferably an aromatic acid or an amino acid, of which aromatic amino acids such as tyrosine, are most preferred. More preferably, dehalogenated compound is an iodated compound, in particular di- or mono-iodo-tyrosine.

### Description of the figures

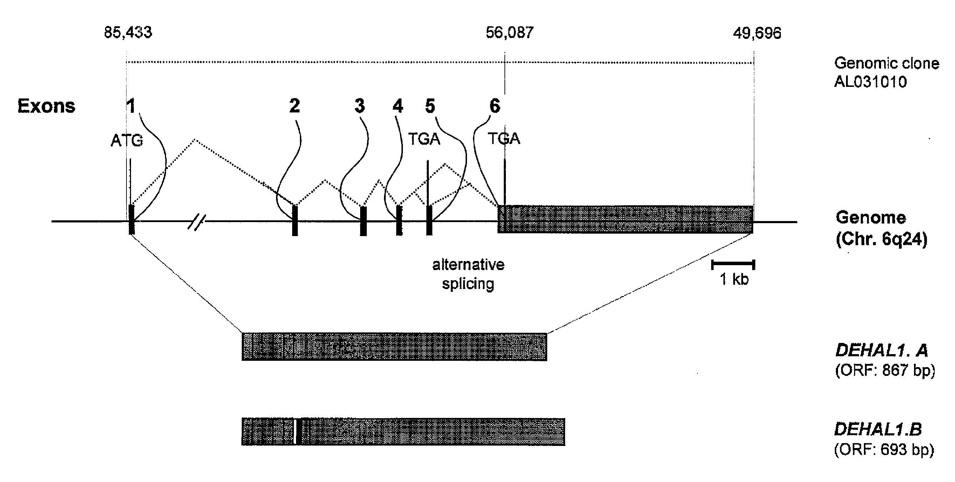

Figure 1: Schematic representation of the genomic organisation of the human iodo-tyrosine dehalogenase gene. Lines represent genomic DNA sequences. Boxes represent cDNA sequences. For the exact position of each exon-intron boundary in the human genomic clone (AL031010), see Table 2.

### Examples

### Example 1

### Isolation and characterisation of cDNA and genomic clones encoding the thyroid iodo-tyrosine dehalogenase

### 1. Methods and materials

For molecular cloning techniques general reference is made to Sambrook and Russell, 2001, Molecular Cloning: A Laboratory Manual, 3rd edition, Cold Spring Harbor Laboratory Press, Cold Spring Harbor NY. Nucleic acid modifying enzymes, kits and automated DNA synthesisers were used according to the manufacturer's instructions.

### 2.1. Isolation of a partial cDNA NM159 and identification of the corresponding genomic sequence in GenBank

As a starting point for the identification of novel thyroid-specific genes involved in thyroid disease, a SAGE-library was made from normal human thyroid tissue (Pauws et al., 2000, J. Clin. Endocrinol. Metab. 85: 1923-1927). This library, at the date of publication, contained 6099 unique tags of which 1839 corresponded to known genes and the other 4260 tags found any match in the current set of known human genes deposited in the GenBank database and, therefore, putatively correspond to novel genes expressed in the thyroid cell.

These so-called "NoMatch" tags were used to identify the corresponding uncharacterised genes. In order to select a reduced number of thyroid-specific "NoMatch" tags out of the large list of SAGE-tags, a computational-subtraction method was developed based on the comparison of the abundance of transcripts in 10 different human tissues (Moreno et al., 2001, Genomics 75:70-76). Following this approach, a group of NoMatch-tags was selected for on the basis of their preferential expression in the thyroid gland. One of these NoMatch-tags is NoMatch159 (NM159), the further analysis of which is herein described.

The 10 bp of NM159 SAGE-tag (aaaagtatta) was firstly linked to a human 3'EST sequence (AA100295) of 296 bp using the SAGE map program of the NCBI (see: http://www.ncbi.nlm.nih.gov/SAGE/SAGEtag.cgi). SAGE-tags are located just after the most 3' located catg site present in the mRNA molecule: the restriction site of NlaIII, one of the enzymes used to isolate each individual tag in the SAGE technique (see Pauws et al. *supra*). The AA100295 EST sequence was used to design primers in order to amplify the gene by RT-PCR and confirm its thyroid-specific pattern of expression using Northern blots (not shown).

An IMAGE clone containing the AA100295 EST sequence that included the NM159 SAGE tag at the proper position, was ordered at the IMAGE consortium (see: http://image.llnl.gov) and the insert was fully sequenced, extending the cDNA sequence to 1.5 kb. Looking for more upstream sequence, a 5' RACE PCR was performed and around 200 additional base pairs of the gene were further identified. However, within this sequence, no ORF was found, indicating that the sequence was partial and probably corresponding to a long 3' UTR. This sequence was blasted to the GenBank database to find out if it had homology with other genes on the human genome or other genomes. The sequence found a perfect hit to a human genomic clone on chromosome 6q 24 (accession number: AL031010). Also some ESTs were found upstream the 1.7 kb sequence, which were all belonging to one large exonic fragment.

### 2.2 Characterisation of the genomic organisation of NM159 gene

The complete sequence of the human genomic clone (AL031010) located upstream the 1.7 kb sequence of NM159 was submitted to different so-called "gene finder" computer programs that can predict exon-intron boundaries. Based on the predicted exons we designed primers to test if they were amplifiable on cDNA and also to confirm the connection between the predicted exons and the 1.7 kb sequence that included the 3'EST that contained the NM159 SAGE tag. First strand cDNA was synthesised from thyroid mRNA using random hexamers and reverse transcriptase according to standard procedures (GibcoBRL). RT-PCR amplifications of multiple fragments were performed using the oligonucleotides given in Table 1. These amplifications confirmed most of the predicted exons and their connections except for the most upstream intron and, in addition identified an additional intron (data not shown).

In order to identify and obtain the most upstream exon, a 5'-RACE-PCR was performed using primers based in a confirmed downstream exon and the SMART RACE cDNA Amplification Kit™ (Clontech, CA, USA).

Together these experiments identified two alternatively spliced mRNA's containing respectively ORF1A and ORF1B. An overview of all the exonic fragments identified for the two ORFs (ORF1A complete and ORF1B partial) is shown in Figure 1. The exact size of each exon and the position within clone AL031010 is given in Table 2.

**Table 2:**

| NM159 exons, their position in AL031010 and their sizes. Exon 5 is the alternatively spliced exon in ORF1B. | | |
|---|---|---|
| Exon | Position in AL031010 | size in bp |
| 6 | 56088 - 56270 | 183 |
| 5 | 58759 - 58874 | 116 |
| 4 | 60070 - 60228 | 159 |
| 3 | 61823 - 61981 | 159 |
| 2 | 64783 - 64973 | 191 |
| 1 | 85116 - 85293 | 178 |

### 2.3 Sequence of the NM159 ORF1

The nucleotide sequence of the reconstructed NM159 ORF1A cDNA was determined using standard procedures and is presented in SEQ ID NO:2. The encoded amino acid sequence is presented in SEQ ID NO:1. The complete ORF1 of 867 bp contains 5 exons, one exon (5) is alternatively spliced in ORF2 (sees Figure 1). The 3' UTR is 6.386 kb giving an mRNA total length of 7.410 kb. This length fits with the length of a major mRNA of approximately 7 kb that was identified on Northern blots using a part of the ORF1A cDNA as probe.

The ORF1A cDNA encodes a predicted protein of 288 amino acids. Using T7 RNA polymerase for in vitro transcription and a reticulocyte lysate for in vitro translation, produced a 32 kDa protein on SDS-PAGE, which is in agreement with the predicted molecular weight of the protein.

The amino acid sequence of ORF 1(A and B) of NM159 was analysed with various computer programs and databases to gain some insight about the function aspects of the protein. Homology search programs found that the NM159 protein is highly homologous to different sequences in the mouse (*Mus musculus* AK002363), fly (*Drosophila melanogaster* AE003546) and *C.Elegans* (L23649). The function of these proteins are not known.

However, the most relevant information achieved with respect to function was that the NM159 protein belongs to the nitroreductase family of proteins. The typical nitroreductase domain is present in SEQ ID NO:1 from positions 90-243. In addition, putative flavin mononucleotide (FMN) ligand binding sites were identified in the NM159 amino acid sequence. These data suggest that the NM159 protein might be the thyroid dehalogenase, which is known to be a reductase and is known to require FMN and which is known to be active in thyroid, liver and kidney.

### 2.4. Expression of a protein with iodo-tyrosine dehalogenase activity from the NM159 cDNA in CHO cells

cDNA's encoding the ORF1A and ORF1B amino acid sequences were cloned into suitable expression vectors for CHO cells (Sambrook and Russell, 2001, *supra*). CHO cells were selected that stably express the ORF1A and ORF1B encoded polypeptides. It was found that the ORF1A encoded polypeptide was capable of converting di-iodo-tyrosine to mono-iodo-tyrosine and free iodide, whereas the ORF1B encoded polypeptide was not. Thus we conclude that the ORF1A encoded polypeptide, i.e. the protein having the amino acid sequence of SEQ ID NO:1, is a human thyroid iodo-tyrosine dehalogenase.

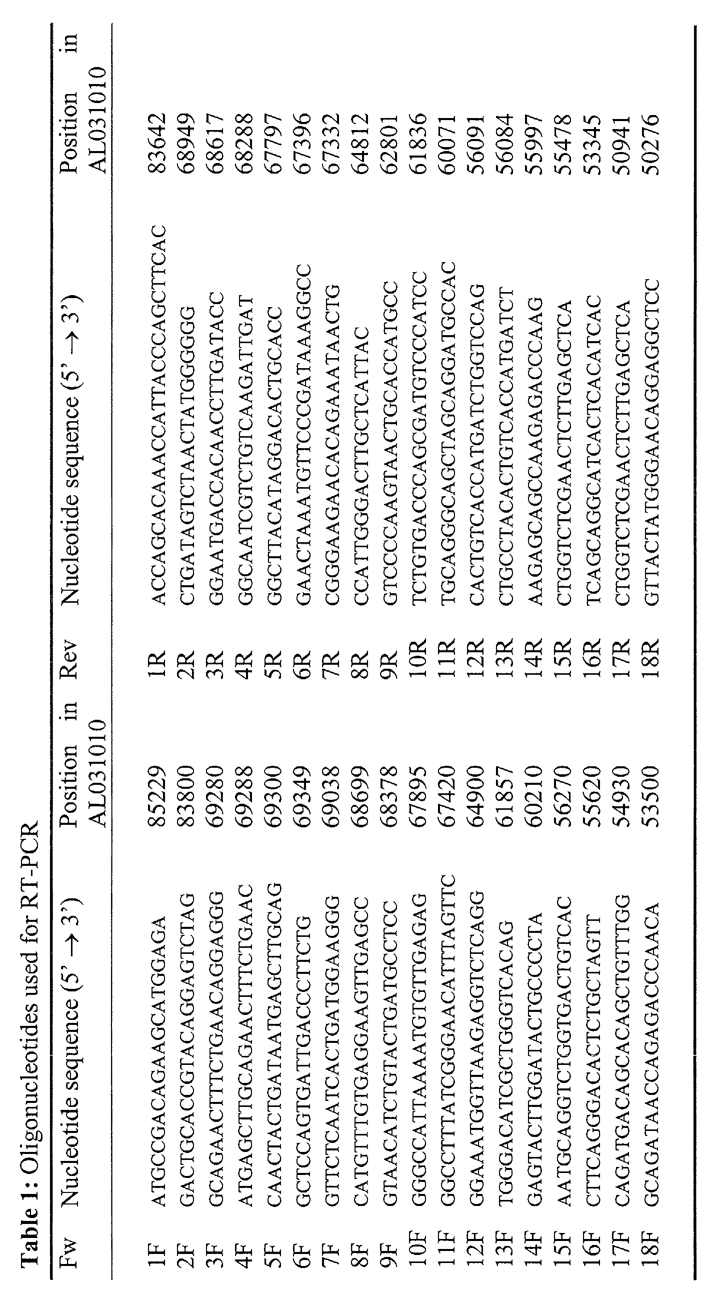

## Claims

1. A nucleic acid molecule comprising a sequence encoding a polypeptide with dehalogenase activity, wherein the nucleic acid molecule is selected from the group consisting of:
(a) nucleic acid molecules encoding a polypeptide comprising an amino acid sequence having at least 90% identity with the amino acid sequence of SEQ ID NO:1;
(b) nucleic acid molecules comprising a nucleotide sequence that has at least 90% with a nucleotide sequence as depicted in SEQ ID NO:2;
(c) nucleic acid molecules the complementary strand of which hybridises to a nucleic acid molecule having a nucleotide sequence as depicted in SEQ ID NO:2; and,
(d) nucleic acid molecules the sequence of which differs from the nucleic acid molecule of (c) due to the degeneracy of the genetic code.

2. A nucleic acid according to claim 1, wherein the polypeptide has di-iodo-tyrosine dehalogenase activity.

3. A nucleic acid according to claims 1 or 2, wherein the polypeptide is a mammalian, preferably a human dehalogenase.

4. A nucleic acid probe comprising a part of a nucleotide sequence that has at least 90% with a nucleotide sequence as depicted in SEQ ID NO: 3.

5. A nucleic acid probe according to claim 4, wherein the probe comprises at least 10 contiguous nucleotides of a nucleotide sequence that has at least 90% with a nucleotide sequence as depicted in SEQ ID NO: 3.

6. A vector comprising a nucleic acid molecule or probe as defined in claims 1 to 5.

7. An expression vector comprising a nucleic acid molecule according to any one of claims 1 to 3, wherein the sequence encoding a polypeptide with dehalogenase activity is operably linked to a promoter capable of directing expression of the coding sequence in host cells for the vector.

8. A host cell comprising a vector according to claims 6 or 7.

9. A method for producing a polypeptide with dehalogenase activity, the method comprising the step of culturing a host cell as defined in claim 8 under conditions conducive to the expression of the polypeptide, and, optionally recovering the polypeptide.

10. A transgenic animal comprising in its somatic and germ cells a vector according to claims 6 or 7.

11. A method for producing a polypeptide with dehalogenase activity, the method comprising the step of recovering a body fluid from a transgenic animal as defined in claim 10 or a female descendant thereof, wherein the body fluid contains the polypeptide, and, optionally recovery of the polypeptide from the body fluid.

12. A transgenic plant comprising in its cells a vector according to claims 6 or 7.

13. A method for producing a polypeptide with dehalogenase activity, the method comprising the step of recovering a part of a transgenic plant as defined in claim 12 or a descendant thereof, where the plant part contains the polypeptide, and, optionally recovery of the polypeptide from the plant part.

14. A polypeptide with dehalogenase activity and comprising an amino acid sequence having at least 90% identity with the amino acid sequence of SEQ ID NO: 1.

15. A polypeptide according to claim 14, wherein the polypeptide has di-iodo-tyrosine dehalogenase activity.

16. An antibody or antibody-fragment that specifically binds to a polypeptide as defined in claims 14 or 15.

17. A method for analysing the iodo-tyrosine dehalogenase genotype of a subject, the method comprising the use of a nucleic acid molecule or probe as defined in any one of claims 1 to 5.

18. A method according to claim 17, wherein the method is a method for diagnosing an iodo-tyrosine dehalogenase deficiency.

19. A method for diagnosing an iodo-tyrosine dehalogenase deficiency, wherein the method comprises the use of an antibody or antibody-fragment as defined in claim 18.

20. An expression vector according to claim 7, wherein the vector is a vector that is suitable for gene therapy.

21. A pharmaceutical preparation comprising a vector as defined in claim 20.

22. Use of a vector according to claim 20 in the manufacture of a medicament for the treatment of an iodo-tyrosine dehalogenase deficiency.

23. A pharmaceutical preparation comprising a polypeptide as defined in claim 14.

24. Use of a polypeptide as defined in claims 14 or 15, in the manufacture of a medicament for the treatment of an iodo-tyrosine dehalogenase deficiency.

25. A method for treating a iodo-tyrosine dehalogenase deficiency, the method comprising the step of administering to a subject suffering from a iodo-tyrosine dehalogenase deficiency, a pharmaceutical composition according to claims 21 or 23, in an amount effective to overcome the deficiency.

26. A method for dehalogenating a halogenated compound, the method comprising the step of bringing the halogenated compound into contact with host cells as defined in claim 8, a polypeptide as defined in claims 14 or 15, or a polypeptide obtained in a method as defined in any one of claims 9, 11 or 13, under conditions whereby the polypeptide dehalogenates the halogenated compound.
